# EUROPEAN PATENT SPECIFICATION

(11) **EP 4 196 608 B1**
(45) Date of publication and mention of the grant of the patent: **23.10.2024**
(21) Application number: 21763270.2
(22) Date of filing: 09.08.2021
(51) Int. Cl.: C12Q 1/6869

(54) **NUCLEOSIDE-5'-OLIGOPHOSPHATES TAGGED WITH POSITIVELY-CHARGED POLYMERS, NANOPORES INCORPORATING NEGATIVE CHARGES, AND METHODS AND SYSTEMS USING THE SAME**
MIT POSITIV GELADENEN POLYMEREN MARKIERTE NUKLEOSID-5'-OLIGOPHOSPHATE, NANOPOREN MIT NEGATIVEN LADUNGEN SOWIE VERFAHREN UND SYSTEME DAMIT
NUCLÉOSIDES -5 '-OLIGOPHOSPHATES MARQUÉS AVEC DES POLYMÈRES À CHARGÉ POSITIVE, NANOPORES INCORPORANT DES CHARGES NÉGATIVES, ET PROCÉDÉS ET SYSTÈMES LES UTILISANT

(30) Priority: 11.08.2020 US 202062706353 P
(43) Date of publication of application: 21.06.2023
(73) Proprietor: F. Hoffmann-La Roche AG, 4070 Basel (CH); Roche Diagnostics GmbH, 68305 Mannheim (DE)
(72) Inventor: AYER, Aruna, Pleasanton, California 94588 (US); BERGMANN, Frank, 82377 Penzberg (DE); CRISALLI, Peter, Pleasanton, California 94588 (US); HILLRINGHAUS, Lars, 82377 Penzberg (DE); KHAKSHOOR, Omid, Pleasanton, California 94588 (US); KUCHELMEISTER, Hannes, 82377 Penzberg (DE); NIE, Rongxin, Pleasanton, California 94588 (US); PORTER, Marshall W., Pleasanton, California 94588 (US); SEIDEL, Christoph, 82377 Penzberg (DE); SHIN, Seong-Ho, Pleasanton, California 94588 (US); TAING, Meng, Pleasanton, California 94588 (US); VARGAS, Adolfo, Pleasanton, California 94588 (US)
(74) Representative: Hildebrandt, Martin K. E.
(86) International application number: PCT/EP2021/072112
(87) International publication number: WO 2022/033998

(56) References cited:
- WO-A1-2017/202917
- WO-A1-2019/166457
- WO-A2-2013/154999
- US-B2- 10 975 426
- US-B2- 8 993 234
- CARL W. FULLER ET AL: "Real-time single-molecule electronic DNA sequencing by synthesis using polymer-tagged nucleotides on a nanopore array", PROCEEDINGS OF THE NATIONAL ACADEMY OF SCIENCES, vol. 113, no. 19, 10 May 2016 (2016-05-10), pages 5233 - 5238, XP055295361, ISSN: 0027-8424, DOI: 10.1073/pnas.1601782113
- SHAOYING WANG: "Development of New Biological Nanopores and Their Application for Biosensing and Disease Detection", 1 January 2016 (2016-01-01), XP055600923, Retrieved from the Internet <URL:https://pdfs.semanticscholar.org/a54e/d185ac534fa9fa44426572de4c87d8df6ae6.pdf> DOI: 10.13023/ETD.2016.428

## Description

### BACKGROUND

### I. Field

The present disclosure relates to nucleoside-5'-oligophosphates and uses thereof, and nanopores and uses thereof, including their use in nanopore-based sequencing-by-synthesis systems and methods.

### II. Brief Discussion of Related Art

At their most basic, nanopore sequencing systems comprise a sensing electrode positioned near a nanopore, such that the sensing electrode can detect and record electrochemical characteristics of ions flowing through the nanopore. When relatively large molecules occupy the nanopore, the electrochemical characteristics detected by the sensing electrode change. The identity of the molecule occupying the nanopore can then be determined based upon the change in the electrochemical characteristics, such as a change in current flowing through the nanopore or a decay in measured voltage. An overview of nanopore-based sequencing systems can be found at Wang I and Feng.

The nanopores used in these sequencing systems typically come in one of three flavors: biological nanopores, solid state nanopores, and hybrid nanopores. Biological nanopores are naturally occurring pore-forming molecules, especially proteins such as porins, hemolysins, and the like. Commonly used pore-forming proteins include α-hemolysin (α-HL) protein from *Staphylococcus aureus,* outer membrane protein G (ompG) from *Escherichia coli,* and porin MspA (MspA) from *Mycobacterium smegmatis.* In some cases, like in the case of ompG, the pore is formed from a single subunit of the protein. In other cases, like with α-HL and MspA, the pore is a multi-subunit assembly of the pore-forming protein. For example, α-HL forms a heptameric pore structure and MspA forms an octameric pore structure. Exemplary engineered nanopores based on these proteins can be found at, for example, WO 2016/069806 (α-HL), WO 2017/050728 (α-HL), WO 2017/184866 (α-HL), WO 2018/002125 (α-HL), WO 2012/178097 (α-HL), Gari (ompG), WO 2017/050722 (ompG), US 2015-0080242 (ompG), Manrao (MspA), Pavlenok (MspA), WO 2013/098562 (MspA), US 2014-0309402 (MspA), US 2013-0146457 (MspA), and Wang II (various). Solid state nanopores are pore structures fabricated from synthetic materials, for example, by forming nanometer-sized holes in synthetic membranes. Exemplary materials from which solid state nanopores can be formed include silicon nitrides, silica, alumina, graphene, boron nitride, and molybdenum disulfide. Solid state nanopores are reviewed by Chen, Lee, Wasfi, Wang I, and Feng. Hybrid nanopores incorporate both biological nanopores and solid state nanopores. For example, a biological nanopore (such as an α-HL nanopore) can be inserted into a solid state nanopore. Hybrid nanopores are reviewed by Lee, Wasfi, and Feng.

One approach for nanopore-based nucleic acid sequencing involves threading single stranded nucleic acids directly through the pore (referred to herein as "direct sequencing"). Each nucleotide (or unique combination of nucleotides) generates a unique change in at least one electrochemical characteristic of the pore. These systems frequently use means to control the rate at which the nucleic acid translocates through the pore, such as tethering enzymes to the pore (including polymerases and helicases), removing negatively charged residues from and adding positively charged residues to the pore channel, and adding double stranded regions to the single stranded nucleic acid. Exemplary direct sequencing approaches are discussed by, for example, Feng, Manrao, and Wang I.

Another method involves a sequencing-by-synthesis (SBS) approach by performing a polymerase-catalyzed amplification reaction near an opening of the nanopore with tagged nucleotide polyphosphate molecules. Each tagged nucleotide polyphosphate includes a distinct tag moiety that generates a unique electrochemical signature when it resides in or near the nanopore. As the tagged nucleotide polyphosphates are incorporated into the amplicon, the tag is passed into or near the nanopore, and the electrochemical signature of the tag is recorded. The sequence of the amplicon is derived from the order in which tag moieties enter into the nanopore. Exemplary tag-based SBS approaches and materials for performing such methods are described at, for example, WO 2012-083249, WO 2013/154999, US 2014/0309144, US 9,017,937, WO 2015/148402, WO 2016/069806, WO 2016/144973, US 2016/0222363, US 2016/0333327, WO 2017/050728, WO 2017/184866, WO 2017/050722, US 2017/0267983, US 2018/0245147, US 2018/0094249, WO 2018/002125, and Kumar. Various tags have been proposed for use in such systems, including tags based on polypeptides (such as polylysine tags) and polynucleotides. *See, e.g.,* US 8,652,779 and WO2017042038A1.

### SUMMARY OF THE INVENTION

Disclosed herein are systems for nanopore-based sequencing-by-synthesis of polynucleotides, the system comprising a set of nucleoside-5'-oligophosphates having a positively-charged tag, a nanopore, a nucleic acid polymerase, and a sensing electrode in proximity to the nanopore, the nanopore having a channel that bearing a plurality of negatively charged moieties. The systems presented herein have significantly reduced background and improved signal-to-noise ratio relative to similar systems using negatively-charged tags and pores that lack the additional negative charges.

Also disclosed herein are tag constructs for use in tag-based sequencing-by-synthesis reactions, as well as tagged nucleoside-5'-oligophosphates incorporating the same. In some instances, the tag constructs include a positively charged segment (PCS) at least 5 monomer units in length having a net-positive charge of at least +5 at pH 7.0. In some cases, the PCS comprises a plurality of repeat units and each repeat unit has a net-positive charge and a charge density of at least +0.1.

Also disclosed herein are polypeptides for use in generating a biological nanopore or a biological component of a hybrid nanopore. In some instances, the polypeptide contains sufficient non-native negatively charged moieties such that a nanopore formed therefrom has sufficient negative charge in the channel to mitigate nucleic acid insertion into channel during sequencing-by-synthesis process. In other instances, the polypeptide contains sufficient native negatively charged moieties that are typically absent in nanopores used for nucleic acid sequencing. In some embodiments, the polypeptide is a component of a biological nanopore, such as OmpG, MspA, and alpha-hemolysin nanopores, among others. In some embodiments, the polypeptide has at least 75% sequence identity with any of the sequences selected from the group consisting of SEQ ID NO: 1 and SEQ ID NO: 31-101, with the proviso that the polypeptide comprises at least one non-native negatively charged amino acid at a position corresponding to the entrance region and/or the beta barrel region of a homoheptameric pore formed from SEQ ID NO: 1. In other embodiments, the polypeptide has at least 70% identity to a sequence selected from the group consisting of SEQ ID NO: 23-28, with the proviso that said polypeptide comprises a set of beta strands β1-β14 having at least 90% identity to beta strands β1-β14 of SEQ ID NO: 23 and at least 1 of said beta strands has a non-native negatively charged amino acid. In other embodiments, the polypeptide has at least 70% identity to SEQ ID NO: 29, with the proviso that the polypeptide has an aspartic acid or glutamic acid at any or each of the positions corresponding to D90, D91, D93, D118, D134, and E139 of SEQ ID NO: 29.

Also disclosed herein are methods of sequencing a nucleic acid, comprising: (a) providing a plurality of nanopore sequencing complexes, each nanopore sequencing complex comprising (a1) a sensing electrode, (a2) a nanopore inserted into an electrochemically-resistive barrier in proximity to the sensing electrode, wherein the channel of the nanopore bears a plurality of negatively-charged moieties, (a3) a nucleic acid polymerase associated with the nanopore, (a4) a template nucleic acid complexed with the nucleic acid polymerase, and (a) a set of tagged nucleoside-5'-oligophosphates comprising a positively-charged polymer tag; (b) at the nanopore sequencing complexes, polymerizing a set of nucleoside-5'-oligophosphates into a complementary nucleic acid of the template nucleic acid by a template-dependent nucleic acid amplification reaction catalyzed by the nucleic acid polymerase, wherein the polymer tag of the tagged nucleoside-5'-oligophosphate moves into or in proximity to the channel of the nanopore as the tagged nucleoside-5'-oligophosphate is incorporated into the complementary nucleic acid, and wherein movement of the polymer tag into or in proximity to the channel changes a characteristic of a current flowing through the nanopore; (c) detecting the change in the characteristic of the current flowing through the nanopore caused by the polymer tags with the sensing electrode and recording the change on the computer system; and (d) correlating each recorded change to one of the tagged nucleoside-5'-oligophosphates.

Other details and inventions are described in detail herein.

### BRIEF DESCRIPTION OF THE DRAWINGS

The patent or application file contains at least one drawing executed in color. Copies of this patent or patent application publication with color drawings(s) will be provided by the Office upon request and payment of the necessary fee.
FIG. 1 is an exemplary SBS-nano sequencing run showing the problem of template/primer insertion.
FIG. 2A is a side view of an exemplary α-hemolysin pore. Amino acids residues corresponding to the channel vestibule are in turquoise. Amino acids of the residues corresponding to the constriction site are in red. Channel-facing amino acids of the beta barrel body and exit are in pink.
FIG. 2B is a cross-section of the α-hemolysin pore from FIG. 2A, illustrating the various regions of the nanopore channel.
FIG. 3 is a schematic of a nanopore sequencing cell performing a tag-based SBS method.
FIG. 4A is a ribbon diagram of a monomeric OmpG nanopore. Arrows **401** are beta strands. Loops **402** and turns **403** are also illustrated.
FIG. 4B is a cross-section of a monomeric OmpG nanopore. In addition to beta strand regions **401,** loops **402,** and turns **403,** a restriction site **404** is illustrated.
FIG. 5A shows the different regions of monomeric OmpG polypeptide according to SEQ ID NO: 23.
FIG. 5B shows the different regions of monomeric OmpG polypeptide according to SEQ ID NO: 24.
FIG. 5C shows the different regions of monomeric OmpG polypeptide according to SEQ ID NO: 25.
FIG. 5D shows the different regions of monomeric OmpG polypeptide according to SEQ ID NO: 26.
FIG. 5E shows the different regions of monomeric OmpG polypeptide according to SEQ ID NO: 27.
FIG. 5F shows the different regions of monomeric OmpG polypeptide according to SEQ ID NO: 28.
FIG. 6 is a top view of an embodiment of a nanopore sensor chip.
FIG. 7 illustrates an embodiment of an example nanopore cell in a nanopore sensor chip.
FIG. 8 illustrates an embodiment of a nanopore cell performing nucleotide sequencing using the Nano-SBS technique.
FIG. 9 illustrates an embodiment of an electric circuit in a nanopore cell.
FIG. 10 shows example data points captured from a nanopore cell during bright periods and dark periods of AC cycles.
FIG. 11 is a plot of expression levels (x-axis, units in A280) against net charge of monomer at pH 7.0 (y-axis).
FIG. 12 shows SDS-PAGE gels of self-oligomerization of monomeric α-hemolysin preparations. Solid boxes show expected migration rate of heptamers. Dashed boxes show expected migration of monomers.
FIG. 13A shows SDS-PAGE gels of self-oligomerization of monomeric α-hemolysin preparation G1637 in the presence of either a 2-(N-morpholino)ethanesulfonic acid (MES) buffer composition or a buffer composition containing 3M trimethylamine N-oxide (TMAO). The lanes illustrated with "M" are preparations before oligomerization treatment. The lanes illustrated with "O" are preparations after oligomerization treatment. Solid boxes show expected migration rate of heptamers. Dashed boxes show expected migration of monomers.

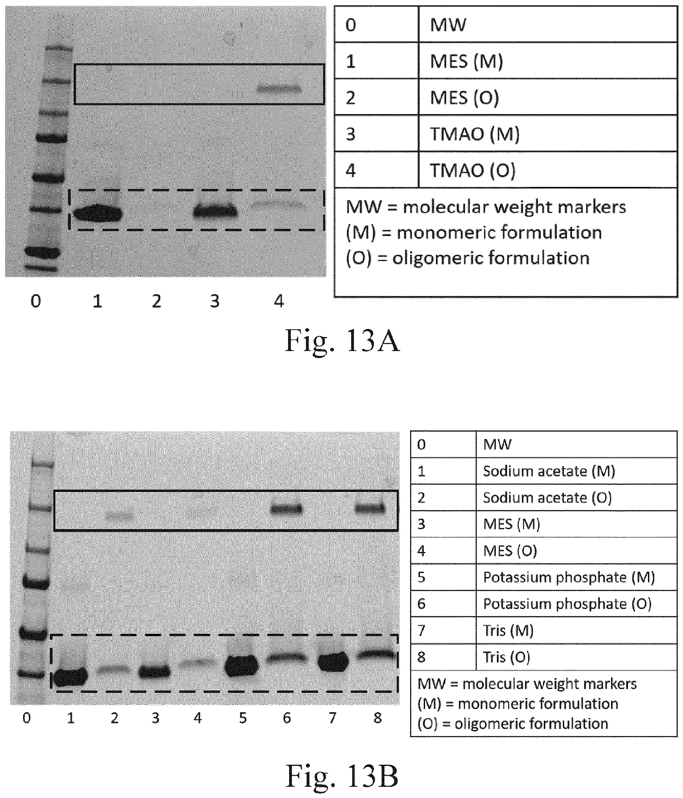

FIG. 13B shows SDS-PAGE gels of self-oligomerization of monomeric α-hemolysin preparation G1637 in the presence of: (lanes 1 & 2) 50 mM sodium acetate, 50 mM NaCl, and 3M TMAO, pH 4.8; (lanes 3 & 4) 50 mM MES, 50 mM NaCl, and 3M TMAO, pH 6.0; (lanes 5 & 6) 50 mM potassium phosphate, 50 mM NaCl, and 3M TMAO, pH 7.4; and (lanes 7 & 8) 50 mM Tris, 50 mM NaCl, and 3M TMAO, pH. 8.0. The lanes illustrated with "M" are preparations before oligomerization treatment. The lanes illustrated with "O" are preparations after oligomerization treatment. Solid boxes show expected migration rate of heptamers. Dashed boxes show expected migration of monomers.
FIG. 14A illustrates oligomerization of monomer G1147 with a SpyCatcher tagged wild-type (WT) α-hemolysin monomer. As indicated by the arrows, 1:6 heteroheptamers (WT:G1147) were formed, as well as 2:5 heteroheptamers.
FIG. 14B illustrates oligomerization with various ratios of G1515 to WT, including examples in which G1515 is in excess (ratios 3:1, 4:1, 5:1, 6:1, 7:1, 8:1, and 9:1), in which G1515 and WT are present in equivalent amounts (ratio 1:1), and in which WT monomer is in excess (ratios of 1:3, 1:5, 1:7, and 1:9). Arrows indicate the expected migration of 1:6 heteroheptamers (1 WT-GFP-SC monomer to 6 G1515 monomers) and 2:5 heteroheptamers (2 WT-GFP-SC monomer to 5 G1515 monomers).
FIG. 14C show a cation exchange chromatography run of a heptamer generated from a 9:1 mix of G1515 to WT.
FIG. 14D is an SDS-PAGE gel of heptamer fractions generated from a 9:1 mix of G1515 to WT (+/- SC-GFP). P1-P6 correspond to the labeled fractions from Fig. 14C. Lanes labeled with a "-" are unlabeled heptamers. Lanes labeled with a "+" are SC-GFP labeled. Arrows indicate the expected migration of 1:6 and 2:5 heptamers (i.e. 1- or 2-copies of the WT-SC-GFP labeled components).
FIG. 14E is a cation exchange chromatography run (bottom graph) of a heptamer generated from a 9:1 mix of G1713 to WT, as well as an SDS-PAGE gel (top image) of the resulting fractions P1-P4. Lanes of the SDS-PAGE gel labeled with a "-" are unlabeled heptamers. Lanes labeled with a "+" illustrate SC-GFP labeled heptamers. Arrows show expected migration of 1:6 and 2:5 heptamers.
FIG. 14F is an SDS-PAGE gel of oligomers further purified from Fraction P2 from Fig. 14E. Lanes of the SDS-PAGE gel labeled with a "-" are unlabeled heptamers. Lanes labeled with a "+" illustrate SC-GFP labeled heptamers. Arrows indicate the expected migration of 1:6 and 2:5 heptamers.
FIG. 14G is an SDS-PAGE gel of purified oligomers formed from G1752 + WT. Lanes of the SDS-PAGE gel labeled with a "-" are unlabeled heptamers. Lanes labeled with a "+" illustrate SC-GFP labeled heptamers. Arrows indicate the expected migration of 1:6 and 2:5 heptamers.
FIG. 14H is an SDS-PAGE gel of purified oligomers formed from G1753 + WT (left 2 columns) and G1933 + WT (right 2 columns). Lanes of the SDS-PAGE gel labeled with a "-" are unlabeled heptamers. Lanes labeled with a "+" illustrate SC-GFP labeled heptamers. Arrows indicate the expected migration of 1:6 and 2:5 heptamers.
FIG. 15A is a graph showing the distributions of tag levels against the probability density using a DOPhPC membrane. X-axis is the fraction of the open channel level recorded when using the indicated tag. Y-axis is reported as the probability density (i.e. percentage of events having the recorded tag level).
FIG. 15B is a graph showing the distributions of tag levels against the probability density using a DPhPC membrane. X-axis is the fraction of the open channel level recorded when using the indicated tag. Y-axis is reported as the probability density (i.e. percentage of events having the recorded tag level).
FIG. 16A illustrates the general structure of an exemplary tagged deoxyribonucleoside hexaphosphate (labeled "dN6P"). A positively charged tag moiety (labeled "Tag") is attached to the dN6P via a chemical linkage between the tag and the terminal phosphate group of the dN6P ("Linkage").
FIG. 16B illustrates an exemplary synthesis scheme for tagged dN6P.
FIG. 16C illustrates an exemplary synthesis scheme for tagged dT6P bearing a tag according to SEQ ID NO: 110 with a linkage according to Formula 7.

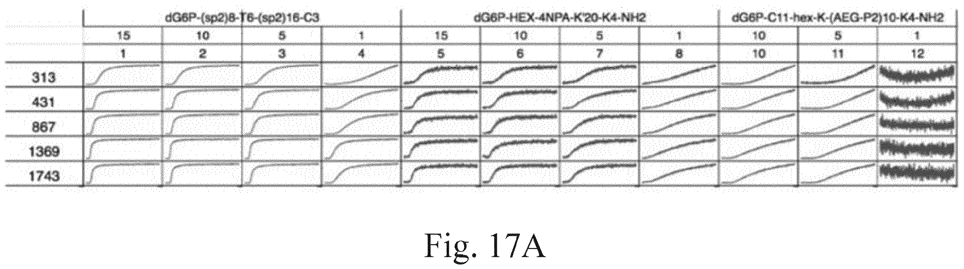

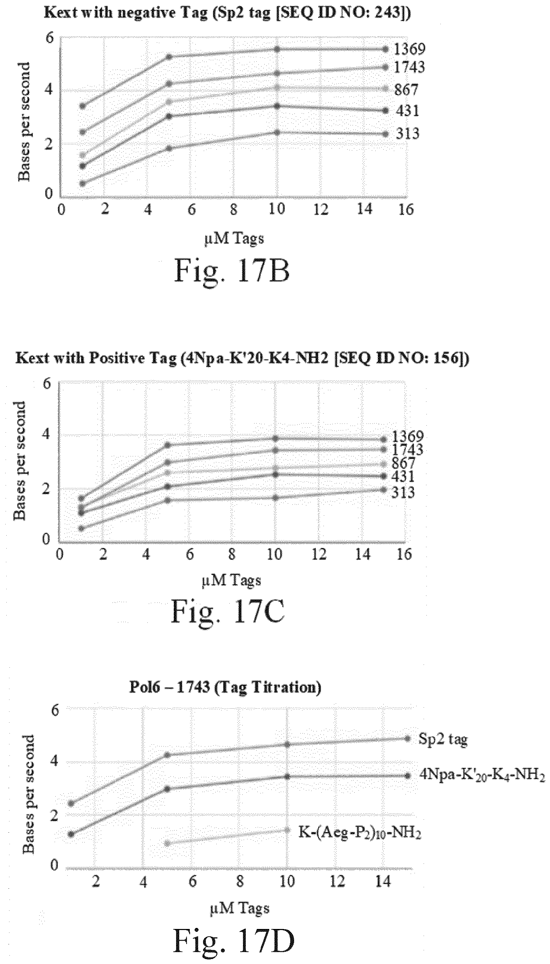

FIG. 17A shows fluorescence intensity (y-axis) versus time (x-axis) for each tag at 15, 10, 5, and 1 µM concentration with Pol6 variants 313, 431, 867, 1369, and 1743. The maximum fluorescence intensity of the positive tags is approximately 10% of the maximum fluorescence intensity of the negative tag. The fluorescence displacement assay was repeated in which each of dA6P, dT6P, dC6P, and dG6P were tagged, and the. Fig. 17B illustrates the Kₑₓₜ using the negative tag, Fig. 17C illustrates the Kₑₓₜ using the 4Npa-K'₂₀-K₄-NH₂ tag, and Fig. 17D illustrates Kₑₓₜ of Pol6 1743 at various concentrations of tagged dN6P.
FIG. 17B shows the Kₑₓₜ of each of Pol6 variants 313, 431, 867, 1369, and 1743 with 1, 5, 10, and 15 µM of a negatively-charged tag (SP2).
FIG. 17C shows the Kₑₓₜ of each of Pol6 variants 313, 431, 867, 1369, and 1743 with 1, 5, 10, and 15 µM of a positively-charged tag.
FIG. 17D shows the Kₑₓₜ of Pol6 variants 1743 with 1, 5, 10, and 15 µM of Sp2 tag, and two different positively charged tags.
FIG. 18 shows the results of a sequencing run using dN6P according to Table 13 that bear the same tag (P-C-83, P-A-82, P-G-80, and P-T-81). The Y-axis is the signal level recorded and the X-axis is time in seconds. Two replicates are shown. Graph A and Graph C show the full sequencing runs of the two replicates. Graph B is a subset of time frame from graph A (running from -381.25 seconds to <382.75 seconds). Graph D is a subset of the time frame from graph D (running from -360 seconds to ~410 seconds).

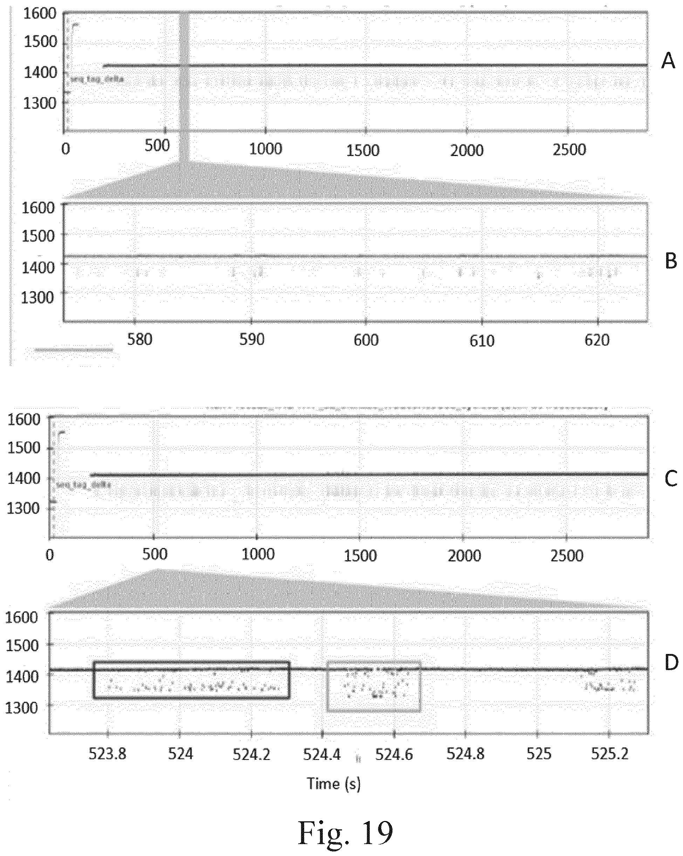

FIG. 19 shows the results of a sequencing run using dN6P according to Table 13 that bear the two different tags tag (P-C-74 & P-A-78 (bearing the same tag as one another), P-G-76 & P-T-70 (bearing the same tag as one another, but different from the tag of P-C-74 & P-A-78)). The Y-axis is the signal level recorded and the X-axis is time in seconds. Two replicates are shown. Graph A and Graph C show the full sequencing runs of the two replicates. Graph B is a subset of time frame from graph A (running from -570 seconds to -625 seconds). Graph D is a subset of the time frame from graph D (running from -523.6 seconds to -525.4 seconds). The blue box and the yellow box are the different threaded levels of different tags respectively within the nanopore during a sequencing experiment.

### DETAILED DESCRIPTION OF THE INVENTION

The invention will now be described in detail by way of reference only using the following definitions and examples.

Unless defined otherwise herein, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this invention. Practitioners are particularly directed to Sambrook et al., 1989, and Ausubel FM et al., 1993, for definitions and terms of the art. It is to be understood that this invention is not limited to the particular methodology, protocols, and reagents described, as these may vary.

Unless otherwise indicated, nucleic acids are written left to right in 5' to 3' orientation; amino acid sequences are written left to right in amino to carboxy orientation, respectively.

The headings provided herein are not limitations of the various aspects or embodiments of the invention, which can be had by reference to the specification as a whole. Accordingly, the terms defined immediately below are more fully defined by reference to the specification as a whole.

### I. Terms

**Active nanopore sequencing** complex: A nanopore sequencing complex at which the polymerase is catalyzing template-based polymerization of the tagged nucleoside-5'-oligophosphates and the sensing electrode is detecting capture events at the nanopore.

**Alpha-hemolysin:** As used herein, "alpha-hemolysin," "α-hemolysin," and "α-BL" are used interchangeably and refer to the monomeric protein that self-assembles into a heptameric water-filled transmembrane channel (i.e., nanopore). Depending on context, the term may also refer to the transmembrane channel formed by seven monomeric proteins.

**Amidated amino acid:** An amino acid in which the carboxy terminus has been amidated.

**Amino** acid: As used herein, the term "amino acid" refers to any compound capable of forming a peptide bond having the general structure HₐN-X-COOH, wherein X is an alkyl chain (optionally substituted) at least one carbon in length, a is 1 or 2 with the proviso that when a is 1, HN-X is a cyclic structure. This explicitly includes but is not limited to α-amino acids, β-amino acids, γ-amino acids, and δ-amino acids and L- and D-enantiomers thereof. The term "amino acid" is used interchangeably with "amino acid residue," and may refer to a free amino acid and/or to an amino acid residue of a peptide. It will be apparent from the context in which the term is used whether it refers to a free amino acid or a residue of a peptide. Unless otherwise indicated, all amino acid sequences are represented herein by formulae whose left and right orientation is in the conventional direction of amino-terminus to carboxy-terminus. Unless otherwise indicated, all amino acid residues of an amino acid sequence recited using the standard 1 or 3 letter amino acid code shall refer to the L-enantiomer of the corresponding α-amino acid.

**Amino acid analog:** As used herein, the term "amino acid analog" shall refer to any chemical structure capable of forming peptide-like linkages and having the same general structure of an amino acid, except that one or more of the carbons of the X alkyl chain is replaced by another moiety (such as a nitrogen or a phenyl group) and/or the sidechain is located at a non-carbon position along the backbone (such as at the amino terminus). Exemplary amino acid analogs include peptoids, azapeptides, oligoureas, arylamides, oligohydrazides, and the like.

**Amino acid derivative:** In some cases, the specification refers to a "derivative of" a specific amino acid or similar constructions. This shall be interpreted to refer to the same amino acid having a specific class of chemical modification to the sidechain. For example, the term "positively charged derivative of an aliphatic amino acid" shall refer to an aliphatic amino acid in which the aliphatic side chain has been modified to have a positively-charged moiety. Likewise, the term "positively charged derivative of an aromatic amino acid" shall refer to an aromatic amino acid in which the side chain has been modified to have a positively-charged moiety.

**Base Pair (bp):** As used herein, base pair refers to a partnership of adenine (A) with thymine (T), adenine (A) with uracil (U) or of cytosine (C) with guanine (G) in a double stranded nucleic acid.

**Capture event:** An insertion of a polymer tag into a nanopore that is sufficient to generate a change in an characteristic of ionic current flowing through the nanopore such that the change is detectable by a sensing electrode.

**Complementary:** As used herein, the term "complementary" refers to the broad concept of sequence complementarity between regions of two polynucleotide strands or between two nucleotides through base-pairing. It is known that an adenine nucleotide is capable of forming specific hydrogen bonds ("base pairing") with a nucleotide which is thymine or uracil. Similarly, it is known that a cytosine nucleotide is capable of base pairing with a guanine nucleotide.

**Expression cassette:** An "expression cassette" or "expression vector" is a nucleic acid construct generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter.

**Heterologous:** A "heterologous" nucleic acid construct or sequence has a portion of the sequence which is not native to the cell in which it is expressed. Heterologous, with respect to a control sequence, refers to a control sequence (i.e. promoter or enhancer) that does not function in nature to regulate the same gene the expression of which it is currently regulating. Generally, heterologous nucleic acid sequences are not endogenous to the cell or part of the genome in which they are present, and have been added to the cell, by infection, transfection, transformation, microinjection, electroporation, or the like. A "heterologous" nucleic acid construct may contain a control sequence/DNA coding sequence combination that is the same as, or different from a control sequence/DNA coding sequence combination found in the native cell.

**Host cell:** By the term "host cell" is meant a cell that contains a vector and supports the replication, and/or transcription or transcription and translation (expression) of the expression construct. Host cells for use in the present invention can be prokaryotic cells, such as E. coli or Bacillus subtilus, or eukaryotic cells such as yeast, plant, insect, amphibian, or mammalian cells. In general, host cells are prokaryotic, e.g., E. coli.

**Isolated:** An "isolated" molecule is a biomolecule that is separated from at least one other molecule with which it is ordinarily associated, for example, in its natural environment.

**Mutation:** As used herein, the term "mutation" refers to a change introduced into a parental sequence, including, but not limited to, substitutions, insertions, and/or deletions (including truncations). The consequences of a mutation include, but are not limited to, the creation of a new character, property, function, phenotype or trait not found in the protein encoded by the parental sequence.

**Nanopore:** The term "nanopore," as used herein, generally refers to a pore, channel or passage formed or otherwise provided in an electrically-resistive barrier (such as a lipid membrane, a silicon layer, a polymeric layer, or a graphene layer) through which an ionic current may pass. Unless otherwise stated, the generic term "nanopore" shall include biological nanopores, solid state nanopores, and hybrid nanopores.

**Nanopore sequencing complex:** A site at which a nanopore-based sequencing method may be performed, generally comprising at least a nanopore through which ions may flow and a sensing electrode configured to detect a characteristic of the ion current flowing through the nanopore (such as voltage decay). In the context of a tag-based SBS nucleotide sequencing system or method, the nanopore sequencing complex comprises at least (a) a nanopore through which ions may flow; (b) a nucleic acid polymerase attached to or otherwise associated with the nanopore in a configuration that enables the polymerase to catalyze template-based polymerization of tagged nucleoside-5'-oligophosphates such that a polymer tag of the nucleoside-5'-oligophosphate can insert into the channel of the nanopore while the nucleoside-5'-oligophosphate is being polymerized; and (c) a sensing electrode configured to detect a characteristic of the ion current flowing through the nanopore.

**Native amino acid:** Any amino acid of an amino acid sequence that, when aligned with a reference amino acid sequence, is the same as the amino acid occupying the corresponding position of the reference sequence.

**Non-native negatively-charged moiety:** A component of a nanopore bearing a net-negative charge at pH 7.0 that is not found in a reference structure. For example, where the nanopore includes a polypeptide, a "non-native negatively charged amino acid" would be any amino acid having a side chain with a net-negative charge at pH 7.0 that represents a substitution or an insertion at a particular position relative to a reference amino acid sequence, or a represents a chemical modification of the side chain of a native amino acid that results in a net-negative charge at pH 7.0.

**Nucleic Acid Molecule:** The term "nucleic acid molecule" includes RNA, DNA and cDNA molecules. It will be understood that, as a result of the degeneracy of the genetic code, a multitude of nucleotide sequences encoding a given protein such as α-hemolysin and/or variants thereof may be produced. The present invention contemplates every possible variant nucleotide sequence.

**Peptide:** The terms "peptide" and "peptide linkage" shall refer to any backbone linkage between two amino acids and/or amino acid analogs resulting from a condensation reaction between a carboxylic acid moiety of one amino acid or amino acid analog and an amino group of a second amino acid or amino acid analog. Unless otherwise clear from the context, these terms shall be understood in all instances as encompassing (but not limited to) linkages between α-amino acids, β-amino acids, γ-amino acids, δ-amino acids, and combinations thereof, as well as linkages between backbone carboxylic acid moieties and side chain amino moieties (such as with ε-linked lysine).

**Peptide chain:** The term "peptide chain" shall refer to any sequence of two or more amino acids and/or amino acid analogs linked by peptide linkages.

**Peptidomimetic:** The terms "peptidomimetic" and "peptidomimetic linkage" shall refer to backbone linkages between two amino acid analogs or between an amino acid and an amino acid analog, including but not limited to peptoids (amino acids in which the sidechain is attached to the amino group), azapeptides (replacement of the α-carbon with a nitrogen), oligourea (peptide linkage replaced by a urea linkage), arylamides, oligohydrazides, and the like.

**Peptidomimetic chain:** The term "peptidomimetic chain" shall refer to any sequence of two or more amino acids and/or amino acid analogs linked by peptidomimetic backbone linkages.

**Polypeptide:** Unless stated otherwise or unless otherwise clear based on the context of the disclosure, the phrase "polypeptide" shall be understood in its broadest sense and shall encompass any sequence of two or more amino acids and/or amino acid analogs linked by peptide linkages and/or peptidomimetic linkages.

**Promoter:** As used herein, the term "promoter" refers to a nucleic acid sequence that functions to direct transcription of a downstream gene. The promoter will generally be appropriate to the host cell in which the target gene is being expressed. The promoter together with other transcriptional and translational regulatory nucleic acid sequences (also termed "control sequences") are necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

**Proteinogenic amino acid:** The L-enantiomer of any genetically encoded α-amino acid.

**Purified:** As used herein, "purified" means that a molecule is present in a sample at a concentration of at least 95% by weight, or at least 98% by weight of the sample in which it is contained.

**Tag:** As used herein, the term "tag" refers to a nanopore-detectable moiety that may be atoms or molecules, or a collection of atoms or molecules. A tag may provide an optical, electrochemical, magnetic, or electrostatic (e.g., inductive, capacitive) signature, which signature may be detected with the aid of a nanopore. Typically, when a nucleotide is attached to the tag it is called a "Tagged Nucleotide."

**Time-To-Thread:** The term "time to thread" or "TTT" means the time it takes a tag to thread into the barrel of the nanopore after associating with a nucleic acid polymerase associated with the nanopore.

**Variant:** As used herein, the term "variant" of a reference polypeptide or a nucleic acid is any such molecule that contains at least one molecular change relative to the reference molecule.

**Vector:** As used herein, the term "vector" refers to a nucleic acid construct designed for transfer between different host cells. An "expression vector" refers to a vector that has the ability to incorporate and express heterologous DNA fragments in a foreign cell. Many prokaryotic and eukaryotic expression vectors are commercially available. Selection of appropriate expression vectors is within the knowledge of those having skill in the art.

**Percent homology:** The term "% homology" is used interchangeably herein with the term "% identity" herein and refers to the level of nucleic acid or amino acid sequence identity between the nucleic acid sequence that encodes any one of the inventive polypeptides or the inventive polypeptide's amino acid sequence, when aligned using a sequence alignment program. For example, as used herein, 80% homology means the same thing as 80% sequence identity determined by a defined algorithm, and accordingly a homologue of a given sequence has greater than 80% sequence identity over a length of the given sequence. Exemplary levels of sequence identity include, but are not limited to, 80, 85, 90, 95, 98% or more sequence identity to a given sequence, e.g., the coding sequence for any one of the inventive polypeptides, as described herein. Exemplary computer programs which can be used to determine identity between two sequences include, but are not limited to, the suite of BLAST programs, e.g., BLASTN, BLASTX, and TBLASTX, BLASTP and TBLASTN, publicly available on the Internet. See also, Altschul, et al., 1990 and Altschul, et al., 1997. Sequence searches are typically carried out using the BLASTN program when evaluating a given nucleic acid sequence relative to nucleic acid sequences in the GenBank DNA Sequences and other public databases. The BLASTX program is may be used for searching nucleic acid sequences that have been translated in all reading frames against amino acid sequences in the GenBank Protein Sequences and other public databases. Both BLASTN and BLASTX are run using default parameters of an open gap penalty of 11.0, and an extended gap penalty of 1.0, and utilize the BLOSUM-62 matrix. (See, e.g., Altschul, S. F., et al., Nucleic Acids Res. 25:3389-3402, 1997.) Unless stated otherwise, reference to an alignment of two amino acid sequences shall refer an alignment obtainable using the EMBOSS Needle pairwise sequence alignment tool with the BLOSUM62 matrix, GAP OPEN setting of 10, GAP EXTEND setting of 0.5, END GAP PENALTY setting of "false", END GAP OPEN setting of 10, and END GAP EXTEND setting of 0.5 (available from EMBL-EBI).

**Foldamer:** An oligomer with a characteristic tendency to fold into a specific structure in solution that is stabilized by non-covalent interactions between non-adjacent subunits.

**Monomer subunit:** A structural subunit of a multimeric protein complex. For example, a heptameric α-hemolysin pore comprises seven α-hemolysin monomeric subunits. A monomeric subunit that has not been oligomerized into a multimeric subunit is referred to herein as a "non-oligomerized monomeric subunit."

### II. Development Background

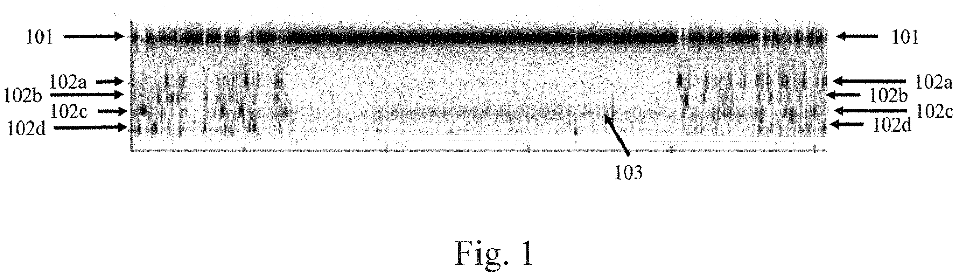

FIG. 1 illustrates a standard tag-based sequencing-by-synthesis (SBS) run using an α-hemolysin nanopore and negatively-charged tags. The dark band at the top is the open channel level **101** and a tag occupying the channel of the nanopore is recorded as a change in signal (in this case, conductance level) relative to open channel, with different tags resulting in different changes in signal **102a-102d.** However, the present inventors have observed that a persistent background band is frequently observed **103.** The increased background results in convoluted tag signals and signal processing, which increases as the threading rate increases. This inherently limits the throughput and accuracy of tag-based SBS.

The present disclosure demonstrates that pairing a set of nucleoside-5'-oligophosphates bearing a positively-charged tag with a nanopores having a channel with more negative charges than nanopores typically used for nanopore-based sequencing mitigates this issue. Without being bound by theory, the aberrant pattern may result at least in part from threading of the template nucleic acid and/or primer through the nanopore, and that addition of negative charges to the channel may provide a repulsive force against the negatively-charged template and primer.

### III. Alpha-hemolysin polypeptides and associated nucleic acids and nanopores

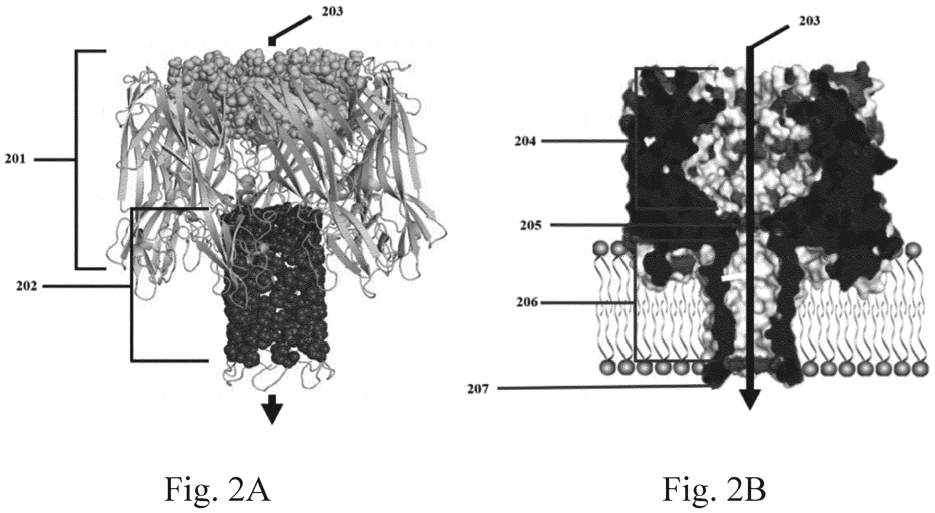

α-HL nanopores are heptameric structures formed from 7 monomeric subunits of the α-HL polypeptide from *Staphylococcus aureus.* Various approaches for engineering α-HL nanopores for use in nanopore-based sequencing can be found at, for example, Ayub, Wang II, WO 2014/100481, WO 2016/069806, WO 2017/050718, WO 2017/184866, and WO 2018/002125. As illustrated at FIG. 2A, α-HL nanopores have a cap region 201 and a beta barrel region 202, with a channel 203 extending axially through the cap and stem regions. FIG. 2B is a cross-section of the α-HL nanopore showing the channel 203, which can be divided into a vestibule 204, a constriction site 205, a beta barrel body 206, and a beta barrel exit 207. References herein to "beta barrel region" includes each of the constriction site 205, the beta barrel body 206, and the beta barrel exit 207. References herein to "α-HL nanopore" shall refer to heptameric pores of 7 α-HL monomeric subunits.

### IIIA. α-HL polypeptides having at least one non-native negatively charged amino acid

In an aspect, the present disclosure relates to polypeptides useful for forming α-HL nanopores. The polypeptides disclosed herein comprise one or more α-HL monomeric subunits having at least one non-native negatively-charged amino acid (such as aspartic acid or glutamic acid) at a position corresponding to the vestibule region and/or the beta barrel region of the α-HL nanopore. An amino acid sequence corresponding to a wild-type α-HL monomeric subunit can be found at SEQ ID NO: 1. Unless otherwise indicated, all amino acid numbering relating to α-HL monomeric subunits are with reference to SEQ ID NO: 1. When reference is made to an α-HL monomeric subunit "comprising substitution at position X" or "comprising a substitution X#Y" it shall be understood to mean that the monomeric subunit amino acid sequence, when aligned with SEQ ID NO: 1, has a substitution at the position corresponding to the recited position of SEQ ID NO: 1. As used herein, a "non-native amino acid" is an amino acid at a position of the monomeric subunit amino acid sequence that represents a substitution or insertion when aligned with SEQ ID NO: 1. In an embodiment, the polypeptides comprise at least one α-HL monomeric subunits having at least 75% identity, at least 80% identity, at least 85% identity, at least 90% identity, at least 91% identity, at least 92% identity, at least 93% identity, at least 94% identity, or at least 95% identity with SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively charged amino acid (such as aspartic acid or glutamic acid) at a position corresponding to a position of SEQ ID NO: 1 listed in Table 1 or Table 2. Table 1 lists the solvent-facing amino acid residues that are located at the entrance 304, constriction zone 305, or beta barrel 306 when a monomeric subunit consisting of SEQ ID NO: 1 is self-assembled into a homoheptameric α-HL nanopore in the presence of DPhPC in aqueous solution of 20mM Tris-HCl pH 8.0, 200 mM NaCl at 37 °C. "#" indicates the position within SEQ ID NO: 1, "AA" indicates the amino acid at the recited position of SEQ ID NO: 1, and "Location" indicates the sub-region of the α-HL nanopore at which the amino acid is located.

**Table 1**

| # | **AA** | **Location** | **#** | **AA** | **Location** | **#** | **AA** | **Location** |
|---|---|---|---|---|---|---|---|---|
| 1 | Ala | Entrance | 17 | Asn | Entrance | 125 | Thr | Beta Barrel |
| 2 | Asp | Entrance | 18 | Thr | Entrance | 129 | Thr | Exit |
| 3 | Ser | Entrance | 19 | Thr | Entrance | 130 | Gly | Exit |
| 4 | Asp | Entrance | 20 | Val | Entrance | 131 | Lys | Exit |
| 5 | Ile | Entrance | 21 | Lys | Entrance | 133 | Gly | Beta Barrel |
| 6 | Asn | Entrance | 22 | Thr | Entrance | 135 | Leu | Beta Barrel |
| 7 | Ile | Entrance | 46 | Lys | Entrance | 137 | Gly | Beta Barrel |
| 8 | Lys | Entrance | 47 | Asn | Entrance | 139 | Asn | Beta Barrel |
| 9 | Thr | Entrance | 106 | Ser | Entrance | 141 | Ser | Beta Barrel |
| 10 | Gly | Entrance | 111 | Glu | Constriction | 143 | Gly | Beta Barrel |
| 11 | Thr | Entrance | 113 | Met | Constriction | 145 | Thr | Beta Barrel |
| 12 | Thr | Entrance | 115 | Thr | Beta Barrel | 147 | Lys | Constriction |
| 13 | Asp | Entrance | 117 | Thr | Beta Barrel | 149 | Val | Entrance |
| 14 | Ile | Entrance | 119 | Gly | Beta Barrel | 239 | Ser | Entrance |
| 15 | Gly | Entrance | 121 | Asn | Beta Barrel | | | |
| 16 | Ser | Entrance | 123 | Asn | Beta Barrel | | | |

Table 2 lists the solvent-facing amino acid residues (other than aspartic acid or glutamic acid) that are located at the entrance 204, constriction zone 205, beta barrel body 206, and beta barrel exit 207 when an α-HL monomeric subunit consisting of SEQ ID NO: 1 is self-assembled into a homoheptameric α-HL nanopore in the presence of DPhPC in aqueous solution of 20mM Tris-HCl pH 8.0, 200 mM NaCl at 37 °C:

**Table 2**

| # | **AA** | **Location** | **#** | **AA** | **Location** | **#** | **AA** | **Location** |
|---|---|---|---|---|---|---|---|---|
| 1 | Ala | Entrance | 18 | Thr | Entrance | 125 | Thr | Beta Barrel |
| 3 | Ser | Entrance | 19 | Thr | Entrance | 129 | Thr | Exit |
| 5 | Ile | Entrance | 20 | Val | Entrance | 130 | Gly | Exit |
| 6 | Asn | Entrance | 21 | Lys | Entrance | 131 | Lys | Exit |
| 7 | Ile | Entrance | 22 | Thr | Entrance | 133 | Gly | Beta Barrel |
| 8 | Lys | Entrance | 46 | Lys | Entrance | 135 | Leu | Beta Barrel |
| 9 | Thr | Entrance | 47 | Asn | Entrance | 137 | Gly | Beta Barrel |
| 10 | Gly | Entrance | 106 | Ser | Entrance | 139 | Asn | Beta Barrel |
| 11 | Thr | Entrance | 113 | Met | Constriction | 141 | Ser | Beta Barrel |
| 12 | Thr | Entrance | 115 | Thr | Beta Barrel | 143 | Gly | Beta Barrel |
| 14 | Ile | Entrance | 117 | Thr | Beta Barrel | 145 | Thr | Beta Barrel |
| 15 | Gly | Entrance | 119 | Gly | Beta Barrel | 147 | Lys | Constriction |
| 16 | Ser | Entrance | 121 | Asn | Beta Barrel | 149 | Val | Entrance |
| 17 | Asn | Entrance | 123 | Asn | Beta Barrel | 239 | Ser | Entrance |

Additionally or alternatively, a non-native negatively charged amino acid may be placed on the N-terminal side of position 1 (termed hereafter "position 0"). In some embodiments, a non-native negatively charged amino acid is at a sufficient number of positions of Table 1 or Table 2 (and/or at position 0) to obtain a channel having a net-negative charge when formed into a homoheptameric nanopore. As used in the context of α-HL polypeptides, the "net charge of the channel" is the sum of the charges of all solvent-facing amino acid side chains within the channel of a homoheptameric α-HL nanopore formed from the monomeric subunit in the presence of DPhPC in aqueous solution of 20mM Tris-HCl pH 8.0, 200 mM NaCl at 37 °C. In an embodiment, the at least one non-native negatively charged amino acid is at a position corresponding to a position of SEQ ID NO: 1 selected from the group consisting of 0, A1, K8, T9, G10, N17, K46, N47, M113, T117, N121, T129, G130, K131, K147, and V149. In some embodiments, the monomeric subunit comprises a non-native negatively charged amino acids at a sufficient number of positions of SEQ ID NO: 1 selected from the group consisting of 0, A1, K8, T9, G10, N17, K46, N47, M113, T117, N121, T129, G130, K131, K147, and V149 to obtain a channel having a net-negative charge.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at a position of the monomeric subunit corresponding to the entrance of the channel. In addition to helping repel the template and/or primer nucleic acid, negative charge(s) at the entrance have the added effect of increasing the arrival rate and/or threading rate of positively-charged tags relative to a homoheptameric α-HL nanopore in which the monomeric subunits consist of SEQ ID NO: 1. As used herein, the "arrival rate" of the α-HL nanopore is a measure of frequency with which the a-HL nanopore captures the tag of a biotinylated tag molecule. For example, arrival rate can be determined by obtaining a chip having a plurality of the pore of interest inserted in the bilayer, flowing a streptavidin-biotin-TAG across the chip, and measuring the average time between capture events at each of the plurality of pores (typically at a very low AC modulation frequency, such as ~50Hz). The arrival rate is the average time between events across all pores. As used herein, the "threading rate" of the α-HL nanopore is the rate at which a tag bound to an active nanopore sequencing complex is captured by the pore. For example, in experiments done with active nanopore sequencing complexes at >1khz, the rate of tag capture is determined for each modulation cycle. Typically, the arrival rate of a pore reasonable correlates with the threading rate of the pore in an active nanopore sequencing complex. Exemplary pores having improved (i.e. faster) tag capture in the present systems include pores comprising monomeric subunits having a non-native negatively charged amino acid at a position corresponding to one or more of 0, A1, S3, I5, N6, I7, K8, T9, G10, T11, T12, I14, G15, S16, N17, T18, T19, V20, K21, T22, K46, N47, S106, and V149 of SEQ ID NO: 1. In an embodiment, the monomeric subunit(s) have a non-native negatively charged amino acid at 1, 2, 3, 4, 5, 6, or 7 positions selected from the group consisting of 0, A1, S3, I5, N6, I7, K8, T9, G10, T11, T12, I14, G15, S16, N17, T18, T19, V20, K21, T22, K46, N47, S106, and V149 of SEQ ID NO: 1. In an embodiment, the non-native negatively charged amino acid(s) are at 1, 2, 3, 4, 5, 6, or 7 positions selected from the group consisting of A1, K8, T9, G10, N17, K46, and N47 of SEQ ID NO: 1. In another embodiment, the non-native negatively charged amino acid(s) are at 1, 2, or 3 positions selected from the group consisting of 0, A1, N17, and N47 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at a position corresponding to the beta barrel. Non-native negative charges introduced at this site have the added effect of increasing the threading rate of positively-charged tags relative to an α-HL nanopore comprising of 7 monomeric subunits having 100% identity to SEQ ID NO: 1. As used herein, "at the beta barrel" includes residues at the constriction site, residues in the body of the beta barrel, and residues at the exit of the beta barrel. Exemplary residues at the beta barrel that could be modified to introduce a negative charge include M113, T115, T117, N121, T129, G130, K131, and K147 of SEQ ID NO: 1. As an example, a monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 is provided, wherein the monomeric subunit has an aspartic acid or a glutamic acid at a position corresponding to one or more of M113, T115, T117, N121, T129, G130, K131, and K147 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at a position corresponding to the beta barrel exit. Exemplary residues at the beta barrel exit that could be modified to introduce a net negative charge include T129, G130, and K131 of SEQ ID NO: 1. As an example, a monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 is provided, wherein the monomeric subunit has an aspartic acid or a glutamic acid at a position corresponding to T129, G130, and/or K131 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at one or more positions corresponding to the constriction site. Non-native negative charges introduced at this site have the added effect of increasing the threading rate of positively-charged tags relative to an α-HL nanopore comprising of 7 monomeric subunits having 100% identity to SEQ ID NO: 1. Exemplary residues that could be modified to introduce a negative charge at the constriction site include M113 and K147 of SEQ ID NO: 1. As an example, the monomeric subunit has at least 75% identity, at least 80% identity, at least 85% identity, at least 90% identity, at least 91% identity, at least 92% identity, at least 93% identity, at least 94% identity, or at least 95% identity with SEQ ID NO: 1, wherein the monomeric subunit(s) has an aspartic acid or a glutamic acid at a position corresponding to M113 and/or K147 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at one or more positions corresponding to the vestibule and one or more positions corresponding to the beta barrel. As an example, the monomeric subunit(s) have at least 75% identity, at least 80% identity, at least 85% identity, at least 90% identity, at least 91% identity, at least 92% identity, at least 93% identity, at least 94% identity, or at least 95% identity with SEQ ID NO: 1 is provided, wherein the monomeric subunit has an aspartic acid or a glutamic acid at: (a) a position corresponding to one or more of 0, A1, K8, T9, G10, N17, K46, and N47 of SEQ ID NO: 1 and (b) a position corresponding to one or more of M113, T115, T117, N121, T129, G130, K131, and K147 of SEQ ID NO: 1. As another example, the monomeric subunit has at least 75% identity, at least 80% identity, at least 85% identity, at least 90% identity, at least 91% identity, at least 92% identity, at least 93% identity, at least 94% identity, or at least 95% identity with SEQ ID NO: 1, wherein the monomeric subunits has an aspartic acid or a glutamic acid at a position corresponding to A1, N17, and N47 of SEQ ID NO: 1 and at a position corresponding to one or more of M113, T115, T117, N121, T129, G130, K131, and K147 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at one or more positions corresponding to the vestibule and at one or more positions corresponding to the exit to the beta barrel. As an example, a monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 is provided, the monomeric subunit(s) having an aspartic acid or a glutamic acid at: (a) a position corresponding to one or more of A1, N17, and N47 of SEQ ID NO: 1 and (b) a position corresponding to K131 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at one or more positions corresponding to the constriction site and one or more positions corresponding to the exit to the beta barrel. As an example, the monomeric subunit(s) have at least 75% identity, at least 80% identity, at least 85% identity, at least 90% identity, at least 91% identity, at least 92% identity, at least 93% identity, at least 94% identity, or at least 95% identity with SEQ ID NO: 1 is provided, and have an aspartic acid or a glutamic acid at: (a) a position corresponding to either or both of M113 and K147 of SEQ ID NO: 1 and (b) a position corresponding to K131 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 has at least one non-native negatively-charged amino acid at one or more positions corresponding to the vestibule, one or more positions corresponding to the constriction site, and one or more positions corresponding to the exit to the beta barrel. As an example, the monomeric subunit(s) have at least 75% identity, at least 80% identity, at least 85% identity, at least 90% identity, at least 91% identity, at least 92% identity, at least 93% identity, at least 94% identity, or at least 95% identity with SEQ ID NO: 1, and have an aspartic acid or a glutamic acid at: (a) a position corresponding to one or more of A1, N17, and N47 of SEQ ID NO: 1, (b) a position corresponding to either or both of M113 and K147 of SEQ ID NO: 1, and (c) a position corresponding to K131 of SEQ ID NO: 1.

In an embodiment, the α-HL monomeric subunit having at least 75% identity, at least 80% identity, at least 85% identity, at least 90% identity, at least 91% identity, at least 92% identity, at least 93% identity, at least 94% identity, or at least 95% identity with SEQ ID NO: 1 and having at least one non-native negatively-charged amino acid disclosed herein may further comprise additional modifications. An example includes substitutions that widen the constriction site. These substitutions replace the sidechain of the amino acids forming the constriction site with amino acids having shorter and/or less bulky side chains. Examples include E111A/S, M113A/S, and K147A/S/N substitutions. In an example, at least 3 monomeric subunits of the α-HL nanopore comprise one or more substitutions selected from the group consisting of E111A/S, M113A/S, and K147A/S/N. In an example, at least 4 monomeric subunits of the α-HL nanopore comprise one or more substitutions selected from the group consisting of E111A/S, M113A/S, and K147A/S/N. In an example, at least 5 monomeric subunits of the α-HL nanopore comprise one or more substitutions selected from the group consisting of E111A/S, M113A/S, and K147A/S/N. In an example, at least 6 monomeric subunits of the α-HL nanopore comprises one or more substitutions selected from the group consisting of E111A/S, M113A/S, and K147A/S/N (including 6:1 monomeric subunits, wherein the "6" component has substitutions corresponding to E111A/S, M113A/S, and K147A/S/N). Another example includes substitutions that control the ability of non-oligomerized monomeric subunits to self-oligomerize. For example, α-HL monomeric subunits having substitutions at H35 (e.g., H35G/L/D/E substitutions) are substantially non-oligomerized as long as they are kept at room temperature or below (e.g. 25 °C or lower), but will stably oligomerize when the temperature is raised to a higher temperature (e.g. 35 °C). In an exemplary embodiment, the α-HL monomeric subunits having the one or more non-native negatively charged amino acids further comprises an H35L substitution. Other examples of substitution strategies for controlling self-oligomerization and/or directing specific patterns of oligomerization are disclosed at, for example, WO/2017/050718. Another example includes substitutions that improve the expression level of the α-HL monomeric subunit(s) in a recombinant cell used to express the monomeric subunit(s). For example, H35L substitutions have been shown to improve expression levels of α-HL monomeric subunits having a plurality of non-native negatively charged amino acids in *E. coli* expression systems. Other examples include substitutions that reduce coefficient of variation of the arrival rate of the pore (CV), such as D227N.

The polypeptides generally comprise from 1 to 7 α-HL monomeric subunits. In an embodiment, the polypeptides disclosed herein comprise a single α-HL monomeric subunit. In another embodiment, the polypeptide comprises from 2 to 7 α-HL monomeric subunits (referred to hereafter as a concatenated α-HL polypeptide), explicitly including polypeptides comprising 2 α-HL monomeric subunits, polypeptides comprising 3 α-HL monomeric subunits, polypeptides comprising 4 α-HL monomeric subunits, polypeptides comprising 5 α-HL monomeric subunits, polypeptides comprising 6 α-HL monomeric subunits, and polypeptides comprising 7 α-HL monomeric subunits. Exemplary methods of generating concatenated α-HL polypeptide and considerations for doing so are disclosed by, for example, Hammerstein and US 2017-0088890 A1. In an embodiment, each monomeric subunit of the concatenated α-HL polypeptide is separated from the other monomeric subunit(s) by a linker sequence. In an embodiment, the linker sequence is a flexible linker. Exemplary flexible linkers are disclosed by, for example, Hammerstein and Chen III.

The polypeptides may also include components useful for purification of the polypeptide, such as, for example, epitope tags, protease cleavage sites, etc.

The polypeptides may also include entities useful for attachment of other active agents (such as polymerases) to the polypeptide (referred to herein as "attachment components"). Exemplary attachment components include, for example, components of the SpyTag/SpyCatcher peptide system (Zakeri et al. PNAS 109: E690-E697 2012), native chemical ligation system (Thapa et al., Molecules 19:14461-14483 2014), sortase system (Wu and Guo, J Carbohydr Chem 31:48-66 2012; Heck et al., Appl Microbiol Biotechnol 97:461-475 2013)), transglutaminase systems (Dennler et al., Bioconjug Chem 25:569 578 2014), formylglycine linkage systems (Rashidian et al., Bio conjug Chem 24:1277-1294 2013), a Click chemistry attachment system, or other chemical ligation techniques known in the art.

### IIIB. Nucleic acids, expression cassettes, expression vectors, recombinant cells, and methods of producing polypeptides

In another aspect of the present disclosure, isolated polynucleotides are provided, said nucleic acid comprising a nucleotide sequence encoding a polypeptide comprising one or more α-HL monomeric subunits having at least one non-native negatively-charged amino acid (such as aspartic acid or glutamic acid) at a position corresponding to the vestibule region, the constriction zone, and/or the beta barrel of the α-HL nanopore, expressly including the polypeptides disclosed in section IIA. In an embodiment, the nucleic acid is an expression cassette comprising the nucleotide sequence encoding the polypeptide linked to a set of nucleic acid transcription elements (such as promoters, enhancers, start and stop codons, ribosomal binding sites, and the like) sufficient for transcription of the nucleotide sequence encoding the polypeptide in a prokaryotic or eukaryotic cell or in a cell-free expression system.

In another aspect, a vector is provided comprising the nucleotide encoding the polypeptide. The vectors may, for example, be cloning or expression vectors. Suitable vector backbones include, for example, those routinely used in the art such as plasmids, artificial chromosomes, BACs, or PACs. Numerous vectors and expression systems are commercially available from such corporations as Novagen (Madison, Wis.), Clonetech (Pal Alto, Calif.), Stratagene (La Jolla, Calif.), and Invitrogen/Life Technologies (Carlsbad, Calif.). Vectors typically contain one or more regulatory regions. Regulatory regions include, without limitation, promoter sequences, enhancer sequences, response elements, protein recognition sites, inducible elements, protein binding sequences, 5' and 3' untranslated regions (UTRs), transcriptional start sites, termination sequences, polyadenylation sequences, *et cetera.*

In another embodiment, a host cell comprising the expression vector is provided. For example, a host cell useful for production of polypeptides is transformed or transiently or stably transfected with the expression vector. In another aspect of the present disclosure, a method of preparing a polypeptide comprising one or more α-HL monomeric subunits having at least one non-native negatively-charged amino acid (such as aspartic acid or glutamic acid) at a position corresponding to the vestibule region, the constriction zone, and/or the beta barrel of the α-HL nanopore as disclosed herein (expressly including the polypeptides disclosed in section IIA) is provided, the method comprising (a) culturing a host cell comprising an expression vector as disclosed herein under conditions sufficient to induce expression of the polypeptide, and (b) purifying the polypeptide from the host cell. Such methods are well known in the art, and many systems for doing so are commercially available.

### IIIC. α-HL nanopores

In an embodiment, a heptameric α-HL nanopore or a hybrid nanopore comprising a heptameric α-HL nanopore as the biological component is provided, having a plurality of non-native negatively-charged amino acid (such as aspartic acid or glutamic acid) at one or more of the vestibule region, the constriction zone, and the beta barrel of the α-HL nanopore. The α-HL nanopores disclosed herein have a channel comprising at least one non-native negatively-charged amino acid at a position correlating to one or more sub-regions of the channel selected from the group consisting of the vestibule region, the constriction zone, and the beta barrel. Sufficient non-native negatively charged amino acids are provided at one or more of the foregoing locations that the template and/or primer nucleic acids are substantially repelled. Additionally or alternatively, sufficient non-native negatively charged amino acids are provided at one or more of the foregoing locations such that positively-charged tags can translocate through the channel of the nanopore. Additionally or alternatively, sufficient non-native negatively charged amino acids are provided at one or more of the foregoing locations such that the arrival rate and/or the threading rate of a positively-charged tag is increased relative to rate of a nanopore having the native amino acid at the same site.

Each monomeric subunit of the α-HL nanopore may have the same primary amino acid sequence (termed a "homoheptamer"), or at least one monomeric subunit of the heptamer may have an amino acid sequence that is different from the amino acid sequence of the other monomeric subunits (termed a "heteroheptamer"). Heteroheptameric α-HL nanopores may be referred to herein by a ratio of the species of different monomeric subunits used in the nanopore. For example, a "6:1 α-HL nanopore" has 6 monomeric subunits with the same amino acid sequence and 1 monomeric subunit with a different amino acid sequence. In such an example, reference to the "6" component shall mean each of the 6 identical monomeric subunits, while reference to the "1" component shall mean the 1 monomeric subunit with the different amino acid sequence. In some embodiments, each monomeric subunit of the α-HL nanopore is disposed in a polypeptide that does not contain additional monomeric subunits (termed herein a "non-oligomerized monomeric subunit"). Exemplary methods of making homoheptamers and heteroheptamers from non-oligomerized monomeric subunits are disclosed at US 2017-0088890 A1. For example, 6:1 heteroheptamers can be generated by mixing two different monomer preparations (for example, one in which the monomer is modified with an entity that can be used to bind to a polymerase and another entity that does not contain such a modification). The entity that is intended to be in excess in the resulting heptamer is provided in a molar excess relative to the other heptamer in the presence of a membrane and the mixture is incubated in an aqueous solution (such as 20mM Tris-HCl pH 8.0, 200 mM NaCl or 20mM Sodium Citrate pH 3, 400mM NaCl, 0.1% TWEEN20 + 0.2 M TMAO) overnight at 37 °C. The resulting heptamers are then purified by cation exchange chromatography. In some embodiments, oligomerization is performed in the presence of trimethylamine N-oxide (TMAO), such as from 0.1 to 5M TMAO, from 1 to 4M TMAO, and the like. In an embodiment, an α-HL monomeric subunit having a set of substitutions relative to SEQ ID NO: 1 comprising an H35G substitution and at least one non-native negatively charged amino acid is oligomerized in the presence of an aqueous buffer comprising from 0.1 to 5M TMAO at 37 °C. In another embodiment, an α-HL monomeric subunit having a set of substitutions relative to SEQ ID NO: 1 comprising an H35G substitution and at least one non-native negatively charged amino acid is oligomerized in the presence of an aqueous buffer comprising from 0.2 to 4M TMAO at 37 °C. In another embodiment, an α-HL monomeric subunit having a set of substitutions relative to SEQ ID NO: 1 comprising an H35G substitution and at least one non-native negatively charged amino acid is oligomerized in the presence of an aqueous buffer comprising about 0.2M to about 3M TMAO at 37 °C. In other embodiments, the nanopore includes at least one set of concatenated monomeric subunits. Exemplary methods of making α-HL nanopores from concatenated monomeric subunits of α-HL monomeric subunits are disclosed at, for example, Hammerstein and US 2017-0088890 A1.

In an embodiment, a heptameric α-HL nanopore comprising 7 monomeric subunits having at least 75% identity, at least 80% identity, at least 85% identity, at least 90% identity, at least 91% identity, at least 92% identity, at least 93% identity, at least 94% identity, or at least 95% identity with SEQ ID NO: 1 is provided, wherein 1, 2, 3, 4, 5, 6, or 7 of the monomeric subunits has at least one non-native negatively charged amino acid (such as aspartic acid or glutamic acid) at a position corresponding to a position of SEQ ID NO: 1 listed in Table 1 or Table 2. In some embodiments, the α-HL nanopore comprises a sufficient number of non-native negatively charged amino acids at a position of Table 1 or Table 2 to obtain a channel having a net-negative charge. As used in the context of α-HL nanopores, the "net charge of the channel" is the sum of the charges of all solvent-facing amino acid side chains within the channel of the nanopore in the presence of DPhPC in aqueous solution of 20mM Tris-HCl pH 8.0, 200 mM NaCl at 37 °C. In an embodiment, the at least one non-native negatively charged amino acid is at a position corresponding to a position of SEQ ID NO: 1 selected from the group consisting of A1, K8, T9, G10, N17, K46, N47, M113, T117, N121, T129, G130, K131, K147, and V149. In some embodiments, the α-HL nanopore comprises a sufficient number of non-native negatively charged amino acids at a position of SEQ ID NO: 1 selected from the group consisting of A1, K8, T9, G10, N17, K46, N47, M113, T117, N121, T129, G130, K131, K147, and V149 to obtain a channel having a net-negative charge.

The α-HL nanopores described herein may also include a polymerase attached thereto. In an embodiment, a single polymerase is attached to the α-HL nanopore. Exemplary polymerases include those derived from DNA polymerase Clostridium phage phiCPV4 (described by GenBank Accession No. YP_00648862, referred to herein as "Pol6"), phi29 DNA polymerase, T7 DNA pol, T4 DNA pol, E. coli DNA pol 1, Klenow fragment, T7 RNA polymerase, and E. coli RNA polymerase, as well as associated subunits and cofactors. In an embodiment, the polymerase is a DNA polymerase derived from Pol6. Exemplary Pol6 derivatives useful in nanopore-based sequencing are disclosed at, for example, US 2016/0222363, US 2016/0333327, US 2017/0267983, US 2018/0094249, and US 2018/0245147. Exemplary methods of attaching a polymerase to an α-HL nanopore include SpyTag/SpyCatcher peptide system (Zakeri et al. PNAS 109: E690-E697 2012), native chemical ligation system (Thapa et al., Molecules 19:14461-14483 2014), sortase system (Wu and Guo, J Carbohydr Chem 31:48-66 2012; Heck et al., Appl Microbiol Biotechnol 97:461-475 2013)), transglutaminase systems (Dennler et al., Bioconjug Chem 25:569 578 2014), formylglycine linkage systems (Rashidian et al., Bio conjug Chem 24:1277-1294 2013), Click chemistry attachment systems, or other chemical ligation techniques known in the art. In an embodiment, the polymerase is attached to an amino acid side chain of one of the monomeric subunits. In an embodiment, the α-HL nanopore is a 6:1 nanopore, wherein the polymerase is attached to the "1" component. In an embodiment, the α-HL nanopore is a 6:1 nanopore, wherein the polymerase is attached to the "1" component, and wherein the polymerase is a DNA polymerase. In another embodiment, the α-HL nanopore is a 6:1 nanopore, wherein the polymerase is attached to the "1" component, and wherein the polymerase is a DNA polymerase derived from Pol6.

### IV. Tagged nucleoside-5'-oligophosphates

In an aspect, tagged nucleotides are disclosed herein, said tagged nucleotides comprising a nucleoside-5'-oligophosphate moiety covalently linked to a nanopore-detectable moiety (referred to hereafter as a "tag") comprising a positively-charged polymer.

The polymer tags comprise a segment from 5 monomer units to 100 monomer units in length having a net-positive charge of at least +5 at pH 7.0 (referred to hereafter as "positively-charged segment" or "PCS"). When used with modified nanopores as described herein, tags having a PCS have improved capture rates and translocation rates relative to negatively-charged tags. As used herein, a "monomer unit" is a monomeric subunit of a polymer when polymerized. Exemplary monomer units include amino acids, amino acid analogs, linear or branched or cyclic poly(amine compounds), quaternized amine compounds, quaternized phosphines, glycols, or metal centers containing coordinated ligands. As used herein, a "positively charged monomer unit" is a monomer unit that has a net-positive charge at pH 7.0 in a buffered aqueous solution (e.g. 20 mM HEPES or 50 mM HEPES). As used herein, a "non-charged monomer unit" is a monomer unit that has a net-neutral charge in a neutrally buffered aqueous solution (e.g. 20 mM HEPES or 50 mM HEPES). As used herein, a "negatively-charged monomer unit" is a monomer unit that has a net-negative charge in a neutrally buffered aqueous solution (e.g. 20 mM HEPES or 50 mM HEPES). In an embodiment, the PCS has a ratio of at least 1 positively-charged monomer unit to 5 non-charged monomer units and a ratio of more than 1 positively-charged monomer unit to each negatively-charged monomer unit. In an embodiment, the PCS comprises less than 1 negatively-charged monomer unit for every 2 monomer units. In an embodiment, the PCS comprises less than 1 negatively-charged monomer unit for every 3 monomer units. In an embodiment, the PCS comprises less than 1 negatively-charged monomer unit for every 4 monomer units. In an embodiment, the PCS comprises less than 1 negatively-charged monomer unit for every 5 monomer units. In an embodiment, the PCS comprises less than 1 negatively-charged monomer unit for every 10 monomer units. In an embodiment, the PCS comprises less than 1 negatively-charged monomer unit for every 20 monomer units. In an embodiment, the PCS does not contain any negatively-charged monomer units. In an embodiment, the PCS is at least 5 monomers in length, has a net charge of at least +5 at pH 7.0, and has a ratio of at least 1 positively-charged monomer unit to 5 non-charged monomer units and a ratio of more than 1 positively-charged monomer unit to each negatively-charged monomer unit. In an embodiment, the PCS is at least 8 monomers in length, has a net charge of at least +5 at pH 7.0, and has a ratio of at least 1 positively-charged monomer unit to 5 non-charged monomer units and a ratio of more than 1 positively-charged monomer unit to each negatively-charged monomer unit. In an embodiment, the PCS is at least 5 monomers in length and has a net charge of at least +5 at pH 7.0. In an embodiment, PCS has a charge density of at least 0.1. In another embodiment, PCS has a charge density of at least 0.2. In another embodiment, PCS has a charge density of at least 0.25. In another embodiment, PCS has a charge density of at least 0.3. In another embodiment, PCS has a charge density of at least 0.4. In another embodiment, PCS has a charge density of at least 0.5. In another embodiment, PCS has a charge density of at least 0.6. In another embodiment, PCS has a charge density of at least 0.7. In another embodiment, PCS has a charge density of at least 0.8. In another embodiment, PCS has a charge density of at least 0.9. As used in the context of a PCS, the "charge density" is determined by dividing the net charge of PCS by the total number of monomer units of PCS.

In an embodiment, the PCS comprises a homopolymeric sequence of positively charged monomer units, with the proviso that said positively charged monomer units are not α-linked lysine. Exemplary homopolymeric PCS sequences include sequences of amino acids or amino acid derivatives, such as epsilon-linked lysine, Aminoethyl-piperazineacetic acid, Triethylenetriamine-succinamic acid, Diethylenetriamine-succinamic acid, aminoethylglycine, and 4-aminoproline. In an embodiment, the PCS comprises a heteropolymeric sequence of monomer units, wherein at least a portion of the monomer units are positively charged monomer units.

In an embodiment, the PCS is a heteropolymeric sequence comprising non-charged and/or negatively charged monomer units in addition to the positively charged monomer units. Non-charged monomer units and negatively-charged monomer units may be useful in the PCS, for example, for adjusting the charge density of the PCS (for example, by adjusting the distance between each positive charge in the PCS or neutralizing a portion of the positively-charged monomer units), for imparting specific secondary structures into the PCS (for example, by inducing turns into the backbone of the PCS to form a helical structure and/or stabilizing secondary structures via electrostatic interactions), and utilizing linear or branched or cyclic sections containing single or multiple positive charges. In some embodiments, the positively charged monomer units are distributed across the entire length of the PCS. The distribution of positively charged monomer units can be evaluated by determining the charge density of pre-defined lengths of the PCS. For example, if the pre-defined length is 10 monomer units, the charge density of every sequence of 10 monomer units within the PCS is determined. In this context, the "charge density" would be determined by dividing the net charge of the sequence by the number of monomer units within the sequence. In an embodiment, at least 50% of the 5 monomer unit sequences of the PCS have a charge density of at least 0.1. In an embodiment, at least 60% of the 5 monomer unit sequences of the PCS have a charge density of at least 0.1. In an embodiment, at least 70% of the 5 monomer unit sequences of the PCS have a charge density of at least 0.1. In an embodiment, at least 80% of the 5 monomer unit sequences of the PCS have a charge density of at least 0.1. In an embodiment, at least 90% of the 5 monomer unit sequences of the PCS have a charge density of at least 0.1. In an embodiment, at least 50% of the 5 monomer units sequences of the PCS have a charge density of at least 0.2. In an embodiment, at least 60% of the 5 monomer unit sequences of the PCS have a charge density of at least 0.2. In an embodiment, at least 70% of the 5 monomer unit sequences of the PCS have a charge density of at least 0.2. In an embodiment, at least 80% of the 5 monomer unit sequences of the PCS have a charge density of at least 0.2. In an embodiment, at least 90% of the 5 monomer unit sequences of the PCS have a charge density of at least 0.2. In an embodiment, at least 50% of the 5 monomer units sequences of the PCS have a charge density of at least 0.3. In an embodiment, at least 60% of the 5 monomer unit sequences of the PCS have a charge density of at least 0.3. In an embodiment, at least 70% of the 5 monomer unit sequences of the PCS have a charge density of at least 0.3. In an embodiment, at least 80% of the 5 monomer unit sequences of the PCS have a charge density of at least 0.3. In an embodiment, at least 90% of the 5 monomer unit sequences of the PCS have a charge density of at least 0.3. In an embodiment, at least 50% of the 5 monomer units sequences of the PCS have a charge density of at least 0.4. In an embodiment, at least 60% of the 5 monomer unit sequences of the PCS have a charge density of at least 0.4. In an embodiment, at least 70% of the 5 monomer unit sequences of the PCS have a charge density of at least 0.4. In an embodiment, at least 80% of the 5 monomer unit sequences of the PCS have a charge density of at least 0.4. In an embodiment, at least 90% of the 5 monomer unit sequences of the PCS have a charge density of at least 0.4. In an embodiment, at least 50% of the 5 monomer units sequences of the PCS have a charge density of at least 0.5. In an embodiment, at least 60% of the 5 monomer unit sequences of the PCS have a charge density of at least 0.5. In an embodiment, at least 70% of the 5 monomer unit sequences of the PCS have a charge density of at least 0.5. In an embodiment, at least 80% of the 5 monomer unit sequences of the PCS have a charge density of at least 0.5. In an embodiment, at least 90% of the 5 monomer unit sequences of the PCS have a charge density of at least 0.5. In an embodiment, at least 50% of the 5 monomer units sequences of the PCS have a charge density of at least 0.6. In an embodiment, at least 60% of the 5 monomer unit sequences of the PCS have a charge density of at least 0.6. In an embodiment, at least 70% of the 5 monomer unit sequences of the PCS have a charge density of at least 0.6. In an embodiment, at least 80% of the 5 monomer unit sequences of the PCS have a charge density of at least 0.6. In an embodiment, at least 90% of the 5 monomer unit sequences of the PCS have a charge density of at least 0.6.

In an embodiment, the PCS comprises, consists essentially of, or consists of polymers of monomer units set forth in Table 3:

**Table 3**

| | | | |
|---|---|---|---|
| K | Lysine | Dap | Diaminopropionic acid |
| | 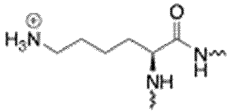 | | 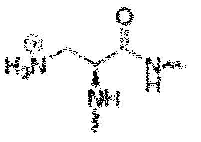 |
| K' | ε-linked Lysine | Dapa | Aminoethyl-substituted diaminopropionic acid |
| | 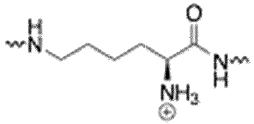 | | |
| P | Proline | Pra | Propargylglycine |
| | 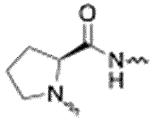 | | 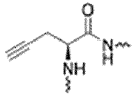 |
| R | Arginine | BnoP | 4-Benzyloxyproline |
| | 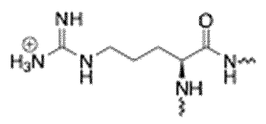 | | 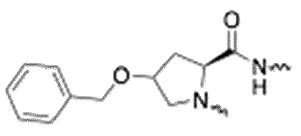 |
| G | Glycine | Apa | Aminoethyl-piperazineacetic acid |
| | 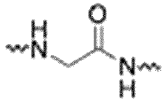 | | |
| A | Alanine | PyrAla | Pyrrolyl alanine |
| | 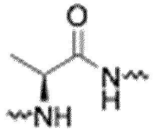 | | |
| F | Phenylalanine | TETA | Triethylenetriamine-succinamic acid |
| | 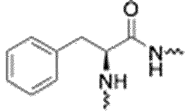 | | 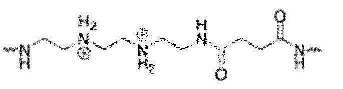 |
| H | Histidine | DETA | Diethylenetriamine-succinamic acid |
| | 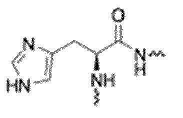 | | 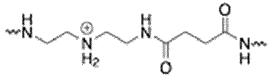 |
| L | Leucine | 4Ap | 4-aminoproline (both *cis* and *trans* isomers) |
| | 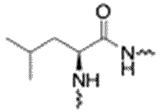 | | 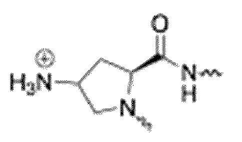 |
| Q | Glutamine | Aeg | Aminoethylglycine |
| | 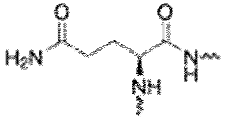 | | 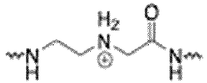 |
| S | Serine | bAla | β-alanine |
| | 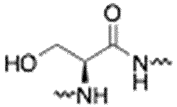 | | 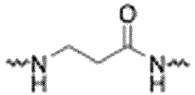 |
| 4Npa | 4-Nitrophenylalanine | Ahx | Aminohexanoic acid |
| | 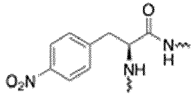 | | 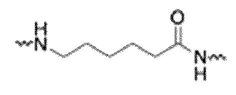 |
| 2Aba | 2-aminobenzoic acid | 3Aba | 3-aminobenzoic acid |
| 4Aba | 4-aminobenzoic acid | OAhx | Oxoaminohexanoic acid |
| | | | 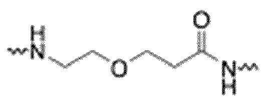 |
| Ahx | Aminohexanoic acid | pnaT | Peptide Nucleic Acid (Thymine) |
| | | | 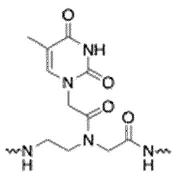 |
| | 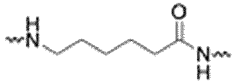 | | |
| Dap' PEG | Gamma-diaminopropionic acid | Hex | Hexynyl |
| | 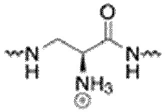 | | 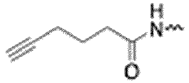 |
| | Ethylene glycol | pnaC | Peptide nucleic acid (cytosine) |
| | | | 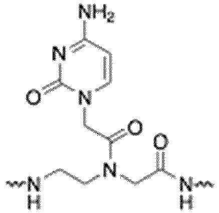 |
| | 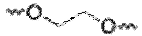 | | |
| Glu | Glutamate | 4-OHP | 4-hydroxoproline |
| | 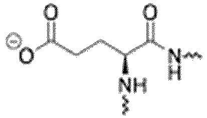 | | 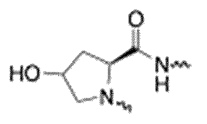 |
| Orn | Ornithine | Cit | Citrulline |
| | 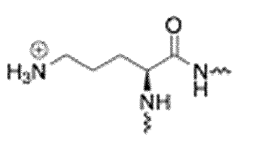 | | 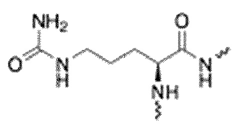 |
| KPeg4 | Lysine-Peg4 | | |
| | 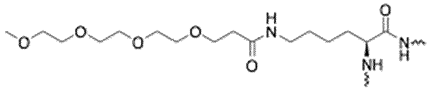 | | |
| KPeg3 | Lysine-Peg3 | | |
| | 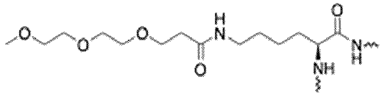 | | |
| Kpeg2 | Lysine-Peg2 | | |
| | 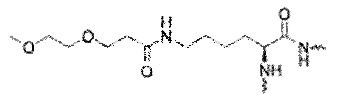 | | |
| Aegpeg4 | Aminoethylglycine-Peg4 | | |
| | 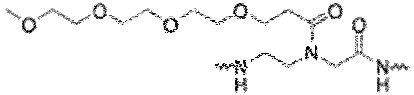 | | |

In an embodiment, the PCS is a homopolymer of a monomer unit of Table 3, except for polylysine. In another embodiment, the PCS is a heteropolymer comprising, consisting essentially of, or consisting of monomer units of Table 3.

In an embodiment, the PCS comprises, consists essentially of, or consists of a peptide chain or a peptidomimetic chain. When used in the context of PCS structures, the terms "amino acid" and "amino acid sequences" shall include the L-enantiomer, the D-enantiomer, and mixtures thereof unless otherwise indicated. Unless otherwise indicated, a reference to a specific amino acid residue shall refer to the α-amino acid. The monomer units of traditional peptide chains are so-called α-amino acids, in which the amino terminus and the carboxy terminus are separated by a single carbon (referred to as the α-carbon) to which the side chain is attached. However, additional carbons can be inserted between the amino and carboxy termini, thereby lengthening the backbone of the polypeptide. Exemplary amino acids modified in this way include β-amino acids (2 carbons separating the amino and carboxy termini), γ-amino acids (3 carbons separating the amino and carboxy termini), and δ-amino acids (4 carbons separating the amino and carboxy termini). The sidechain of these amino acids can occur at any of the carbons between the carboxy and amino termini. Additionally, lysine has two amino groups that can form a peptide bond: one at the α-carbon and one on the side chain at the ε-carbon. When linked to an adjacent amino acid via the amino group at the α-carbon, it will be referred to as the amino acid "lysine" or "K." When linked to an adjacent amino acid via the amino group at the ε-carbon, it will be referred to as "ε-linked lysine" or "K'." Additional examples of amino acids include amino acids having unnatural side chains, such as 4-aminoproline, 4-nitrophenylalanine, 4-benzyloxyproline, propargylglycine, and pyrrolyl alanine.

One way to ensure distribution of positive charges throughout the tag is to design the PCS to contain a plurality of repeating units, each having the same or similar structures and/or charge densities. In an embodiment, the PCS comprises, consists essentially of, or consists of, one or more structures according to Formula 1:

[REPEAT]ₐ **Formula 1,**

wherein "REPEAT" is a heteropolymeric sequence, wherein at least one monomer unit of the heteromonomeric sequence has a net-positive charge at pH 7.0, "a" is an integer ≥ 1, and wherein "a" is selected such that the heteropolymeric sequence is from 10 to 100 monomer units in length and has a net charge of at least +5 at pH 7.0. In an embodiment, REPEAT has a charge density of at least 0.1. In another embodiment, REPEAT has a charge density of at least 0.2. In another embodiment, REPEAT has a charge density of at least 0.25. In another embodiment, REPEAT has a charge density of at least 0.3. In another embodiment, REPEAT has a charge density of at least 0.4. In another embodiment, REPEAT has a charge density of at least 0.5. In another embodiment, REPEAT has a charge density of at least 0.6. In another embodiment, REPEAT has a charge density of at least 0.7. In another embodiment, REPEAT has a charge density of at least 0.8. In another embodiment, REPEAT has a charge density of at least 0.9. As used herein, the "charge density" is determined by dividing the net charge of REPEAT by the total number of monomer units of REPEAT.

In another embodiment, PCS comprises, consists essentially of, or consists of a single instance of Formula 1, wherein "a" is greater than 1. In such an embodiment, the same repeat unit of Formula 1 is repeated

In another embodiment, PCS comprises, consists essentially of, or consists of a multiple different instances of Formula 1. In an embodiment, PCS comprises, consists essentially of, or consists of a structure of Formula 1a

REPEAT1-REPEAT2 **Formula 1a,**

or Formula 1b:

REPEAT1-REPEAT2-REPEAT3 **Formula 1b,**

wherein each of REPEAT1, REPEAT2, and REPEATS is a structure according to Formula 1 and at least REPEAT2 is different from REPEAT1 and REPEATS.

In an embodiment, the PCS according to Formula 1, Formula 1a, or Formula 1b is provided, wherein REPEAT has a structure according to Formula 1c:

X_{b}-Y_{c} **Formula 1c,**

or Formula 1d:

Y_{c}-X_{b} **Formula 1d,**

wherein: X is a monomer unit bearing a net positive charge; Y is a monomer unit different from X and polymerizable with X; and b and c are integers selected such that REPEAT has a charge density of at least 0.1 at pH 7.0. In an embodiment, b = 1, 2, 3, 4, or 5 and c = 1, 2, 3, 4, or 5. In an embodiment, REPEAT is a structure of Formula 1c or Formula 1d, wherein Y is a non-charged monomer unit, and b and c are selected from the group consisting of: (1) b = 1 and c = 1; (2) b = 2, c = 1; (3) b = 2 and c = 2; (4) b = 2 and c = 3; and (5) b = 1 and c = 3. In an embodiment, REPEAT is a repeat unit of Formula 1c or 1d, wherein Y is a non-charged monomer unit, at least one of X and Y is selected to induce a bend in the backbone of the repeat unit such that multiple repeat units strung together have a helical structure, and wherein b and c are selected from the group consisting of: (1) b=1 and c=1; (2) b=1 and c=2; (3) b=2 and c=1; (4) b=2 and c=2; and (5) b=2 and c=3.

In another embodiment, the PCS according to Formula 1 is provided, wherein REPEAT has a structure according to any of Formula 1e - Formula 1j:

X_{b}-Y_{c}-Z_{d} **Formula 1e,**

Z_{d}-Y_{c}-X_{b} **Formula 1f,**

X_{b}-Z_{d}-Y_{c} **Formula 1g,**

Y_{b}-Z_{c}-Xₐ **Formula 1h,**

Z_{d}-X_{b}-Y_{c} **Formula 1i,**

Y_{c}-X_{b}-Z_{d} **Formula 1j,**

wherein: X is a monomer unit bearing a net positive charge; Y is a monomer unit polymerizable with X; Z is a monomer unit polymerizable with X and Y, wherein each of X, Y, and Z is different from the immediately adjacent monomer unit within the REPEAT structure; and b, c, and d are integers selected such that REPEAT has a charge density of at least 0.1 at pH 7.0. In an embodiment, b = 1, 2, 3, 4, or 5; c = 1, 2, 3, 4, or 5; and d = 1, 2, 3, 4, or 5. In another embodiment, b = 1, 2, 3, 4, or 5; c = 1, 2, 3, 4, or 5; and d = 1, 2, 3, 4, or 5, and a + b + c ≤ 10. In this context, the phrase "wherein each of X, Y, and Z is different from the immediately adjacent monomer unit within the REPEAT structure" means that each of X, Y, and Z can be the same or different, with the proviso that at least the "middle" variable in the repeat structure is different from the other variables. Thus, for example, in Formula 1e and 1f, X and Z can be the same or different, but Y is different from both X and Z. As another example, in Formula 1g and 1h, X and Y can be the same or different, but Z is different from both X and Y. As another example, in Formula 1i and 1j, Y and Z can be the same or different, but X is different from both Y and Z. In an embodiment, REPEAT is a structure of any of Formula 1e to Formula 1j, wherein Y and Z are non-charged monomer units, and b, c, and d are selected from the group consisting of: (1) b=1, c=1, and d=0; (2) b=1 and c+d=2; (3) b=2, c=1, and d=0; (4) b=2 and c+d=2; and (5) b=2 and c+d=3. In an embodiment, X is a positively charged monomer unit and Y and Z are non-charged monomer units and at least one of X, Y, and Z is selected to induce a bend in the backbone of the repeat unit, such that multiple repeat units strung together have a helical structure, and wherein b, c, and d are selected from the group consisting of: (1) b=1, c=1, and d=0; (2) b=1 and c+d=2; (3) b=2, c=1, and d=0; (4) b=2 and c+d=2; and (5) b=2 and c+d=3.

In an embodiment, the monomer units of REPEAT of Formula 1 (as well as X, Y, and Z of Formulae 1c-1j) are peptides and/or peptidomimetics. In an embodiment, the PCS comprises a plurality of repeat units according to Formula 1, wherein the REPEAT is a peptide chain comprising one or more positively charged amino acid(s) selected from the group consisting of lysine, ε-linked lysine, arginine, ornithine, positively charged derivatives of aliphatic amino acids (such as aminoethylglycine), positively-charged derivatives of aromatic amino acids, positively-charged derivatives of proline (such as 4-aminoproline), Dap, and Dapa. In an embodiment, the REPEAT is a structure according to any of Formula Ia-Ih, wherein X, Y, and Z (if present) are each selected from the moieties of Table 3. In other embodiments, the peptide or peptidomimetic PCS may also contain non-amino acid substituents, such as ethylene glycol monomers (PEG), which can act as a flexible hydrophobic linker that can coordinate potassium to provide a positive charge, or hexynyl (Hex), which can act as a reactive moiety to linker to attach the tag to a nucleotide via a Click reaction.

In another exemplary embodiment, REPEAT is a structure according to any of Formula Ia-Ih, wherein at least one Y and Z is a non-charged amino acid or amino acid analog, such as an aliphatic amino acid (such as glycine, alanine, valine, leucine, or isoleucine), an aromatic amino acid, proline or a non-charged proline derivative. For example, inclusion of A, F, H, and the like can give spacing of the charges along the backbone of the tag molecule to prevent aggregation of DNA or inhibition of the DNA polymerase or provide further steric bulk to impart desired level characteristics or provide a more rigid secondary structure to the tag molecule. As one example, X is a positively-charged amino acid selected from the group consisting of lysine, β-lysine, γ-lysine, δ-lysine, and ε-linked lysine; each of Y and Z is a non-charged amino acid selected from the group consisting of an aliphatic amino acid (such as α-, β-, γ-, or δ-glycine; α-, β-, γ-, or δ-alanine; α-, β-, γ-, or δ-valine; α-, β-, γ-, or δ-leucine; or α-, β-, γ-, or δ-isoleucine), an aromatic amino acid, proline, or a proline derivative; and b, c, and d are selected from the group consisting of: (1) b=1, c=1, and d=0; (2) b=1 and c+d=2; (3) b=2, c=1, and d=0; (4) b=2 and c+d=2; and (5) b=2 and c+d=3. As another example, Y and Z are non-charged amino acids; b, c, and d are selected from the group consisting of (1) b=1, c=1, and d=0; (2) b=1 and c+d=2; (3) b=2, c=1, and d=0; (4) b=2 and c+d=2; and (5) b=2 and c+d=3; and each of X, Y, and Z is selected from the moieties of Table 3.

In an embodiment, the specific monomer units of the peptide or peptidomemetic chain are selected to obtain PCS having specific secondary structure. For example, helical structures can be generated by inclusion of proline and proline derivatives into the repeat units. Helical structures provide more rigid tag structures that are likely to have less signal variability and afford a more discrete level and also reduce potential interaction of the tag molecule with the DNA sample or polymerase present in sequencing. Additionally, amino acids having unnatural side chains (e.g., 4-aminoproline, 4-nitrophenylalanine, 4-benzyloxyproline, propargylglycine, and pyrrolyl alanine) can be used to generate defined structural features to control the observed tag level or allow handles for subsequent modification with the appropriate reactive partners. These different building blocks can be used to design PCS having specifically-defined characteristics, for example, specified lengths, backbone rigidity, sidechain and/or charge density along the backbone, secondary structures (such as helices and sheet structures), enhance polymerase interaction with the tag molecule, prevent aggregation of DNA, or enhance pore threading interactions. These types of peptides and peptidomimetics are frequently referred to as foldamers. References discussing design considerations for peptide and peptidomimetic foldamers include Licini, Martinek, Goodman, among others. As an example within this embodiment, the PCS is a peptide chain having a foldamer structure, wherein a plurality of the monomer subunits are proline or a proline derivatives. For example, a PCS of Formula 1 can be provided, wherein REPEAT comprises one or more proline or proline derivatives. In another embodiment, REPEAT of Formula 1 comprises or consists of a structure according to any of Formula 1c-1j, wherein at least one of Y and Z is a proline or a proline derivative. As another example, the PCS comprises the structure of Formula 1, wherein REPEAT is a structure according to any of Formula 1c-1j, and wherein: X is selected from the group consisting of lysine, β-lysine, γ-lysine, δ-lysine, and ε-linked lysine; each of Y and Z is a non-charged amino acid selected from the group consisting of an aliphatic amino acid (such as glycine, alanine, valine, leucine, or isoleucine), a non-charged derivative of an aliphatic amino acid (such as β-, γ-, or δ-glycine, β-, γ-, or δ-alanine, β-, γ-, or δ-valine, β-, γ-, or δ-leucine, or β-, γ-, or δ-isoleucine), an aromatic amino acid, a non-charged derivative of an aromatic amino acid, proline, or a proline derivative, with the proviso that either Y or Z is proline or a proline derivative; and b, c, and d are selected from the group consisting of: (1) b=1, c=1, and d=0; (2) b=1 and c+d=2; (3) b=2, c=1, and d=0; (4) b=2 and c+d=2; and (5) b=2 and c+d=3. As another example, the PCS comprises the structure of Formula 1, wherein REPEAT is a structure according to any of Formula 1c-1j, wherein Y and Z are non-charged amino acids or amino acid analogs, with the proviso that at least one of Y and Z is proline or a proline derivative; b, c, and d are selected from the group consisting of (1) b=1, c=1, and d=0; (2) b=1 and c+d=2; (3) b=2, c=1, and d=0; (4) b=2 and c+d=2; and (5) b=2 and c+d=3; and each of X, Y, and Z is selected from the moieties of Table 3.

Exemplary variable combinations of Formula 1c-1j include, but are not limited to, the combinations of Table 4a:

**Table 4a**

| **Formula 1c-1j Variables** | | | | | | **Charge Density** | **Formula 1c-1j Variables** | | | | | | **Charge Density** |
|---|---|---|---|---|---|---|---|---|---|---|---|---|---|
| **X** | **Y** | **Z** | **b** | **c** | **d** | | **X** | **Y** | **Z** | **b** | **c** | **d** | |
| K | P | - | 1 | 1 | - | 0.5 | K | S | P | 1 | 1 | 2 | 0.25 |
| K | P | - | 2 | 1 | - | 0.66 | K' | P | - | 1 | 2 | - | 0.33 |
| K | P | - | 2 | 2 | - | 0.5 | K' | P | - | 2 | 1 | - | 0.66 |
| K | P | - | 2 | 3 | - | 0.4 | K' | P | - | 2 | 2 | - | 0.5 |
| K | G | - | 1 | 1 | - | 0.5 | K' | BnoP | P | 2 | 1 | 1 | 0.5 |
| K | G | - | 2 | 2 | - | 0.5 | K | bAla | - | 1 | 1 | - | 0.5 |
| K | G | P | 2 | 1 | 1 | 0.5 | K | bAla | - | 1 | 2 | - | 0.33 |
| K | G | P | 1 | 1 | 2 | 0.25 | Ds | Dap | - | 1 | 1 | - | 0.5 |
| K | A | P | 1 | 1 | 2 | 0.25 | 4Ap | P | - | 1 | 1 | - | 0.5 |
| K | A | - | 1 | 3 | - | 0.25 | 4Ap | P | - | 2 | 2 | - | 0.5 |
| K | F | P | 1 | 1 | 2 | 0.25 | R | P | - | 2 | 2 | - | 0.5 |
| K | H | P | 1 | 1 | 2 | 0.25 | R | P | - | 2 | 3 | - | 0.4 |
| K | L | P | 1 | 1 | 2 | 0.25 | Aeg | P | - | 1 | 2 | - | 0.33 |
| K | Q | P | 1 | 1 | 2 | 0.25 | Aeg | P | - | 2 | 2 | - | 0.5 |

As used in Table 4a, the "charge density" is determined by dividing the net charge of the repeat unit by the total number of amino acids of the repeat unit. Specific REPEAT structures within the scope of Formula 1 include, but are not limited to, the peptides of Table 4b and the retro-inverso sequence thereof:

**Table 4b**

| **Repeat** | **SEQ ID NO** | **Repeat** | **SEQ ID NO** | **Repeat** | **SEQ ID NO** | **Repeat** | **SEQ ID NO** |
|---|---|---|---|---|---|---|---|
| K-P | N/A | K'₂-P₂ | 14 | K'₂-Ahx | N/A | Aeg₂-G | N/A |
| K₂-P | N/A | K'₂-BnoP-P | 15 | K'₂-G₂ | 159 | G-KPeg4₂-P | 161 |
| K₂-P₂ | 2 | bAla-K | N/A | K'₂-G | N/A | Kpeg-G | N/A |
| K₂-P₃ | 3 | bAla₂-K | N/A | K'₂-A | N/A | Aeg-pnaT₃ | 162 |
| K-G | N/A | Ds-Dap | N/A | K'₂-S | N/A | G-Kpeg3-G-Kpeg3-P | 163 |
| K₂-G₂ | 4 | 4Ap-P | N/A | K'₂-F | N/A | Aegpeg4₂-P | N/A |
| K₂-G-P | 5 | 4Ap₂-P₂ | 16 | K'₂-Bnop | N/A | pnaC₂-P | N/A |
| K-G-P₂ | 6 | R₂-P₂ | 17 | K'₂-Q | N/A | K'-R-P₃ | 164 |
| K-A-P₂ | 7 | R₂-P₃ | 18 | pnaT₂-P | N/A | K₃-P | 165 |
| K-A₃ | 8 | Aeg-P₂ | N/A | K'-OAhx | N/A | K-P-R-P | 166 |
| K-F-P₂ | 9 | Aeg₂-P₂ | 19 | K'₂-OAhx | N/A | bAla-K | N/A |
| K-H-P₂ | 10 | Aeg₂-P | N/A | Aeg-OAhx | N/A | K₂-R-P | 167 |
| K-L-P₂ | 11 | G-4Ap-P | N/A | K'-OAhx₂ | N/A | K₂-pnaT | N/A |
| K-Q-P₂ | 12 | K'-K-P | N/A | pnaT₂-G | N/A | K'₂-pnaT | N/A |
| K-S-P₂ | 13 | K'₂-pnaT | N/A | pnaT₂-K | N/A | R-bAla | N/A |
| K'-P₂ | N/A | Dap'₂-P₂ | 158 | Kpeg4₄-P | N/A | Acp₂-P | N/A |
| K'₂-P | N/A | K'-4Ap-P | N/A | G-Kpeg-G-Kpeg-P | 160 | K'-K-P | N/A |

As used herein, a "retro-inverso sequence" of the disclosed REPEAT is a repeat unit having the same sequence of monomer units, except in the reverse order. Thus, for example, for a give set of variables within the scope of Formula 1c:

X_{b}-Y_{c} **Formula 1c,**

the retro-inverso sequence would be the sequence of Formula 1d containing the same set of variables:

Y_{c}--X_{b} **Formula 1d.**

In an embodiment, the polymer tag comprises, consists of, or consists essentially of a structure according to Formula 2:

U-PCS-V **Formula 2**

wherein: U is optional and, when present, comprises 1 to 10 monomer units; V is optional and, when present, comprises 1 to 10 monomer units; and PCS is as described above. The structure defined by Formula 2 has a net-positive charge at pH 7.0. U can be used as an adapter to attach the PCS to a nucleoside oligophosphate, and the monomer subunits of U may be selected to maintain or improve interaction of the tag molecule interaction with the polymerase utilized in sequencing. V can be varied to potentially enhance the attraction properties of the PCS for the pore mutant to improve the time to thread properties of the system, for example, by adding additional negative charges. In some embodiments, the constituents of U, PCS, and V are selected such that the polymer tag has an overall length sufficient to extend beyond the constriction site of the nanopore when threaded into the channel of the nanopore (for example, a minimum length equal to at least the length from the *cis* terminus of the vestibule to the exit of the channel). Dimensions of various nanopores and distances to constriction sites are generally known. *See generally,* Song and Wang II. For example, an α-hemolysin heptameric pore have a channel that is ~10 nm in length with the constriction site located ~4.8 nm from the vestibule. *See* Song. In such a case, the structure formed by U-PCS-V would be at least 4.8 nm in length to reach the constriction site and at least 10 nm in length to extend all the way through the channel of the nanopore. In an embodiment, a polymer tag having the structure according to Formula 2 is provided, wherein the PCS has a charge density of at least 0.25.

In an embodiment, the polymer tag of Formula 2 comprises a peptide chain in which PCS has the structure of Formula 1. In an embodiment, the overall backbone length of the peptide chain is at least as long as a nanopore channel with which the tag is intended to be used. As used herein, the term "overall backbone length" shall refer to the combined length of the bonds that form the peptide backbone. In another embodiment, d is an integer that results in a PCS having an overall backbone length that is at least as long as a channel of a nanopore with which the tag is intended to be used. For example, for a peptide tag that is intended to be used with an α-hemolysin pore, d may be an integer that results in a PCS having an overall backbone length of at least 10 nm. In other embodiments, the structure of Formula 1i is a foldamer, wherein d is selected such that the overall length of the foldamer is at least as long as a channel of a nanopore with which the tag is intended to be used. In an embodiment, the PCS is a structure of Formula 1, wherein d is an integer such that the peptide backbone of PCS is at least 10 nm. In another embodiment, PCS is a structure of Formula 1, wherein REPEAT is a structure of any of formula 1c-1j, with the proviso that a(c+d+d) ≤ 100, including but not limited to examples in which d is selected such that: 7 ≤ a(c+d+d) ≤ 100, or 7 ≤ a(c+d+d) ≤ 70, or 7 ≤ a(c+d+d) ≤ 60, or 8 ≤ a(c+d+d) ≤ 100, or 8 ≤ a(c+d+d) ≤ 70, or 8 ≤ a(c+d+d) ≤ 60.

In an embodiment, the polymer tag of Formula 2 wherein U and V have a net-neutral or a net-positive charge. In an embodiment, U comprises, consists essentially of, or consists of 1 to 10 amino acids having a net-neutral charge or net-positive charge at pH 7.0, such as such as lysine, lysine derivatives (such as ε-linked lysine), arginine, ornithine, aliphatic amino acids, derivatives of aliphatic amino acids (such as pyrrolyl alanine, aminoethylglycine or propargylglycine), aromatic amino acids, derivatives of aromatic amino acids (such as 4-Nitrophenylalanine), proline, derivatives of proline (such as 4-aminoproline), Dap, and Dapa. In another embodiment, U comprises, consists essentially of, or consists of one or more amino acids selected from the group consisting of lysine, lysine derivatives (such as ε-linked lysine), arginine, ornithine, derivatives of aliphatic amino acids (such as aminoethylglycine or propargylglycine), derivatives of aromatic amino acids (such as 4-Nitrophenylalanine and Pyrrolyl alanine), derivatives of proline (such as 4-aminoproline), Dap, and Dapa. In yet another embodiment, U comprises, consists essentially of, or consists of one or more amino acids selected from the group consisting of lysine, ε-linked lysine, arginine, ornithine, aminoethylglycine, propargylglycine, 4-Nitrophenylalanine, Pyrrolyl alanine, 4-aminoproline, Dap, and Dapa. In another embodiment, U is 1 or 2 amino acids in length, wherein the 1 or 2 amino acids are selected from the group consisting of lysine, ε-linked lysine, arginine, ornithine, aminoethylglycine, propargylglycine, 4-Nitrophenylalanine, Pyrrolyl alanine, 4-aminoproline, Dap, and Dapa. In an embodiment, U is 1 or 2 amino acids in length, wherein the 1 or 2 amino acids are selected from the group consisting of lysine, ε-linked lysine, arginine, propargylglycine, 4-Nitrophenylalanine, and pyrrolyl alanine. In an embodiment, U is selected from the group consisting of K, K₂, K', K'K, K'K₂, K'R, Pra, PyrAla, and 4Npa. In another embodiment, V is a homopeptide chain comprising, consisting essentially of, or consisting of lysine, Dap, or Dapa. In an embodiment, the C-terminus of V is modified such that the carboxylic acid moiety is replaced with a primary amide.

In a specific embodiment, the PCS of Formula 2 has a structure according to Formula 2a:

U - [X_{b} - Y_{c} - Z_{d}]ₐ -Vₑ **Formula 2a,**

or Formula 2b:

U - [Z_{d} - Y_{c} - X_{b}]ₐ -Vₑ **Formula 2b,**

wherein: U is absent or is a chain of amino acids or amino acid analogs from 1 to 10 amino acids and/or amino acid analogs in length; a-c are integers and d is 0 or an integer, with the proviso that a(b+c+d) ≤ 100; V is a positively charged amino acid or amino acid analog, and e is from 0 to 10. In an example within this embodiment, U is a peptide chain from 1 to 3 amino acids or amino acid analogs in length. In an embodiment, V is a positively charged amino acid or amino acid analog. In an embodiment, U and V are positively charged amino acids or amino acid analogs selected from Table 3. Exemplary peptide tag structures within the scope of Formula 2a or 2b include, but are not limited to, the combinations of Table 5a:

**Table 5a**

| **U** | **X** | **Y** | **Z** | **V** | **b** | **c** | **d** | **a** | **e** |
|---|---|---|---|---|---|---|---|---|---|
| K | K | P | - | K | 2 | 2 | 0 | 1-25 | 1-10 |
| K₂ | K | P | - | K | 1 | 1 | 0 | 1-50 | 1-10 |
| K'K | K | P | - | K | 2 | 3 | 0 | 1-25 | 1-10 |
| K'K₂ | K | P | - | K | 1 | 1 | 0 | 1-50 | 1-10 |
| K'R | R | P | - | R | 2 | 2 | 0 | 1-25 | 1-10 |
| K'R | R | P | - | R | 2 | 3 | 0 | 1-20 | 1-10 |
| K' | Aeg | P | - | K | 2 | 2 | 0 | 1-25 | 1-10 |
| K' | Aeg | P | - | K | 1 | 2 | 0 | 1-33 | 1-10 |
| K'K | K | P | - | K | 2 | 1 | 0 | 1-33 | 1-10 |
| K'K | K | P | - | K | 2 | 3 | 0 | 1-20 | 1-10 |
| K' | K' | P | - | K | 2 | 1 | 0 | 1-33 | 1-10 |
| - | K' | P | - | K | 2 | 2 | 0 | 1-25 | 1-10 |
| K' | K' | P | - | K | 1 | 2 | 0 | 1-33 | 1-10 |
| - | K' | BnoP | P | K | 2 | 1 | 1 | 1-25 | 1-10 |
| K' | K | G | P | K | 1 | 1 | 2 | 1-25 | 1-10 |
| K' | K' | P | - | K | 1 | 2 | 0 | 1-33 | 1-10 |
| - | K' | P | - | K | 2 | 2 | 0 | 1-25 | 1-10 |
| - | K' | P | - | K | 2 | 2 | 0 | 1-25 | 1-10 |
| K' | K | P | - | K | 2 | 2 | 0 | 1-25 | 1-10 |
| K' | K | P | - | K | 2 | 2 | 0 | 1-25 | 1-10 |
| K' | K | A | P | K | 1 | 1 | 2 | 1-25 | 1-10 |
| K' | K | F | P | K | 1 | 1 | 2 | 1-25 | 1-10 |
| K' | K | H | P | K | 1 | 1 | 2 | 1-25 | 1-10 |
| K' | K | L | P | K | 1 | 1 | 2 | 1-25 | 1-10 |
| K' | K | Q | P | K | 1 | 1 | 2 | 1-25 | 1-10 |
| K' | K | S | P | K | 1 | 1 | 2 | 1-25 | 1-10 |
| Pra | K | P | - | K | 2 | 2 | 0 | 1-25 | 1-10 |
| PyrAla | K | P | - | K | 2 | 2 | 0 | 1-25 | 1-10 |
| K' | K | P | - | K | 2 | 2 | 0 | 1-25 | 1-10 |
| K' | K | P | - | Dap | 2 | 2 | 0 | 1-25 | 1-10 |
| K' | K | P | - | R | 2 | 2 | 0 | 1-25 | 1-10 |
| K' | K | P | - | R | 2 | 2 | 0 | 1-25 | 1-10 |
| K' | K | P | - | Dapa | 2 | 2 | 0 | 1-25 | 1-10 |
| K' | bAla | K | - | K | 1 | 1 | 0 | 1-50 | 1-10 |
| K' | bAla | K | - | K | 2 | 1 | 0 | 1-33 | 1-10 |
| K' | Ds | Dap | - | K | 1 | 1 | 0 | 1-50 | 1-10 |
| K' | K | A | - | K | 1 | 3 | 0 | 1-25 | 1-10 |
| K' | K | G | - | K | 1 | 1 | 0 | 1-50 | 1-10 |
| K' | K | G | - | K | 2 | 2 | 0 | 1-25 | 1-10 |
| K' | K | G | P | K | 2 | 1 | 1 | 1-25 | 1-10 |
| K' | 4Ap | P | - | K | 2 | 2 | 0 | 1-25 | 1-10 |
| K' | 4Ap | P | - | K | 2 | 1 | 0 | 1-33 | 1-10 |
| 4Npa | K | P | - | K | 2 | 1 | 0 | 1-33 | 1-10 |
| 4Npa | Aeg | P | - | K | 1 | 2 | 0 | 1-33 | 1-10 |
| 4Npa | K' | P | - | K | 2 | 1 | 0 | 1-33 | 1-10 |
| Hex-4NPA | Aeg | P | - | K | 1 | 2 | 0 | 1-33 | 1-10 |
| Hex-4NPA | K' | P | - | K | 2 | 1 | 0 | 1-33 | 1-10 |
| Hex-4NPA | Aeg | P | - | K | 2 | 2 | 0 | 1-25 | 1-10 |
| Hex-4NPA | Aeg | P | - | K | 2 | 1 | 0 | 1-33 | 1-10 |
| Pra | K' | P | - | K' | 2 | 2 | 0 | 1-25 | 1-10 |
| 4NPA | G | 4Ap | P | K | 1 | 1 | 1 | 1-33 | 1-10 |
| 4NPA | K' | P | - | K' | 2 | 1 | 0 | 1-33 | 1-10 |
| Hex-4NPA | K | G | - | K | 1 | 1 | 0 | 1-50 | 1-10 |
| Hex-4NPA | K' | K | P | K | 1 | 1 | 1 | 1-33 | 1-10 |
| Hex-4NPA | K' | P | - | K | 2 | 2 | 0 | 1-25 | 1-10 |
| 4NPA | K' | pnaT | - | K | 2 | 1 | 0 | 1-33 | 1-10 |
| Hex-4NPA | K | G | - | K | 2 | 2 | 0 | 1-25 | 1-10 |
| Hex-4NPA | Dap' | P | - | K | 2 | 2 | 0 | 1-25 | 1-10 |
| Hex-4NPA | K' | 4Ap | P | K | 1 | 1 | 1 | 1-33 | 1-10 |
| Hex-4NPA | K' | Ahx | - | K' | 2 | 1 | 0 | 1-33 | 1-10 |
| Hex-4NPA | K' | G | - | K' | 2 | 2 | 0 | 1-25 | 1-10 |
| Hex-4NPA | K' | G | - | K' | 2 | 1 | 0 | 1-33 | 1-10 |
| Hex-4NPA | K' | A | - | K' | 2 | 1 | 0 | 1-33 | 1-10 |
| Hex-4NPA | K' | S | - | K' | 2 | 1 | 0 | 1-33 | 1-10 |
| Hex-4NPA | K' | F | - | K' | 2 | 1 | 0 | 1-33 | 1-10 |
| Hex-4NPA | K' | Bnop | - | K' | 2 | 1 | 0 | 1-33 | 1-10 |
| Hex-4NPA | K' | Q | - | K' | 2 | 1 | 0 | 1-33 | 1-10 |
| Hex-4NPA | K' | OAhx | - | K' | 1 | 1 | 0 | 1-50 | 1-10 |
| Hex-4NPA | K' | OAhx | - | K' | 2 | 1 | 0 | 1-33 | 1-10 |
| Hex-4NPA | Aeg | OAhx | - | K' | 1 | 1 | 0 | 1-50 | 1-10 |
| Hex-4NPA | K' | OAhx | - | K' | 1 | 2 | 0 | 1-33 | 1-10 |
| Hex-K | K | P | - | K | 2 | 2 | 0 | 1-25 | 1-10 |
| Hex-K | K | P | - | K | 2 | 3 | 0 | 1-20 | 1-10 |
| Hex-K' | K' | P | - | K | 2 | 1 | 0 | 1-33 | 1-10 |
| Hex-4NPA | K' | P | - | K | 2 | 1 | 0 | 1-33 | 1-10 |
| Hex-4NPA | Aeg | P | - | K | 1 | 2 | 0 | 1-33 | 1-10 |
| Hex-4NPA | Aeg | P | - | K | 2 | 1 | 0 | 1-33 | 1-10 |
| Hex-4NPA | Aeg | P | - | K | 2 | 2 | 0 | 1-25 | 1-10 |
| Hex-4NPA | K' | R | P | R | 1 | 1 | 3 | 1-20 | 1-10 |
| Hex-4NPA | K | P | - | K | 3 | 1 | 0 | 1-25 | 1-10 |
| Hex-4NPA | K | P | - | K | 2 | 2 | 0 | 1-25 | 1-10 |
| Hex-4NPA | K | G | - | K | 1 | 1 | 0 | 1-50 | 1-10 |
| Hex-4NPA | K | P | - | K | 2 | 1 | 0 | 1-33 | 1-10 |
| Hex-4NPA | U | K | - | K | 1 | 1 | 0 | 1-50 | 1-10 |
| Hex-4NPA | K | G | P | K | 2 | 1 | 1 | 1-25 | 1-10 |
| Hex-4NPA | K | G | - | K | 2 | 2 | 0 | 1-25 | 1-10 |
| Hex-4NPA | K | R | P | K | 2 | 1 | 1 | 1-25 | 1-10 |
| 4NPA | K | pnaT | - | K | 2 | 1 | 0 | 1-33 | 1-10 |
| 4NPA | K' | pnaT | - | K | 2 | 1 | 0 | 1-33 | 1-10 |
| 4NPA | K | P | - | K | 2 | 2 | 0 | 1-25 | 1-10 |
| 4NPA | R | U | - | K | 1 | 1 | 0 | 1-50 | 1-10 |
| Hex-4NPA | Acp | P | - | K | 2 | 1 | 0 | 1-33 | 1-10 |
| Hex-K' | Aeg | P | - | K | 2 | 2 | 0 | 1-25 | 1-10 |
| Pra | K' | P | - | K | 2 | 2 | 0 | 1-25 | 1-10 |
| 4NPA | G | 4Ap | P | K | 1 | 1 | 1 | 1-33 | 1-10 |
| 4NPA | K' | P | - | K' | 2 | 1 | 0 | 1-33 | 1-10 |
| Hex-4NPA | K' | K | P | K | 1 | 1 | 1 | 1-33 | 1-10 |
| Hex-4NPA | Aeg | G | - | K | 1 | 2 | 0 | 1-33 | 1-10 |

In another embodiment, a tag within the scope of Formula 2 is provided, wherein PCS is a homopolymeric sequence of length f, and wherein the variables are selected from a variable set according to Table 5b

**Table 5b**

| **Tag Structure** | **U** | **PCS** | **V** | **f** | **e** |
|---|---|---|---|---|---|
| K'-[Apa]_{f}-Kₑ | K' | Poly-Apa | K or K' | 5-100 | 1-10 |
| K'-[Ds]_{f}-Kₑ | K' | Poly-Ds | K or K' | 5-100 | 1-10 |
| K'-[Ts]_{f}-Kₑ | K' | Poly-Ts | K or K' | 5-100 | 1-10 |
| K'-[4Ap]_{f}-Kₑ | K' | Poly-4Ap | K or K' | 5-100 | 1-10 |
| 4Npa-[K']_{f}-Kₑ | 4Npa | Poly-K' | K or K' | 5-100 | 1-10 |

In another embodiment, a tag within the scope of Formula 2 is provided, wherein PCS is a heteropolymeric sequence of a structure according to Formula 2c:

[REPEAT1]_{g}-[REPEAT2]ₕ-[REPEAT3]ᵢ-[REPEAT4]ⱼ **Formula 2c,**

wherein REPEAT3 and REPEAT4 are optional; REPEAT 1, REPEAT2, REPEAT3 (if present), and REPEAT4 (if present) are independently selected from the group consisting of K'₂-P, PEG, Aeg₂-P, Aeg₂-G, Aeg-OAhx-P, pnaT₂-P, pnaT₂-G, pnaT₂-K, (Kpeg4)₄-P, (G-Kpeg)₂-P, G-(Kpeg4)₂-P, Kpeg-G, (G-(Kpeg3))₂-P, (Aegpeg4)₂-P, and pnaC₂-P; g and h are integers from 1-30, i is an integer from 1-30 if REPEAT3 is present, j is an integer from 1-30 if REPEAT4 is present, g, h, i, and j are selected such that the length of PCS is ≤ 100. In an embodiment, REPEAT1 is selected from the group consisting of K'₂-P, PEG, Aeg₂-P, Aeg₂-G, and Aeg-OAhx-P; REPEAT2 is different from REPEAT1 and is selected from the group consisting of pnaT₂-P, pnaT₂-G, pnaT₂-K, (Kpeg4)₄-P, (G-Kpeg)₂-P, G-(Kpeg4)₂-P, Kpeg-G, (G-(Kpeg3))₂-P, (Aegpeg4)₂-P, pnaC₂-P, PEG, Aeg₂-P, Aeg-OAhx-P; REPEAT2 is different from REPEAT1 and is selected from the group consisting of pnaT₂-P, pnaT₂-G, pnaT₂-K, (Kpeg4)₄-P, (G-Kpeg)₂-P, G-(Kpeg4)₂-P, Kpeg-G, (G-(Kpeg3))₂-P, (Aegpeg4)₂-P, pnaC₂-P, PEG, Aeg₂-P, Aeg-OAhx-P; REPEAT3, if present, is different from REPEAT2 and is selected from the group consisting of K'₂-P, Aeg₂-P, Aeg₂-G, and pnaT₂-P; and REPEAT4, if present, is different from REPEAT3 and is Aeg₂-P. In a specific embodiment, REPEAT1 is PEG, g is from 12-36 (including 12-24, 12, or 24), REPEAT2 is selected from the group consisting of pnaT₂-P, pnaT₂-G, pnaT₂-K, and K', h is from 1-5 (including 1, 2, 3, 4, or 5), and REPEAT3 and REPEAT4 are absent. In another specific embodiment, REPEAT1 is PEG, g is 24, REPEAT2 is selected from the group consisting of pnaT₂-P, pnaT₂-G, pnaT₂-K, and K', h is 3, and REPEAT3 and REPEAT4 are absent. In another embodiment, REPEAT1 is selected from the group consisting of K'₂-P, Aeg₂-P, Aeg₂-G, and Aeg-OAhx-P; g is 3-10 (including 3, 4, 5, 6, 7, 8, 9, and 10); REPEAT2 is different from REPEAT1 and is selected from the group consisting of pnaT₂-P, pnaT₂-G, pnaT₂-K, (Kpeg4)₄-P, (G-Kpeg)₂-P, G-(Kpeg4)₂-P, Kpeg-G, (G-(Kpeg3))₂-P, (Aegpeg4)₂-P, pnaC₂-P, Aeg₂-P, and Aeg-OAhx-P; h is from 1-5 (including h=1, h=2, h=3, h=4, and h=5); and REPEAT3 and REPEAT4 are absent. In another embodiment, REPEAT1 is selected from the group consisting of K'₂-P, Aeg₂-P, Aeg₂-G, and Aeg-OAhx-P; g is 3-10 (including 3, 4, 5, 6, 7, 8, 9, and 10); REPEAT2 is different from REPEAT1 and is selected from the group consisting of pnaT₂-P, pnaT₂-G, pnaT₂-K, (Kpeg4)₄-P, (G-Kpeg)₂-P, G-(Kpeg4)₂-P, Kpeg-G, (G-(Kpeg3))₂-P, (Aegpeg4)₂-P, pnaC₂-P, Aeg₂-P, and Aeg-OAhx-P; h is from 1-5 (including h=1, h=2, h=3, h=4, and h=5); and REPEAT3 is different from REPEAT2 and is selected from the group consisting of K'₂-P, Aeg₂-P, Aeg₂-G, and pnaT₂-P; i is from 1-5 (including i=1, i=2, i=3, i=4, and i=5); REPEAT4, if present, is different from REPEAT3 and is Aeg₂-P; and if REPEAT4 is present, j=1 or j=2. In another embodiment, REPEAT1 is selected from the group consisting of K'₂-P, Aeg₂-P, Aeg₂-G, and Aeg-OAhx-P; g is 3-10 (including 3, 4, 5, 6, 7, 8, 9, and 10); REPEAT2 is PEG; h is 12; REPEAT3 is selected from the group consisting of K'₂-P, Aeg₂-P, Aeg₂-G, and pnaT₂-P; i is from 1-5 (including i=1, i=2, i=3, i=4, and i=5); and REPEAT4 is absent.

In an embodiment, U, V, e, REPEAT1, REPEAT2, REPEAT3, and REPEAT4 are selected from a variable set according to Table 5c

**Table 5c**

| **U** | **REPEAT1** | **REPEAT2** | **REPEAT3** | **REPEAT4** | **V** | **e** |
|---|---|---|---|---|---|---|
| hex-4NPA | K'₂-P | pnaT₂-P | K'₂-P | - | K or K' | 1-10 |
| 4NPA | PEG | pnaT₂-P | - | - | K or K' | 1-10 |
| 4NPA | PEG | pnaT₂-G | - | - | K or K' | 1-10 |
| 4NPA | PEG | pnaT₂-K | - | - | K or K' | 1-10 |
| 4NPA | PEG | K' | - | - | K or K' | 1-10 |
| 4NPA | Aeg₂-P | pnaT₂-P | Aeg₂-P | - | K or K' | 1-10 |
| 4NPA | K'₂-P | (Kpeg4)₄-P | K'₂-P | - | K or K' | 1-10 |
| 4NPA | K'₂-P | (G-Kpeg)₂-P | K'₂-P | - | K or K' | 1-10 |
| 4NPA | K'₂-P | pnaT₂-P | K'₂-P | - | K or K' | 1-10 |
| Hex-4NPA | Aeg₂-G | pnaT₂-P | Aeg₂-G | - | K or K' | 1-10 |
| Hex-4NPA | Aeg₂-P | G-(Kpeg4)₂-P | Aeg₂-P | - | K or K' | 1-10 |
| Hex-4NPA | Aeg₂-P | Kpeg-G | Aeg₂-P | - | K or K' | 1-10 |
| Hex-4NPA | Aeg₂-P | (G-(Kpeg3))₂-P | Aeg₂-P | - | K or K' | 1-10 |
| Hex-4NPA | Aeg₂-P | (Aegpeg4)₂-P | Aeg₂-P | - | K or K' | 1-10 |
| Hex-4NPA | Aeg₂P | pnaC₂-P | Aeg₂-P | - | K or K' | 1-10 |
| Hex-4NPA | Aeg₂-P | PEG | Aeg₂-P | - | K or K' | 1-10 |
| Hex-4NPA | Aeg-OAhx-P | pnaT₂-P | Aeg₂-P | - | K or K' | 1-10 |
| Hex-4NPA | Aeg-OAhx-P | Aeg₂-P | pnaT₂-P | Aeg₂-P | K or K' | 1-10 |
| Hex-4NPA | Aeg₂-P | Aeg-OAhx-P | pnaT₂-P | Aeg₂-P | K or K' | 1-10 |
| Hex-4NPA | Aeg₂-P | Aeg-OAhx-P | - | - | K or K' | 1-10 |

In an embodiment, the tag as described in Table 5a, 5b, or 5c is provided, wherein the C-terminal carboxylic acid of V is replaced with an amide.

Any of the polymer tags as described herein may be covalently linked to a nucleoside oligophosphate. In some embodiments, the polymer tag is covalently attached to the base of the nucleoside. In other embodiments, the polymer tag is covalently linked to the sugar moiety of the nucleoside. In yet other embodiments, the polymer tag is covalently linked to a phosphate group. In an embodiment, the tag is attached to the nucleoside oligophosphate such that the "U" moiety (if present) proximate to the nucleoside oligophosphate and the "V" moiety is distal from the nucleoside oligophosphate. In another embodiment, the tag is attached to the nucleoside oligophosphate via the N-terminal amino acid of the tag, either directly or via a linker moiety.

In an embodiment, a tagged nucleoside oligophosphate is provided, having a structure according to Formula 3: 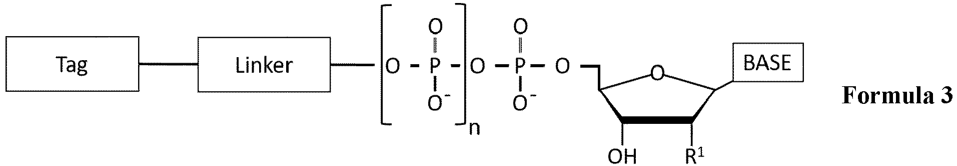 wherein Base is selected from adenine, cytosine, guanine, thymine, and uracil; R¹ is H, OH, O-CHs, or F; n is from 2 to 12; Tag is any of the polymer tags having the net-positive charge as described above; and Linker is a chemical moiety resulting from covalently bonding an untagged nucleotide oligophosphate to Tag. Exemplary Linker structures include ester, ether, thioether, amine, amide, imide, carbonate, carbamate, squarate, thiazole, thiazolidine, hydrazone, oxime, triazole, dihydropyridazine, phosphodiester, polyethylene glycol (PEG), Pictet-Spengler adduct, and any combination thereof. In an embodiment, Linker has a structure of Formula 4:

R⁴-R²-R³ **Formula 4**

wherein R² is selected from the group consisting of ester linkage, ether linkage, thioether linkage, amine linkage, amide linkage, imide linkage, carbonate linkage, carbamate linkage, squarate linkage, thiazole linkage, thiazolidine linkage, hydrazone linkage, oxime linkage, triazole linkage, dihydropyridazine linkage, phosphodiester linkage, polyethylene glycol (PEG) linkage, Pictet-Spengler adduct, and any combination thereof, R³ comprises a saturated or unsaturated, branched or unbranched, substituted or unsubstituted carbon chain at least 2 carbons in length covalently bonded at one end to one of the phosphate moieties of the nucleotide oligophosphate and at the other end to R², and R⁴ comprises a saturated or unsaturated, branched or unbranched, substituted or unsubstituted carbon chain at least 2 carbons in length covalently bonded at one end to R² and at the other end to Tag. In an embodiment, carbon chains R³ and R⁴ are each less than 20 carbons in length. In an embodiment, R³ is from 5 to 20 carbons in length and R⁴ is from 2 to 12 carbons in length. In an embodiment, R³ is from 5 to 20 carbons in length and R⁴ is from 2 to 12 carbons in length, with the proviso that R³ is longer than R⁴. In an embodiment, the bond between LINKER and the oligophosphate involves the terminal phosphate group. In an embodiment, the bond between TAG and LINKER involves an amino group of the N-terminal residue of the TAG. In an embodiment, the carboxy group of the C-terminal residue of TAG is converted to an amide, and the bond between TAG and LINKER involves the amino group of the C-terminal amide of TAG. In an embodiment, the bond between TAG and LINKER involves a carboxy group C-terminal residue of TAG. Schemes for generating Linker structures of Formula 4 include those set forth in US 2018-0057870. In an embodiment, Linker has a structure of Formula 4, wherein R² is a triazole of Formula 4a: 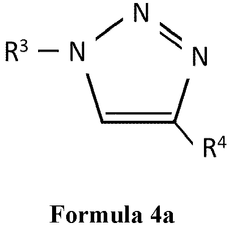 wherein the sum of the carbons in carbon chain R₃ and carbon chain R₄ is ≤ 96. In an embodiment, Tag is a peptide chain and Linker has a structure of Formula 4, wherein R² is a triazole of Formula 4b: 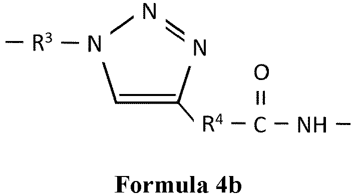 and wherein the -NH- of the peptide bond connected to R⁴ is contributed by the N-terminal amino acid or amino acid analog of the peptide chain.

### V. SBS sequencing systems and methods

In an embodiment, a system for performing nucleic acid sequencing-by-synthesis (SBS) is provided, the system comprising: (a) a nanopore having a channel bearing sufficient negatively charged moieties to substantially repel template and/or primer nucleic acid, (b) a nucleic acid polymerase associated with the nanopore, (c) a set of nucleotide oligophosphates disposed in an electrolyte solution, said nucleotide oligophosphates comprising a positively-charged tag capable of threading through the nanopore of (a), and (d) at least one electrode position to record a characteristic of a current flowing through the channel.

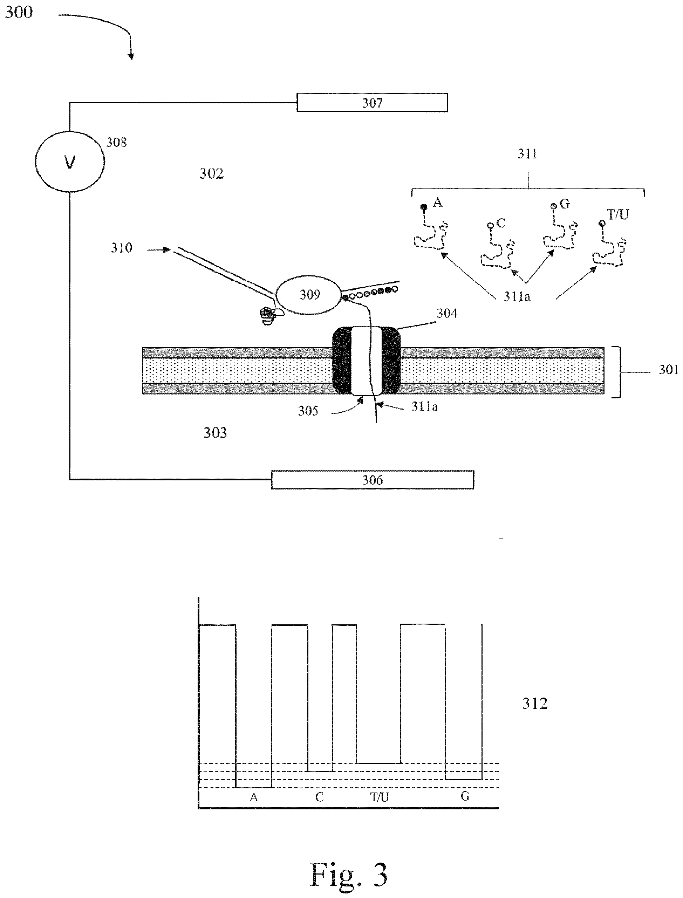

FIG. 3 illustrates an exemplary embodiment of a nanopore sequencing complex 300 for performing a tag-based SBS nucleotide sequencing. An electrically-resistive barrier 301 separates a bulk electrolyte solution 302 from a second electrolyte solution 303. A nanopore 304 is disposed in the electrically-resistive barrier 301, and the channel of the nanopore 305 provides a path through which ions can flow between the bulk electrolyte 302 and the second electrolyte 303. A working electrode 306 is disposed on the side of the electrically-resistive barrier 301 containing the second electrolyte 303 (termed the *"trans* side" of the electrically-resistive barrier) and positioned near the nanopore 304. A counter electrode 307 is positioned on the side of the electrically-resistive barrier 301 containing the bulk electrolyte 302 (termed the *"cis* side" of the electrically-resistive barrier). A signal source 308 is adapted to apply a voltage signal between the working electrode 306 and the counter electrode 307. A polymerase 309 is associated with nanopore 304, and a primed template nucleic acid 310 is associated with the polymerase. The bulk electrolyte 302 includes four different polymer-tagged nucleoside oligophosphates 311 (tag illustrated as 311a). The polymerase 309 catalyzes incorporation of the polymer-tagged nucleotides 311 into an amplicon of the template. When a polymer-tagged nucleoside oligophosphate 311 is correctly complexed with polymerase 309, the tag 311a can be pulled (e.g., loaded) into the nanopore by an electrical force, such as a force generated in the presence of an electric field generated by a voltage applied across the electrically-resistive barrier 301 and/or nanopore 304. While the tag 311a occupies the channel of the nanopore 304, it affects ionic flow through the nanopore 304, thereby generating an ionic blockade signal 312. Each nucleotide 311 has a unique polymer tag 311a that generates a unique ionic blockade signal due to the distinct chemical structure and/or size of the tag 311a. By identifying the unique ionic blockade signal 312, the identity of the unique tags 311a (and therefore, the nucleotide 310 with which it is associated) can be identified. This process is repeated iteratively with each nucleotide 310 incorporated into the amplicon.

### VA. Nanopores for use in the system

The nanopores of the present systems comprise a concentration of negative charge disposed in or near the channel of the nanopore. Without being bound by theory, it is believed that the negative charges repel the negatively charged nucleic acids, and thus discourage insertion of templates, amplicons, primers, etc. into the nanopore.

In an embodiment, the nanopore is a biological nanopore. Commonly used proteins for generating biological nanopores include α-hemolysin (α-HL) from *Staphylococcus aureus* (canonical full-length unprocessed sequence disclosed at Uniprot Accession No. P09616-1 (SEQ ID NO: 20)), outer membrane porin G (OmpG) nanopore from *Escherichia coli* (canonical full-length unprocessed sequence disclosed at Uniprot Accession No. P76045-1 (SEQ ID NO: 21)), and *Mycobacterium smegmatis* porin A (MspA) (canonical full-length unprocessed sequence disclosed at Uniprot Accession No. A0QR29-1 (SEQ ID NO: 22)). Other exemplary biological nanopores include leukocidin nanopore, outer membrane porin F (OmpF) nanopore, cytolysin A (ClyA) nanopore, outer membrane phospholipase A nanopore, *Neisseria* autotransporter lipoprotein (NalP) nanopore, WZA nanopore, *Nocardia farcinica* NfpA/NfpB cationic selective channel nanopore, lysenin nanopore, aerolysin, and Curlin sigma S-dependent growth subunit G (CsgG) nanopore. In yet other embodiments, the nanopore is a hybrid nanopore comprising a biological nanopore. In other embodiments, the nanopore is a solid state nanopore that comprises a negative charge localized to the opening of the channel.

### VA1. α-HL nanopores

α-HL nanopores are heptameric structures formed from 7 monomeric subunits of the α-HL polypeptide from *Staphylococcus aureus.* Various approaches for engineering α-HL nanopores for use in nanopore-based sequencing can be found at, for example, Ayub, Wang II, WO 2014/100481, WO 2016/069806, WO 2017/050718, WO 2017/184866, and WO 2018/002125. As illustrated at FIG. 2A, α-HL nanopores have a cap region 201 and a beta barrel region 202, with a channel 203 extending axially through the cap and stem regions. FIG. 2B is a cross-section of the α-HL nanopore showing the channel 203, which can be divided into a vestibule 204, a constriction site 205, a beta barrel body 206, and a beta barrel exit 207. References herein to "beta barrel region" includes each of the constriction site 205, the beta barrel body 206, and the beta barrel exit 207. References herein to "α-HL nanopore" shall refer to heptameric pores of 7 α-HL monomeric subunits.

The α-HL nanopores useful in the present systems have a channel comprising a non-native negatively-charged amino acid at a plurality of solvent-facing positions within the channel. In an embodiment, one or more of the α-HL monomeric subunits (including 1, 2, 3, 4, 5, 6, or 7 of the subunits) of the nanopore have at least one non-native negatively-charged amino acid (such as aspartic acid or glutamic acid) at a position corresponding to the vestibule region and/or the beta barrel region of the α-HL nanopore. An amino acid sequence corresponding to a wild-type α-HL monomeric subunit can be found at SEQ ID NO: 1. Unless otherwise indicated, all amino acid numbering relating to α-HL monomeric subunits are with reference to SEQ ID NO: 1. When reference is made to an α-HL monomeric subunit "comprising substitution at position #" or "comprising a substitution X#Y" it shall be understood to mean that the monomeric subunit amino acid sequence, when aligned with SEQ ID NO: 1, has a substitution at the position corresponding to the recited position of SEQ ID NO: 1. As used herein, a "non-native amino acid" is an amino acid at a position of the monomeric subunit amino acid sequence that represents a substitution or insertion when aligned with SEQ ID NO: 1. In an embodiment, the polypeptides comprise at least one α-HL monomeric subunits having at least 75% identity, at least 80% identity, at least 85% identity, at least 90% identity, at least 91% identity, at least 92% identity, at least 93% identity, at least 94% identity, or at least 95% identity with SEQ ID NO: 1.

Sufficient non-native negatively charged amino acids are provided that (a) the template and/or primer nucleic acids are less likely to insert into the pore relative to an α-HL nanopores having the native amino acid residue, and (b) positively-charged tags can translocate through the channel of the nanopore. Exemplary α-HL nanopores are disclosed above in section II.

### VA2. ompG nanopores

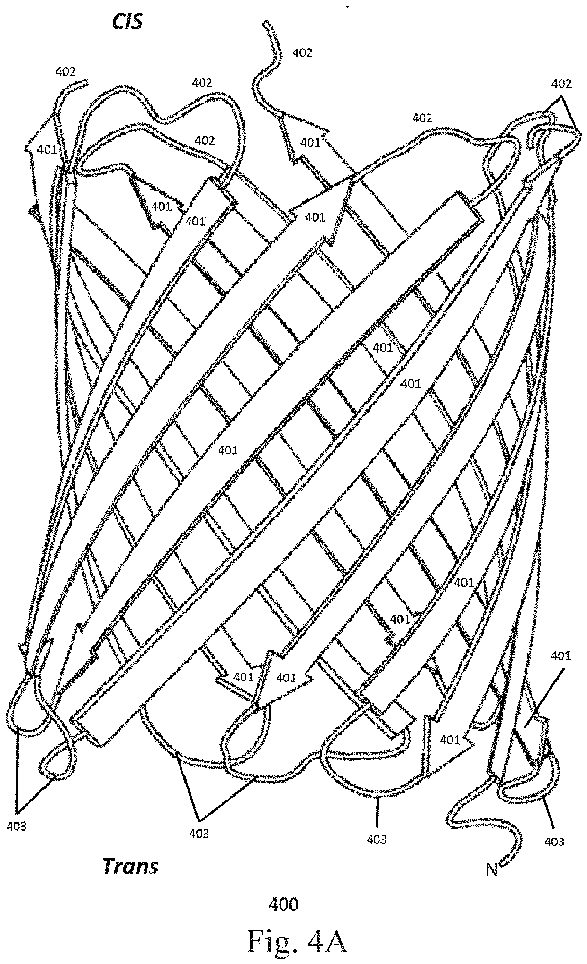

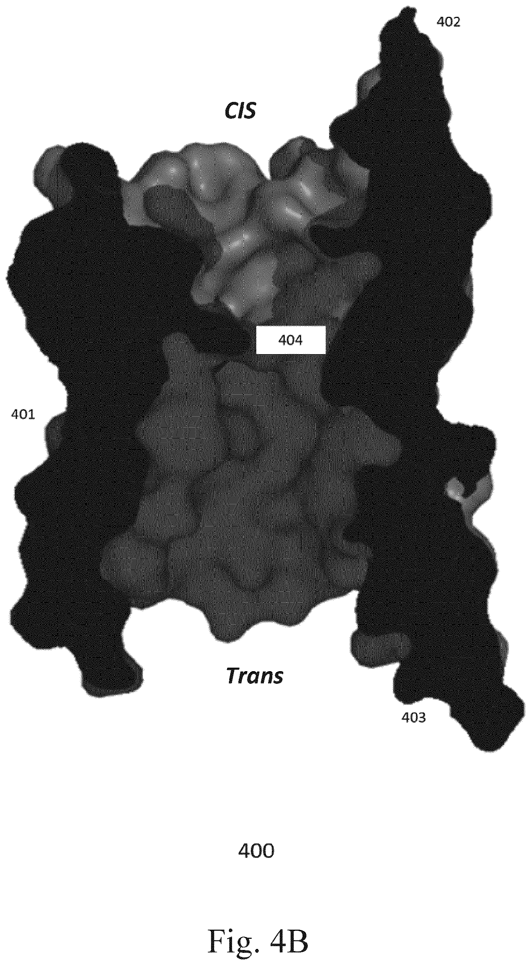

In an embodiment, the nanopore is an ompG nanopore or a hybrid nanopore comprising an ompG nanopore as the biological component. As illustrated at FIGS. 4A and 4B, ompG nanopores **400** are formed from a single chain polypeptide arranged with the following structural features: a channel comprising 14 beta strands β1-β14 **401;** 7 loop regions: L1-L7 **402;** and 6 short turns T1-T6 **403.** A constriction site **404** is located inside the channel. The channel has a *cis* side **405,** which corresponds to the extracellular portion in the native protein, and a *trans* side **406,** which corresponds to the intracellular portion in the native protein. References describing engineered ompG nanopores for use in nanopore-based sequencing apparatuses include Gari, Grosse, Chen II, and WO 2017/050722.

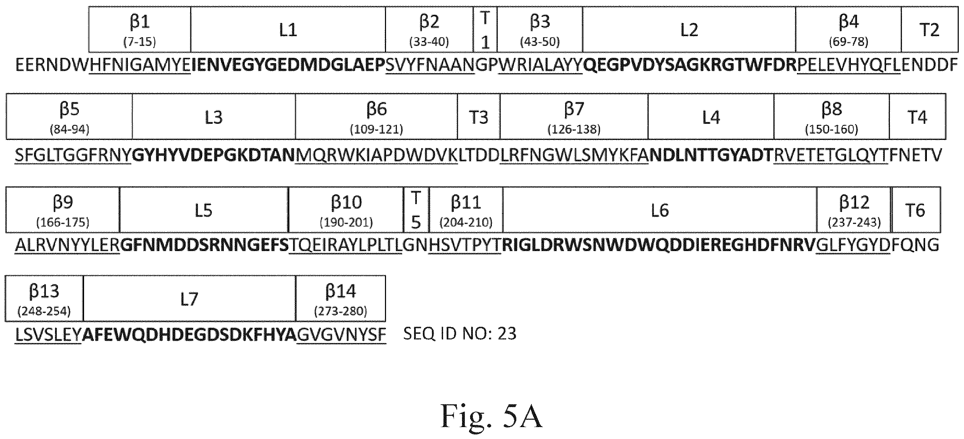

An exemplary polypeptide used to form ompG nanopores is disclosed at SEQ ID NO: 23, which is a mature form of SEQ ID NO: 21 lacking the 21 amino acid N-terminal signal peptide. Amino acids corresponding to the beta strands, loops, and turns of SEQ ID NO: 23 are illustrated at Fig. 5A. As illustrated in Fig. 5A, the beta strands correspond to the following amino acid residues of SEQ ID NO: 23: β1 = 7-15, β2 = 33-40, β3 = 43-50, β4 = 69-78, β5 = 84-94, β6 = 109-121, β7 = 126-138, β8 = 150-160, β9 = 166-175, β10 = 190-201, β11 = 204-210, β12 = 237-243, β13 = 248-254, and β14 = 273-280.

In an embodiment, the nanopore is an ompG nanopore comprising a channel formed by a set of beta strands β1-β14 having at least 90% identity to beta strands β1-β14 of SEQ ID NO: 23, wherein the set of beta strands β1-β14 has at least one non-native negatively charged amino acid relative to beta strands β1-β14 of SEQ ID NO: 23. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids.

In an embodiment, the ompG nanopore comprises an amino acid sequence having at least 70%, at least 75%, at least 80%, at least 85%, at least 90%, or at least 95% sequence identity to SEQ ID NO: 23, the ompG nanopore having a channel comprising a set of beta strands β1-β14 having at least one non-native negatively charged amino acid relative to beta strands β1-β14 of SEQ ID NO: 23. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids.

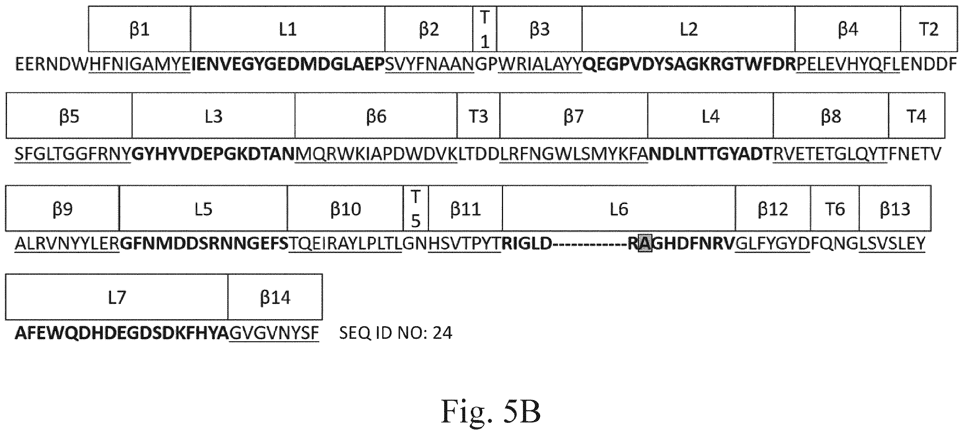

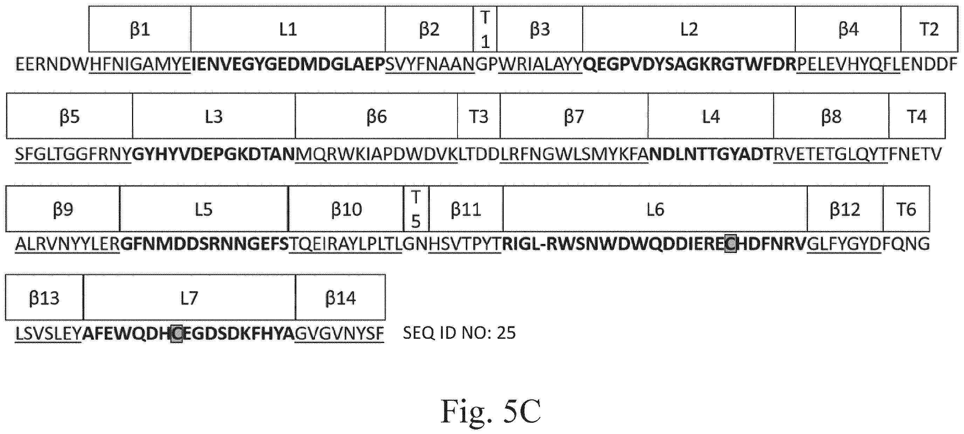

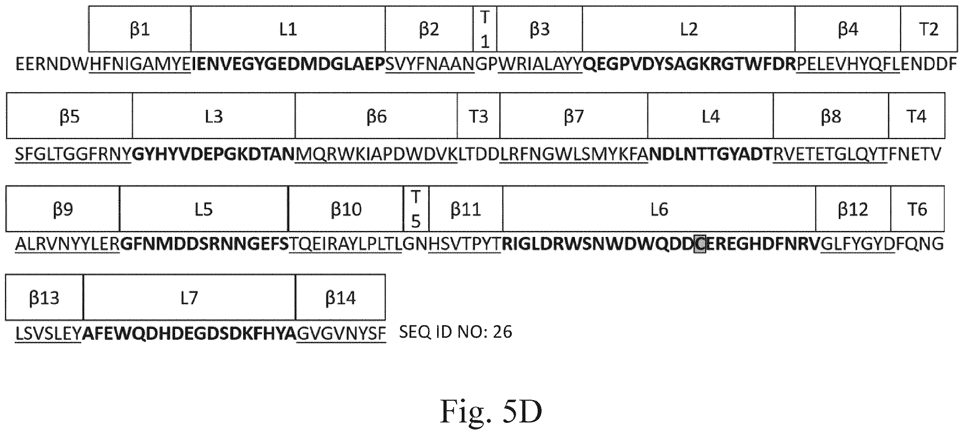

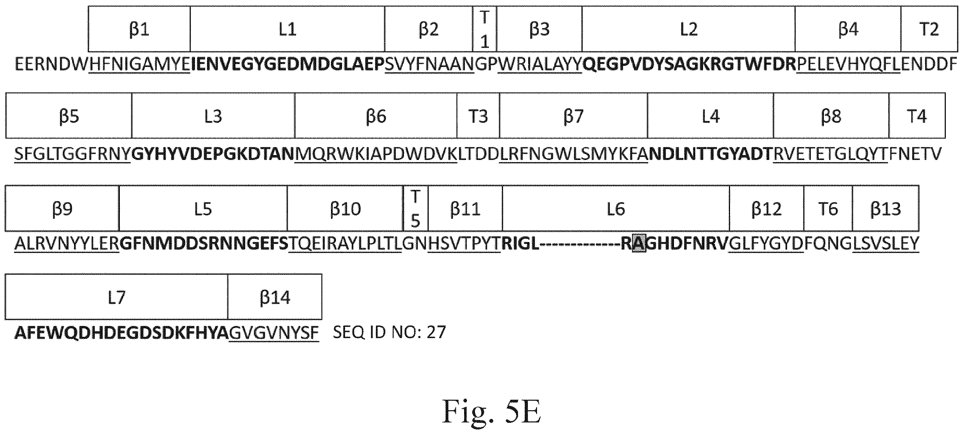

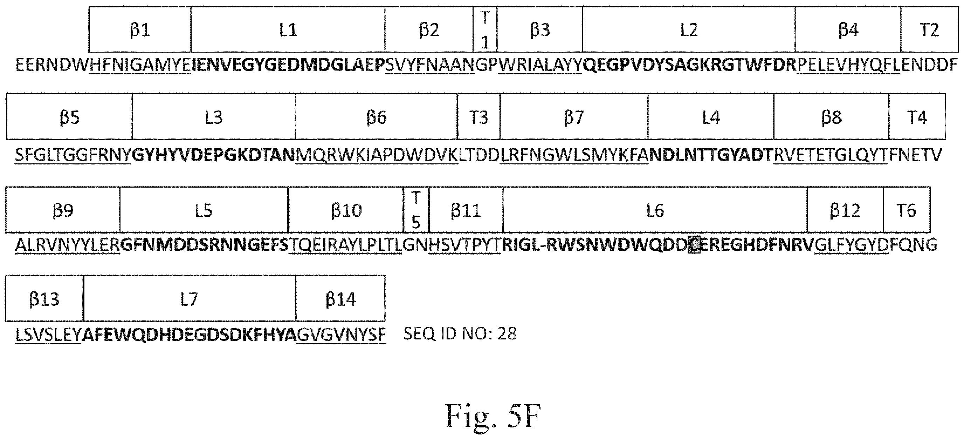

The ompG nanopores disclosed herein can contain additional modifications that improve the ability of the nanopore to be used in biosensing. For example, it is known that loop region L6 causes pore gating - spontaneous blocking of current through the pore during an applied potential. Different strategies have been used to mitigate pore gating, including truncation of the loop (*see, e.g*., Gari, Grosse, & WO 2017/050722), reducing mobility of the loop by, for example, introducing a disulfide bond by adding cysteine residues to the extracellular ends of strands β12 and β13 (such as G230 and D262 of SEQ ID NO: 23) (Chen II), and/or introducing a lipid anchor into L6, for example, by alkylation of an engineered cysteine (such as, for example, by introduction of an I226C substitution). Mobility of loop L6 may further be minimized by optimizing hydrogen bonding between strands β11 and β12 by deletion of residue D215 (Chen II & WO 2017/050722). An exemplary ompG polypeptide having a truncated loop region L6 is disclosed herein at SEQ ID NO: 24, corresponding to SEQ ID NO: 23 with ΔR216-E227 and E229A modifications. Changes relative to SEQ ID NO: 23 are illustrated at Fig. 5B, with deleted residues illustrated by dash marks (-) and substitutions illustrated by shaded boxes. An exemplary ompG polypeptide having a stabilized loop region and stabilization of hydrogen bonding between strands β11 and β12 is disclosed herein at SEQ ID NO: 25, corresponding to SEQ ID NO: 23 with G230C, D262C, and ΔD215 modifications. Changes relative to SEQ ID NO: 23 are illustrated at Fig. 5C, with deleted residues illustrated by dash marks (-) and substitutions illustrated by shaded boxes. An exemplary ompG polypeptide having an engineered cysteine in loop L6 to which a lipid anchor can be attached is disclosed herein at SEQ ID NO: 26, corresponding to SEQ ID NO: 23 with an I226C substitution. Changes relative to SEQ ID NO: 23 are illustrated at Fig. 5D, with deleted residues illustrated by dash marks (-) and substitutions illustrated by shaded boxes. An exemplary ompG polypeptide having a truncated loop region L6 and stabilization of hydrogen bonding between strands β11 and β12 is disclosed herein at SEQ ID NO: 27, corresponding to SEQ ID NO: 23 with ΔD215-E227 + E229A modifications. Changes relative to SEQ ID NO: 23 are illustrated at Fig. 5E, with deleted residues illustrated by dash marks (-) and substitutions illustrated by shaded boxes. An exemplary ompG polypeptide having an engineered cysteine in loop L6 to which a lipid anchor can be attached and stabilization of hydrogen bonding between strands β11 and β12 is disclosed herein at SEQ ID NO: 28, corresponding to SEQ ID NO: 23 with a ΔD215 + I226C modifications. Changes relative to SEQ ID NO: 23 are illustrated at Fig. 5F, with deleted residues illustrated by dash marks (-) and substitutions illustrated by shaded boxes.

In an embodiment, the nanopore is an ompG nanopore comprising: (a) a channel formed by a set of beta strands β1-β14 having at least 90% identity to beta strands β1-β14 of SEQ ID NO: 23, wherein the set of beta strands β1-β14 has at least one non-native negatively charged amino acid relative to beta strands β1-β14 of SEQ ID NO: 23, (b) a modification selected from the group consisting of a truncated or deleted loop region L6 or a stabilized loop region L6, and optionally (c) a modification that stabilizes of hydrogen bonding between strands β11 and β12. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids.

In an embodiment, the nanopore is an ompG nanopore comprising: (a) a channel formed by a set of beta strands β1-β14 having at least 90% identity to beta strands β1-β14 of SEQ ID NO: 23, wherein the set of beta strands β1-β14 has at least one non-native negatively charged amino acid relative to beta strands β1-β14 of SEQ ID NO: 23, and (b) a loop region L6 having ΔR216-E227 and E229A modifications relative to SEQ ID NO: 23. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids. In an embodiment, the nanopore is an ompG nanopore having at least 70%, at least 75%, at least 80%, at least 85%, at least 90%, or at least 95% sequence identity to SEQ ID NO: 24.

In an embodiment, the nanopore is an ompG nanopore comprising: (a) a channel formed by a set of beta strands β1-β14 having at least 90% identity to beta strands β1-β14 of SEQ ID NO: 23, wherein the set of beta strands β1-β14 has at least one non-native negatively charged amino acid relative to beta strands β1-β14 of SEQ ID NO: 23, and (b) G230C, D262C, and ΔD215 modifications relative to SEQ ID NO: 23. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids. In an embodiment, the nanopore is an ompG nanopore having at least 70%, at least 75%, at least 80%, at least 85%, at least 90%, or at least 95% sequence identity to SEQ ID NO: 25.

In an embodiment, the nanopore is an ompG nanopore comprising: (a) a channel formed by a set of beta strands β1-β14 having at least 90% identity to beta strands β1-β14 of SEQ ID NO: 23, wherein the set of beta strands β1-β14 has at least one non-native negatively charged amino acid relative to beta strands β1-β14 of SEQ ID NO: 23, and (b) an I226C modification relative to SEQ ID NO: 23, wherein said cysteine optionally is alkylated. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids. In an embodiment, the nanopore is an ompG nanopore having at least 70%, at least 75%, at least 80%, at least 85%, at least 90%, or at least 95% sequence identity to SEQ ID NO: 26.

In an embodiment, the nanopore is an ompG nanopore comprising: (a) a channel formed by a set of beta strands β1-β14 having at least 90% identity to beta strands β1-β14 of SEQ ID NO: 23, wherein the set of beta strands β1-β14 has at least one non-native negatively charged amino acid relative to beta strands β1-β14 of SEQ ID NO: 23, and (b) ΔD215-E227 + E229A modifications relative to SEQ ID NO: 23. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids. In an embodiment, the nanopore is an ompG nanopore having at least 70%, at least 75%, at least 80%, at least 85%, at least 90%, or at least 95% sequence identity to SEQ ID NO: 27.

In an embodiment, the nanopore is an ompG nanopore comprising: (a) a channel formed by a set of beta strands β1-β14 having at least 90% identity to beta strands β1-β14 of SEQ ID NO: 23, wherein the set of beta strands β1-β14 has at least one non-native negatively charged amino acid relative to beta strands β1-β14 of SEQ ID NO: 23, (b) an I226C modification relative to SEQ ID NO: 23, wherein said cysteine optionally is alkylated, and (c) a ΔD215 modification relative to SEQ ID NO: 23. In an embodiment, the set of beta strands β1-β14 has at least 3, at least 4, at least 5, at least 6, at least 7, at least 8, at least 9, at least 10, at least 11, at least 12, at least 13, or at least 14 non-native negatively charged amino acids relative to beta strands β1-β14 of SEQ ID NO: 23. In an embodiment, at least a portion of the non-native negatively charged amino acids is localized around the constriction site. In an embodiment, at least a portion of the non-native negatively charged amino acids is localized around the *cis* entrance to the channel. In an embodiment, at least a portion of the non-native negatively charged amino acids is localized around the *trans* exit to the channel. In an embodiment, at least a portion of the non-native negatively charged amino acids is localized around the *cis* entrance to the channel and at least a portion of the non-native negatively charged amino acids is localized around the *trans* exit to the channel. In an embodiment, at least a portion of the non-native negatively charged amino acids is localized around the constriction site and at least a portion of the non-native negatively charged amino acids is localized around the *trans* exit to the channel. In an embodiment, at least 3, at least 4, at least 5, at least 6, at least 7, at least 8, at least 9, at least 10, at least 11, at least 12, at least 13, or each of beta strands β1-β14 comprises at least 1 of the non-native negatively charged amino acids. In an embodiment, the nanopore is an ompG nanopore having at least 70%, at least 75%, at least 80%, at least 85%, at least 90%, or at least 95% sequence identity to SEQ ID NO: 28.

### VA3. MspA nanopores

In an embodiment, the nanopore is a MspA nanopore. MspA nanopores typically comprise 8 MspA monomeric subunits. References describing engineered MspA nanopores for use in nanopore-based sequencing apparatuses include Butler, Manrao, Pavlenok, WO 2013/098562, US 2014-0309402, US 2013-0146457, and Wang II. An exemplary amino acid sequence used to engineer MspA monomeric subunits for use in nanopore-based sequencing is disclosed herein at SEQ ID NO: 29. MspA nanopores for use in nanopore-based nucleic acid sequencing typically have a neutral constriction site (for example, by making D90N, D91N, and D93N substitutions relative to SEQ ID NO: 29) and positively charged amino acids near the *cis* channel entrance (for example, by substituting one or more of D118, D134, E139 of SEQ ID NO: 29 with a positively charged amino acid, such as D118R, D134R, and/or E139K substitutions). These changes typically are made to enhance the interaction of single stranded DNA with the pore. In contrast, the MspA nanopores of the present system comprise a negatively charged amino acid at each of these positions.

In an embodiment, an octameric MspA nanopore is provided comprising 8 monomeric subunits having at least 70, at least 80, at least 90, or at least 95% identity with SEQ ID NO: 29, wherein at least 4, at least 5, at least 6, at least 7, or all 8 of the monomeric subunits have an aspartic acid or glutamic acid at positions corresponding to D90, D91, D93, D118, D134, and E139 of SEQ ID NO: 29.

### VB. Tagged nucleoside oligophosphate sets

The system further comprises a set of at least 4 tagged nucleoside oligophosphates, the set comprising an adenosine nucleoside oligophosphate having a first polymer tag, a cytosine nucleoside oligophosphate having a second polymer tag, a guanine nucleoside oligophosphate having a third polymer tag, and either a thymine nucleoside oligophosphate having a fourth polymer tag or a uracil nucleoside oligophosphate having a fourth polymer tag, and each of the first through fourth polymer tags has the following characteristics: (a) is capable of occupying the channel of the nanopore in a manner that generates an ionic blockade signal that is detectable under normal operating conditions of the nanopore sequencing complex; (b) is capable of being released from the nucleoside oligophosphate after the ionic blockade signal is generated; and (c) is capable of flowing through the nanopore. In some embodiments, each ionic blockade signal generated by each polymer tag is distinguishable from the ionic blockade signal generated by each of the other polymer tags under normal operating conditions of the nanopore sequencing complex. In other embodiments (for example, when the nucleoside oligophosphates are intended to be flowed one-at-a-time onto the system), at least some of the polymer tags may generate ionic blockade signals that are effectively indistinguishable from one another under normal operating conditions of the nanopore sequencing complex.

In an embodiment, the polymer tags have a net-neutral to net-positive charge. In some embodiments, at least one of the at least 4 nucleoside oligophosphates has a net-positively charged polymer tag. In some embodiments, at least 2 of the at least 4 tagged nucleoside oligophosphates has a net-positively charged polymer tag. In some embodiments, at least 3 of the at least 4 tagged nucleoside oligophosphates has a net-positively charged polymer tag. In some embodiments, at least 4 of the tagged nucleoside oligophosphates has a net-positively charged polymer tag. In some embodiments, each tagged nucleoside oligophosphates has a net-positively charged polymer tag. In an embodiment, the net-positively charged polymer tag is a peptide tag according to Formula 2, Formula 2a, or Formula 2b. In an embodiment, the tagged nucleoside oligophosphate set comprises a deoxyadenosine oligophosphate (dAP), a deoxycytosine (dCP) oligophosphate, a deoxyguanine oligophosphate (dGP), and a deoxythymine oligophosphate (dTP), wherein each of dAP, dCP, dGP, and dTP is tagged with a peptide tag according to Formula 2, including but not limited to the peptide tags set forth in Table 5. In an embodiment, each of dAP, dCP, dGP, and dTP has a structure according to Formula 3, wherein TAG is the peptide tag according to Formula 2, 2a, or 2b, including but not limited to the peptide tags set forth in Table 5.

### VC. Nucleic acid polymerases

The present systems and methods may incorporate any nucleic acid polymerase that is useful in SBS sequencing and is capable of sequence-specific polymerization of nucleoside polyphosphates having 4, 5, 6, 7, 8 or more phosphates.

In some embodiments, the DNA-dependent DNA polymerase is a variant of a naturally occurring polypeptide having DNA-dependent DNA polymerase activity, such as Pol6, phi29 DNA polymerase, T7 DNA pol, T4 DNA pol, E. coli DNA pol 1, Klenow fragment, as well as associated subunits and cofactors. In an embodiment, the DNA-dependent DNA polymerase is a DNA polymerase derived from Pol6. A His-tagged wild-type sequence of Pol6 is available at SEQ ID NO: 30. Exemplary Pol6 derivatives useful in nanopore-based sequencing are disclosed at, for example, US 2016/0222363, US 2016/0333327, US 2017/0267983, US 2018/0094249, and US 2018/0245147.

### VD. Nanopore Sensor Chips

In some embodiments, a nanopore sensor chip is provided, comprising an array of nanopore cells comprising a nanopore sequencing complex as described above.

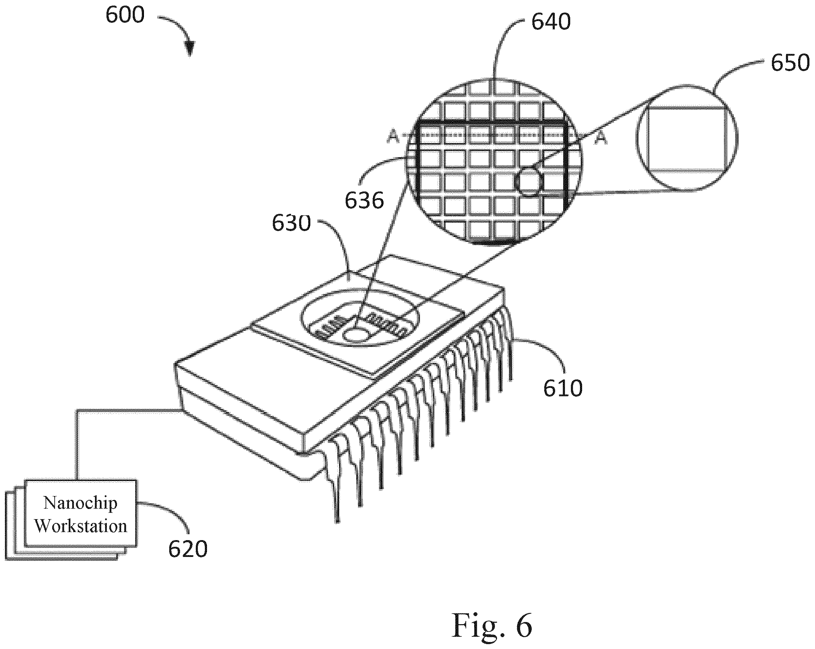

FIG. 6 is a top view of an embodiment of a nanopore sensor chip 600 having an array 640 of nanopore cells 650. Each nanopore cell 650 includes a control circuit integrated on a silicon substrate of nanopore sensor chip 600. In some embodiments, side walls 636 are included in array 740 to separate groups of nanopore cells 650 so that each group can receive a different sample for characterization. Each nanopore cell can be used to sequence a nucleic acid. In some embodiments, nanopore sensor chip 600 includes a cover plate 630. In some embodiments, nanopore sensor chip 600 also includes a plurality of pins 610 for interfacing with other circuits, such as a computer processor.

In some embodiments, nanopore sensor chip 600 includes multiple chips in a same package, such as, for example, a Multi-Chip Module (MCM) or System-in-Package (SiP). The chips can include, for example, a memory, a processor, a field-programmable gate array (FPGA), an application-specific integrated circuit (ASIC), data converters, a high-speed I/O interface, etc.

In some embodiments, nanopore sensor chip 600 is coupled to (e.g., docked to) a nanochip workstation 620, which can include various components for carrying out (e.g., automatically carrying out) various embodiments of the processes disclosed herein. These process can include, for example, analyte delivery mechanisms, such as pipettes for delivering lipid suspension or other membrane structure suspension, analyte solution, and/or other liquids, suspension or solids. The nanochip workstation components can further include robotic arms, one or more computer processors, and/or memory. A plurality of polynucleotides can be detected on array 640 of nanopore cells 650. In some embodiments, each nanopore cell 650 is individually addressable.

### A. Nanopore sequencing cell structure

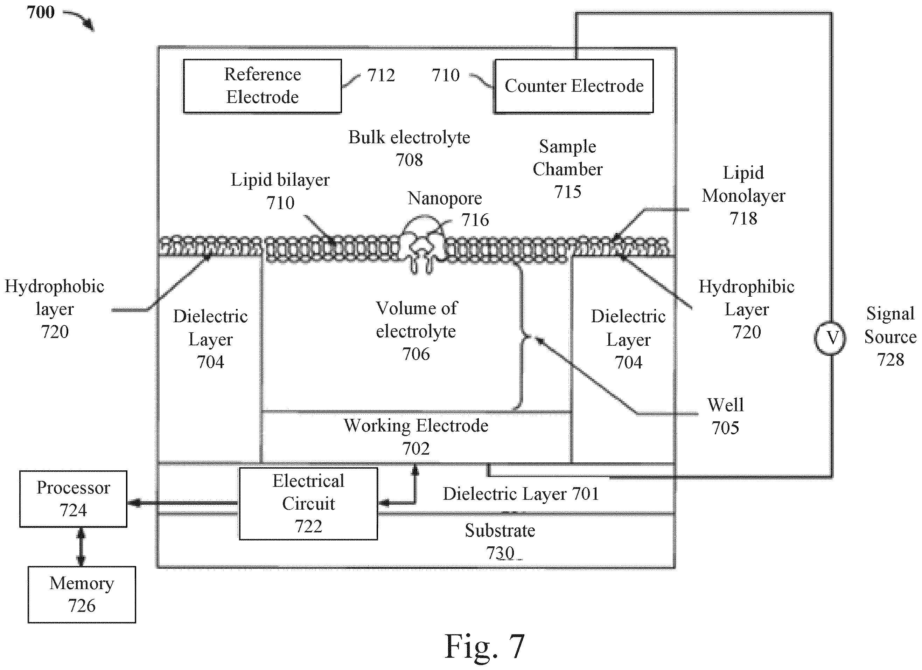

FIG. 7 illustrates an embodiment of an example nanopore cell 800 in a nanopore sensor chip, such as nanopore cell 750 in nanopore sensor chip 700 of FIG. 7, that can be used to characterize a polynucleotide. Nanopore cell 700 can include a well 705 formed of dielectric layers 701 and 704; a membrane, such as a lipid bilayer 714 formed over well 705; and a sample chamber 715 on lipid bilayer 714 and separated from well 705 by lipid bilayer 714. Well 705 can contain a volume of electrolyte 706, and sample chamber 715 can hold bulk electrolyte 708 containing a nanopore, e.g., a soluble protein nanopore transmembrane molecular complexes (PNTMC), and the analyte of interest (e.g., a nucleic acid molecule to be sequenced).

Nanopore cell 700 can include a working electrode 702 at the bottom of well 705 and a counter electrode 710 disposed in sample chamber 715. A signal source 728 can apply a voltage signal between working electrode 702 and counter electrode 710. A single nanopore (e.g., a PNTMC) can be inserted into lipid bilayer 714 by an electroporation process caused by the voltage signal, thereby forming a nanopore 716 in lipid bilayer 714. The individual membranes (e.g., lipid bilayers 714 or other membrane structures) in the array can be neither chemically nor electrically connected to each other. Thus, each nanopore cell in the array can be an independent sequencing machine, producing data unique to the single polymer molecule associated with the nanopore that operates on the analyte of interest and modulates the ionic current through the otherwise impermeable lipid bilayer.

As shown in FIG. 2, nanopore cell 700 can be formed on a substrate 730, such as a silicon substrate. Dielectric layer 701 can be formed on substrate 730. Dielectric material used to form dielectric layer 701 can include, for example, glass, oxides, nitrides, and the like. An electric circuit 722 for controlling electrical stimulation and for processing the signal detected from nanopore cell 700 can be formed on substrate 730 and/or within dielectric layer 701. For example, a plurality of patterned metal layers (e.g., metal 1 to metal 6) can be formed in dielectric layer 701, and a plurality of active devices (e.g., transistors) can be fabricated on substrate 730. In some embodiments, signal source 728 is included as a part of electric circuit 722. Electric circuit 722 can include, for example, amplifiers, integrators, analog-to-digital converters, noise filters, feedback control logic, and/or various other components. Electric circuit 722 can be further coupled to a processor 724 that is coupled to a memory 726, where processor 724 can analyze the sequencing data to determine sequences of the polymer molecules that have been sequenced in the array.

Working electrode 702 can be formed on dielectric layer 701, and can form at least a part of the bottom of well 705. In some embodiments, working electrode 702 is a metal electrode. For non-faradaic conduction, working electrode 702 can be made of metals or other materials that are resistant to corrosion and oxidation, such as, for example, platinum, gold, titanium nitride, and graphite. For example, working electrode 702 can be a platinum electrode with electroplated platinum. In another example, working electrode 702 can be a titanium nitride (TiN) working electrode. Working electrode 702 can be porous, thereby increasing its surface area and a resulting capacitance associated with working electrode 702. Because the working electrode of a nanopore cell can be independent from the working electrode of another nanopore cell, the working electrode can be referred to as cell electrode in this disclosure.

Dielectric layer 704 can be formed above dielectric layer 701. Dielectric layer 704 forms the walls surrounding well 705. Dielectric material used to form dielectric layer 704 can include, for example, glass, oxide, silicon mononitride (SiN), polyimide, or other suitable hydrophobic insulating material. The top surface of dielectric layer 704 can be silanized. The silanization can form a hydrophobic layer 720 above the top surface of dielectric layer 704. In some embodiments, hydrophobic layer 720 has a thickness of about 1.5 nanometer (nm).

Well 705 formed by the dielectric layer walls 704 includes volume of electrolyte 706 above working electrode 702. Volume of electrolyte 706 can be buffered and can include one or more of the following: lithium chloride (LiCl), sodium chloride (NaCl), potassium chloride (KCl), lithium glutamate, sodium glutamate, potassium glutamate, lithium acetate, sodium acetate, potassium acetate, calcium chloride (CaCl₂), strontium chloride (SrCl₂), manganese chloride (MnCl₂), and magnesium chloride (MgCl₂). In some embodiments, volume of electrolyte 706 has a thickness of about three microns (µm).

As also shown in FIG. 7, a membrane can be formed on top of dielectric layer 704 and spanning across well 705. In some embodiments, the membrane includes a lipid monolayer 718 formed on top of hydrophobic layer 720. As the membrane reaches the opening of well 705, lipid monolayer 708 can transition to lipid bilayer 714 that spans across the opening of well 705. The lipid bilayer can comprise or consist of phospholipid, for example, selected from diphytanoylphosphatidylcholine (DPhPC), 1,2-diphytanoyl-sn-glycero-3-phosphocholine, 1,2-di-O-phytanyl-sn-glycero-3-phosphocholine (DOPhPC), palmitoyl-oleoyl-phosphatidylcholine (POPC), dioleoyl-phosphatidyl-methylester (DOPME), dipalmitoylphosphatidylcholine (DPPC), phosphatidylcholine, phosphatidylethanolamine, phosphatidylserine, phosphatidic acid, phosphatidylinositol, phosphatidylglycerol, sphingomyelin, 1,2-di-O-phytanyl-sn-glycerol, 1,2-dipalmitoyl-sn-glycero-3-phosphoethanolamine-N-[methoxy(polyethylene glycol)-350], 1,2-dipalmitoyl-sn-glycero-3-phosphoethanolamine-N-[methoxy(polyethylene glycol)-550], 1,2-dipalmitoyl-sn-glycero-3-phosphoethanolamine-N-[methoxy(polyethylene glycol)-750], 1,2-dipalmitoyl-sn-glycero-3-phosphoethanolamine-N-[methoxy(polyethylene glycol)-1000], 1,2-dipalmitoyl-sn-glycero-3-phosphoethanolamine-N-[methoxy(polyethylene glycol)-7000], 1,2-dioleoyl-sn-glycero-3-phosphoethanolamine-N-lactosyl, GM1 Ganglioside, Lysophosphatidylcholine (LPC), or any combination thereof.

As shown, lipid bilayer 714 is embedded with a single nanopore 716, e.g., formed by a single PNTMC. As described above, nanopore 716 can be formed by inserting a single PNTMC into lipid bilayer 714 by electroporation. Nanopore 716 can be large enough for passing at least a portion of the analyte of interest and/or small ions (e.g., Na⁺, K⁺, Ca²⁺, CT) between the two sides of lipid bilayer 714.

Sample chamber 715 is over lipid bilayer 714, and can hold a solution of the analyte of interest for characterization. The solution can be an aqueous solution containing bulk electrolyte 708 and buffered to an optimum ion concentration and maintained at an optimum pH to keep the nanopore 716 open. Nanopore 716 crosses lipid bilayer 714 and provides the only path for ionic flow from bulk electrolyte 708 to working electrode 702. In addition to nanopores (e.g., PNTMCs) and the analyte of interest, bulk electrolyte 708 can further include one or more of the following: lithium chloride (LiCl), sodium chloride (NaCl), potassium chloride (KCl), lithium glutamate, sodium glutamate, potassium glutamate, lithium acetate, sodium acetate, potassium acetate, calcium chloride (CaCl₂), strontium chloride (SrCl₂), manganese chloride (MnCl₂), and magnesium chloride (MgCl₂).

Counter electrode (CE) 710 can be an electrochemical potential sensor. In some embodiments, counter electrode 710 is shared between a plurality of nanopore cells, and can therefore be referred to as a common electrode. In some cases, the common potential and the common electrode can be common to all nanopore cells, or at least all nanopore cells within a particular grouping. The common electrode can be configured to apply a common potential to the bulk electrolyte 708 in contact with the nanopore 716. Counter electrode 710 and working electrode 702 can be coupled to signal source 728 for providing electrical stimulus (e.g., voltage bias) across lipid bilayer 714, and can be used for sensing electrical characteristics of lipid bilayer 714 (e.g., resistance, capacitance, voltage decay, and ionic current flow). In some embodiments, nanopore cell 700 can also include a reference electrode 712.

Although these embodiments refer to lipid bilayers, it is also appreciated that the present systems may use any semi-permeable membrane that permits the transmembrane flow of water but has limited to no permeability to the flow of ions or other osmolytes. For example, the disclosed methods and systems can be used with membranes that are polymeric. In some embodiments, the membrane is a copolymer. In some embodiments, the membrane is a triblock copolymer. In an exemplary embodiment, the membrane is an A-B-A triblock copolymer wherein "A" is poly-b-(methyloxazoline) and "B" is poly(dimethylsiloxane)-poly-b-(methyloxazoline) (Pmoxa-PDMS-Pmoxa membrane).

In some embodiments, various checks are made during creation of the nanopore cell as part of calibration. Once a nanopore cell is created, further calibration steps can be performed, e.g., to identify nanopore cells that are performing as desired (e.g., one nanopore in the cell). Such calibration checks can include physical checks, voltage calibration, open channel calibration, and identification of cells with a single nanopore.

### Detection signals of nanopore sequencing cell

Nanopore cells in nanopore sensor chip, such as nanopore cells 750 in nanopore sensor chip 700, can enable parallel sequencing using a single molecule nanopore based sequencing by synthesis (Nano-SBS) technique.

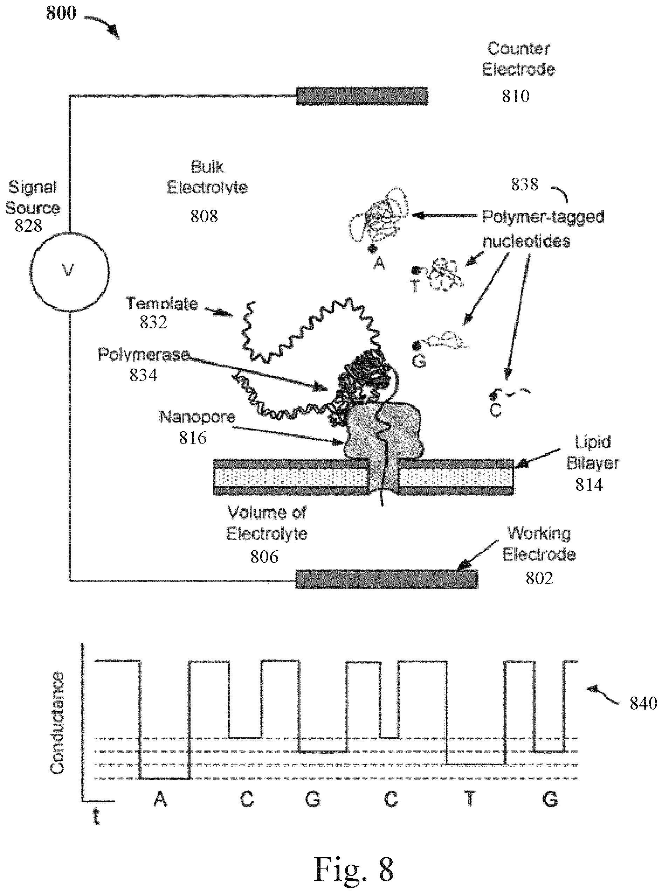

FIG. 8 illustrates an embodiment of a nanopore cell 800 performing nucleotide sequencing using the Nano-SBS technique. In the Nano-SBS technique, a template 832 to be sequenced (e.g., a nucleotide acid molecule or another analyte of interest) and a primer can be introduced into bulk electrolyte 808 in the sample chamber of nanopore cell 800. As examples, template 832 can be circular or linear. A nucleic acid primer can be hybridized to a portion of template 832 to which four differently polymer-tagged nucleotides 838 can be added.

In some embodiments, an enzyme (e.g., a polymerase 834, such as a DNA polymerase) is associated with nanopore 816 for use in the synthesizing a complementary strand to template 832. For example, polymerase 834 can be covalently attached to nanopore 816. Polymerase 834 can catalyze the incorporation of nucleotides 838 onto the primer using a single stranded nucleic acid molecule as the template. Nucleotides 838 can comprise tag species ("tags") with the nucleotide being one of four different types: A, T, G, or C for deoxyribonucleotides, or A, U, G, or C for ribonucleotides. When a tagged nucleotide is correctly complexed with polymerase 834, the tag can be pulled (e.g., loaded) into the nanopore by an electrical force, such as a force generated in the presence of an electric field generated by a voltage applied across lipid bilayer 814 and/or nanopore 816. The tail of the tag can be positioned in the barrel of nanopore 816. The tag held in the barrel of nanopore 816 can generate a unique ionic blockade signal 840 due to the tag's distinct chemical structure and/or size, thereby electronically identifying the added base to which the tag attaches.

As used herein, a "loaded" or "threaded" tag is one that is positioned in and/or remains in or near the nanopore for an appreciable amount of time, e.g., 0.1 millisecond (ms) to 10000 ms. In some cases, a tag is loaded in the nanopore prior to being released from the nucleotide. In some instances, the probability of a loaded tag passing through (and/or being detected by) the nanopore after being released upon a nucleotide incorporation event is suitably high, e.g., 80% to 99%.

In some embodiments, before polymerase 834 is connected to nanopore 816, the conductance of nanopore 816 is high, such as, for example, about 300 picosiemens (300 pS). As the tag is loaded in the nanopore, a unique conductance signal (e.g., signal 840) is generated due to the tag's distinct chemical structure and/or size. For example, the conductance of the nanopore can be about 60 pS, 80 pS, 100 pS, or 120 pS, each corresponding to one of the four types of tagged nucleotides. The polymerase can then undergo an isomerization and a transphosphorylation reaction to incorporate the nucleotide into the growing nucleic acid molecule and release the tag molecule.

In some cases, some of the tagged nucleotides may not match (complementary bases) with a current position of the nucleic acid molecule (template). The tagged nucleotides that are not base-paired with the nucleic acid molecule can also pass through the nanopore. These non-paired nucleotides can be rejected by the polymerase within a time scale that is shorter than the time scale for which correctly paired nucleotides remain associated with the polymerase. Tags bound to non-paired nucleotides can pass through the nanopore quickly, and be detected for a short period of time (e.g., less than 10 ms), while tags bounded to paired nucleotides can be loaded into the nanopore and detected for a long period of time (e.g., at least 10 ms). Therefore, non-paired nucleotides can be identified by a downstream processor based at least in part on the time for which the nucleotide is detected in the nanopore.

A conductance (or equivalently the resistance) of the nanopore including the loaded (threaded) tag can be measured via a signal value (e.g., voltage or a current passing through the nanopore), thereby providing an identification of the tag species and thus the nucleotide at the current position. In some embodiments, a direct current (DC) signal is applied to the nanopore cell (e.g., so that the direction in which the tag moves through the nanopore is not reversed). However, operating a nanopore sensor for long periods of time using a direct current can change the composition of the electrode, unbalance the ion concentrations across the nanopore, and have other undesirable effects that can affect the lifetime of the nanopore cell. Applying an alternating current (AC) waveform can reduce the electro-migration to avoid these undesirable effects and have certain advantages as described below. The nucleic acid sequencing methods described herein that utilize tagged nucleotides are fully compatible with applied AC voltages, and therefore an AC waveform can be used to achieve these advantages.

The ability to re-charge the electrode during the AC detection cycle can be advantageous when sacrificial electrodes, electrodes that change molecular character in the current-carrying reactions (e.g., electrodes comprising silver), or electrodes that change molecular character in current-carrying reactions are used. An electrode can deplete during a detection cycle when a direct current signal is used. The recharging can prevent the electrode from reaching a depletion limit, such as becoming fully depleted, which can be a problem when the electrodes are small (e.g., when the electrodes are small enough to provide an array of electrodes having at least 500 electrodes per square millimeter). Electrode lifetime in some cases scales with, and is at least partly dependent on, the width of the electrode.

Suitable conditions for measuring ionic currents passing through the nanopores are known in the art and examples are provided herein. The measurement can be carried out with a voltage applied across the membrane and pore. In some embodiments, the voltage used ranges from -400 mV to +400 mV. The voltage used is preferably in a range having a lower limit selected from -400 mV, -300 mV, -200 mV, -150 mV, -100 mV, -50 mV, -20 mV, and 0 mV, and an upper limit independently selected from +10 mV, +20 mV, +50 mV, +100 mV, +150 mV, +200 mV, +300 mV, and +400 mV. The voltage used can be more preferably in the range from 100 mV to 240 mV and most preferably in the range from 160 mV to 240 mV. It is possible to increase discrimination between different nucleotides by a nanopore using an increased applied potential. Sequencing nucleic acids using AC waveforms and tagged nucleotides is described in US Patent Publication No. US 2014/0134616 entitled "Nucleic Acid Sequencing Using Tags," filed on November 6, 2013.

In addition to the tagged nucleotides described in US 2014/0134616, sequencing can be performed using nucleotide analogs that lack a sugar or acyclic moiety, e.g., (S)-glycerol nucleoside triphosphates (gNTPs) of the five common nucleobases: adenine, cytosine, guanine, uracil, and thymine (Horhota et al., Organic Letters, 8:5345-5347 [2006]).

### Electric circuit of nanopore sequencing cell

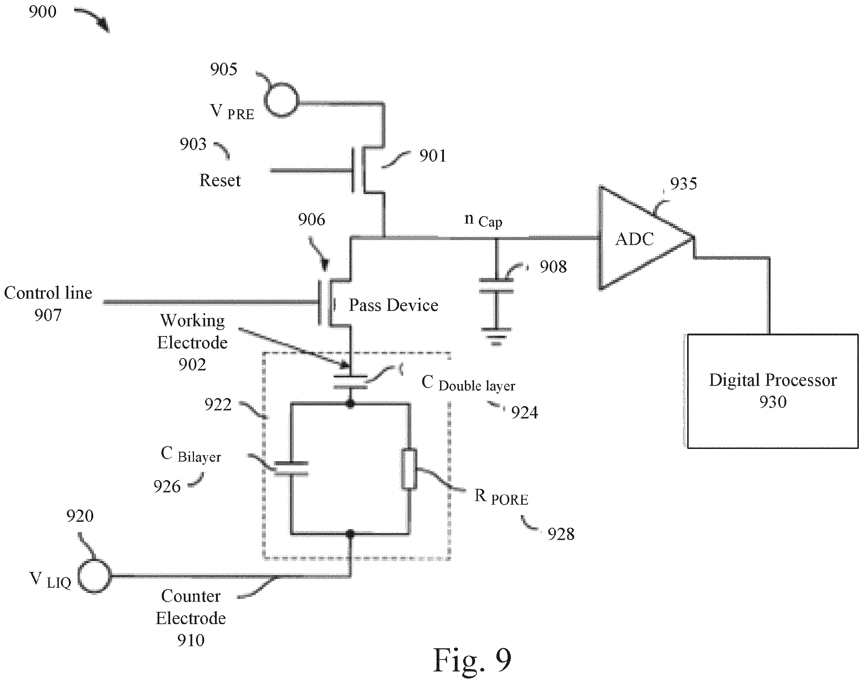

FIG. 9 illustrates an embodiment of an electric circuit 900 (which may include portions of electric circuit 722 in FIG. 7) in a nanopore cell, such as nanopore cell 800. As described above, in some embodiments, electric circuit 900 includes a counter electrode 910 that can be shared between a plurality of nanopore cells or all nanopore cells in a nanopore sensor chip, and can therefore also be referred to as a common electrode. The common electrode can be configured to apply a common potential to the bulk electrolyte (e.g., bulk electrolyte 808) in contact with the lipid bilayer (e.g., lipid bilayer 814) in the nanopore cells by connecting to a voltage source V_{LIQ} 920. In some embodiments, an AC non-Faradaic mode is utilized to modulate voltage V_{LIQ} with an AC signal (e.g., a square wave) and apply it to the bulk electrolyte in contact with the lipid bilayer in the nanopore cell. In some embodiments, V_{LIQ} is a square wave with a magnitude of ±200-250 mV and a frequency between, for example, 25 and 400 Hz. The bulk electrolyte between counter electrode 910 and the lipid bilayer (e.g., lipid bilayer 814) can be modeled by a large capacitor (not shown), such as, for example, 100 µF or larger.

FIG. 9 also shows an electrical model 922 representing the electrical properties of a working electrode 902 (e.g., working electrode 802) and the lipid bilayer (e.g., lipid bilayer 814). Electrical model 922 includes a capacitor 926 (C_{Bilayer}) that models a capacitance associated with the lipid bilayer and a resistor 928 (R_{PORE}) that models a variable resistance associated with the nanopore, which can change based on the presence of a particular tag in the nanopore. Electrical model 922 also includes a capacitor 924 having a double layer capacitance (C _{Double Layer}) and representing the electrical properties of working electrode 902 and well 705. Working electrode 902 can be configured to apply a distinct potential independent from the working electrodes in other nanopore cells.

Pass device 906 is a switch that can be used to connect or disconnect the lipid bilayer and the working electrode from electric circuit 900. Pass device 906 can be controlled by control line 907 to enable or disable a voltage stimulus to be applied across the lipid bilayer in the nanopore cell. Before lipids are deposited to form the lipid bilayer, the impedance between the two electrodes may be very low because the well of the nanopore cell is not sealed, and therefore pass device 906 can be kept open to avoid a short-circuit condition. Pass device 906 can be closed after lipid solvent has been deposited to the nanopore cell to seal the well of the nanopore cell.

Circuitry 900 can further include an on-chip integrating capacitor 908 (n_{cap}). Integrating capacitor 908 can be pre-charged by using a reset signal 903 to close switch 901, such that integrating capacitor 908 is connected to a voltage source V_{PRE} 905. In some embodiments, voltage source V_{PRE} 905 provides a constant reference voltage with a magnitude of, for example, 900 mV. When switch 901 is closed, integrating capacitor 908 can be pre-charged to the reference voltage level of voltage source V_{PRE} 905.

After integrating capacitor 908 is pre-charged, reset signal 903 can be used to open switch 901 such that integrating capacitor 908 is disconnected from voltage source V_{PRE} 905. At this point, depending on the level of voltage source V_{LIQ}, the potential of counter electrode 910 can be at a higher level than that of the potential of working electrode 902 (and integrating capacitor 908), or vice versa. For example, during a positive phase of a square wave from voltage source V_{LIQ} (e.g., the bright or dark period of the AC voltage source signal cycle), the potential of counter electrode 910 is at a level higher than the potential of working electrode 902. During a negative phase of the square wave from voltage source V_{LIQ} (e.g., the dark or bright period of the AC voltage source signal cycle), the potential of counter electrode 910 is at a lower level than that of the potential of working electrode 902. Thus, in some embodiments, integrating capacitor 908 can be further charged during the bright period from the pre-charged voltage level of voltage source V_{PRE} 905 to a higher level, and discharged during the dark period to a lower level, due to the potential difference between counter electrode 910 and working electrode 902. In other embodiments, the charging and discharging occur in dark periods and bright periods, respectively.

Integrating capacitor 908 can be charged or discharged for a fixed period of time, depending on the sampling rate of an analog-to-digital converter (ADC) 935, which can be higher than 1 kHz, 5 kHz, 10 kHz, 100 kHz, or more. For example, with a sampling rate of 1 kHz, integrating capacitor 908 can be charged/discharged for a period of about 1 ms, and then the voltage level can be sampled and converted by ADC 935 at the end of the integration period. A particular voltage level would correspond to a particular tag species in the nanopore, and thus correspond to the nucleotide at a current position on the template.

After being sampled by ADC 935, integrating capacitor 908 can be pre-charged again by using reset signal 903 to close switch 901, such that integrating capacitor 908 is connected to voltage source V_{PRE} 905 again. The steps of precharging integrating capacitor 908, waiting for a fixed period of time for integrating capacitor 908 to charge or discharge, and sampling and converting the voltage level of integrating capacitor by ADC 935 can be repeated in cycles throughout the sequencing process.

A digital processor 930 can process the ADC output data, e.g., for normalization, data buffering, data filtering, data compression, data reduction, event extraction, or assembling ADC output data from the array of nanopore cells into various data frames. In some embodiments, digital processor 930 performs further downstream processing, such as base determination. Digital processor 930 can be implemented as hardware (e.g., in a graphics processing unit (GPU), FPGA, ASIC, etc.) or as a combination of hardware and software.

Accordingly, the voltage signal applied across the nanopore can be used to detect particular states of the nanopore. One of the possible states of the nanopore is an open-channel state when a tag-attached polyphosphate is absent from the barrel of the nanopore, also referred to herein as the unthreaded state of the nanopore. Another four possible states of the nanopore each correspond to a state when one of the four different types of tag-attached polyphosphate nucleotides (A, T, G, or C for deoxyribonucleotides, or A, U, G, or C for ribonucleotides) is held in the barrel of the nanopore. Yet another possible state of the nanopore is when the lipid bilayer is ruptured.

When the voltage level on integrating capacitor 908 is measured after a fixed period of time, the different states of a nanopore can result in measurements of different voltage levels. This is because the rate of the voltage decay (decrease by discharging or increase by charging) on integrating capacitor 908 (i.e., the steepness of the slope of a voltage on integrating capacitor 908 versus time plot) depends on the nanopore resistance (e.g., the resistance of resistor R_{PORE} 928). More particularly, as the resistance associated with the nanopore in different states is different due to the molecules' (tags') distinct chemical structures, different corresponding rates of voltage decay can be observed and can be used to identify the different states of the nanopore. The voltage decay curve can be an exponential curve with an RC time constant τ = RC, where R is the resistance associated with the nanopore (i.e., R_{PORE} resistor 928) and C is the capacitance associated with the membrane (i.e., C_{Bilayer} capacitor 926) in parallel with R. A time constant of the nanopore cell can be, for example, about 200-500 ms. The decay curve may not fit exactly to an exponential curve due to the detailed implementation of the bilayer, but the decay curve can be similar to an exponential curve and be monotonic, thus allowing detection of tags.

In some embodiments, the resistance associated with the nanopore in an open-channel state is in the range of 100 MOhm to 20 GOhm. In some embodiments, the resistance associated with the nanopore in a state where a tag is inside the barrel of the nanopore can be within the range of 200 MOhm to 100 GOhm. In other embodiments, integrating capacitor 908 is omitted, as the voltage leading to ADC 935 will still vary due to the voltage decay in electrical model 922.

The rate of the decay of the voltage on integrating capacitor 908 can be determined in different ways. As explained above, the rate of the voltage decay can be determined by measuring a voltage decay during a fixed time interval. For example, the voltage on integrating capacitor 908 can be first measured by ADC 935 at time 11, and then the voltage is measured again by ADC 935 at time t2. The voltage difference is greater when the slope of the voltage on integrating capacitor 908 versus time curve is steeper, and the voltage difference is smaller when the slope of the voltage curve is less steep. Thus, the voltage difference can be used as a metric for determining the rate of the decay of the voltage on integrating capacitor 908, and thus the state of the nanopore cell.

In other embodiments, the rate of the voltage decay is determined by measuring a time duration that is required for a selected amount of voltage decay. For example, the time required for the voltage to drop or increase from a first voltage level V1 to a second voltage level V2 can be measured. The time required is less when the slope of the voltage vs. time curve is steeper, and the time required is greater when the slope of the voltage vs. time curve is less steep. Thus, the measured time required can be used as a metric for determining the rate of the decay of the voltage on integrating capacitor n_{cap} 908, and thus the state of the nanopore cell. One skilled in the art will appreciate the various circuits that can be used to measure the resistance of the nanopore, e.g., including signal value measurement techniques, such as voltage or current measurements.

In some embodiments, electric circuit 900 does not include a pass device (e.g., pass device 906) and an extra capacitor (e.g., integrating capacitor 908 (n_{cap})) that are fabricated on-chip, thereby facilitating the reduction in size of the nanopore based sequencing chip. Due to the thin nature of the membrane (lipid bilayer), the capacitance associated with the membrane (e.g., capacitor 926 (C_{Bilayer})) alone can suffice to create the required RC time constant without the need for additional on-chip capacitance. Therefore, capacitor 926 can be used as the integrating capacitor, and can be pre-charged by the voltage signal V_{PRE} and subsequently be discharged or charged by the voltage signal V_{LIQ}. The elimination of the extra capacitor and the pass device that are otherwise fabricated on-chip in the electric circuit can significantly reduce the footprint of a single nanopore cell in the nanopore sequencing chip, thereby facilitating the scaling of the nanopore sequencing chip to include more and more cells (e.g., having millions of cells in a nanopore sequencing chip).

### B. Data sampling in nanopore cell

To perform sequencing of a nucleic acid, the voltage level of integrating capacitor (e.g., integrating capacitor 908 (n_{cap}) or capacitor 926 (C_{Bilayer})) can be sampled and converted by the ADC (e.g., ADC 935) while a tagged nucleotide is being added to the nucleic acid. The tag of the nucleotide can be pushed into the barrel of the nanopore by the electric field across the nanopore that is applied through the counter electrode and the working electrode, for example, when the applied voltage is such that V_{LIQ} is lower than V_{PRE}.

### 1. Threading

A threading event is when a tagged nucleotide is attached to the template (e.g., nucleic acid fragment), and the tag moves in and out of the barrel of the nanopore. This movement can happen multiple times during a threading event. When the tag is in the barrel of the nanopore, the resistance of the nanopore can be higher, and a lower current can flow through the nanopore.

During sequencing, a tag may not be in the nanopore in some AC cycles (referred to as an open-channel state), where the current is the highest because of the lower resistance of the nanopore. When a tag is attracted into the barrel of the nanopore, the nanopore is in a bright mode. When the tag is pushed out of the barrel of the nanopore, the nanopore is in a dark mode.

### 2. Bright and dark period

During an AC cycle, the voltage on integrating capacitor can be sampled multiple times by the ADC. For example, in one embodiment, an AC voltage signal is applied across the system at, e.g., about 100 Hz, and an acquisition rate of the ADC can be about 2000 Hz per cell or higher (including for example about 4000 Hz per cell). Thus, there can be at least about 20 data points (voltage measurements) captured per AC cycle (cycle of an AC waveform). Data points corresponding to one cycle of the AC waveform can be referred to as a set. In a set of data points for an AC cycle, there can be a subset captured when, for example, V_{LIQ} is lower than V_{PRE}, which can correspond to a bright mode (period) when the tag is forced into the barrel of the nanopore. Another subset can correspond to a dark mode (period) when the tag is pushed out of the barrel of the nanopore by the applied electric field when, for example, V_{LIQ} is higher than V_{PRE}.

### 3. Measured voltages

For each data point, when the switch 901 is opened, the voltage at the integrating capacitor (e.g., integrating capacitor 908 (n_{cap}) or capacitor 926 (C_{Bilayer})) will change in a decaying manner as a result of the charging/discharging by V_{LIQ}, e.g., as an increase from V_{PRE} to V_{LIQ} when V_{LIQ} is higher than V_{PRE} or a decrease from V_{PRE} to V_{LIQ} when V_{LIQ} is lower than V_{PRE}. The final voltage values can deviate from V_{LIQ} as the working electrode charges. The rate of change of the voltage level on the integrating capacitor can be governed by the value of the resistance of the bilayer, which can include the nanopore, which can in turn include a molecule (e.g., a tag of a tagged nucleotides) in the nanopore. The voltage level can be measured at a predetermined time after switch 901 opens.

Switch 901 can operate at the rate of data acquisition. Switch 901 can be closed for a relatively short time period between two acquisitions of data, typically right after a measurement by the ADC. The switch allows multiple data points to be collected during each sub-period (bright or dark) of each AC cycle of V_{LIQ}. If switch 901 remains open, the voltage level on the integrating capacitor, and thus the output value of the ADC, fully decays and stays there. If instead switch 901 is closed, the integrating capacitor is precharged again (to V_{PRE}) and becomes ready for another measurement. Thus, switch 901 allows multiple data points to be collected for each sub-period (bright or dark) of each AC cycle. Such multiple measurements can allow higher resolution with a fixed ADC (e.g. 8-bit to 14-bit due to the greater number of measurements, which may be averaged). The multiple measurements can also provide kinetic information about the molecule threaded into the nanopore. The timing information can allow the determination of how long a threading takes place. This can also be used in helping to determine whether multiple nucleotides that are added to the nucleic acid strand are being sequenced.

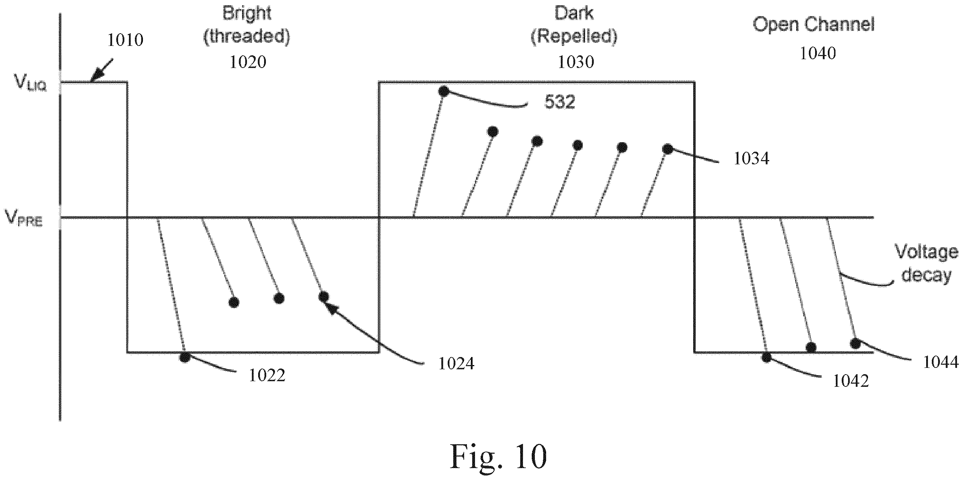

FIG. 10 shows example data points captured from a nanopore cell during bright periods and dark periods of AC cycles. In FIG. 10, the change in the data points is exaggerated for illustration purpose. The voltage (V_{PRE}) applied to the working electrode or the integrating capacitor is at a constant level, such as, for example, 900 mV. A voltage signal 1010 (V_{LIQ}) applied to the counter electrode of the nanopore cells is an AC signal shown as a rectangular wave, where the duty cycle can be any suitable value, such as less than or equal to 50%, for example, about 40%.

During a bright period 1020, voltage signal 1010 (V_{LIQ}) applied to the counter electrode is lower than the voltage V_{PRE} applied to the working electrode, such that a tag can be forced into the barrel of the nanopore by the electric field caused by the different voltage levels applied at the working electrode and the counter electrode (e.g., due to the charge on the tag and/or flow of the ions). When switch 1001 is opened, the voltage at a node before the ADC (e.g., at an integrating capacitor) will decrease. After a voltage data point is captured (e.g., after a specified time period), switch 1001 can be closed and the voltage at the measurement node will increase back to V_{PRE} again. The process can repeat to measure multiple voltage data points. In this way, multiple data points can be captured during the bright period.

As shown in FIG. 10, a first data point 1022 (also referred to as first point delta (FPD)) in the bright period after a change in the sign of the V_{LIQ} signal can be lower than subsequent data points 1024. This can be because there is no tag in the nanopore (open channel), and thus it has a low resistance and a high discharge rate. In some instances, first data point 1022 can exceed the V_{LIQ} level as shown in FIG. 10. This can be caused by the capacitance of the bilayer coupling the signal to the on-chip capacitor. Data points 1024 can be captured after a threading event has occurred, i.e., a tag is forced into the barrel of the nanopore, where the resistance of the nanopore and thus the rate of discharging of the integrating capacitor depends on the particular type of tag that is forced into the barrel of the nanopore. Data points 1024 can decrease slightly for each measurement due to charge built up at C_{Double Layer} 1024, as mentioned below.

During a dark period 1030, voltage signal 1010 (V_{LIQ}) applied to the counter electrode is higher than the voltage (V_{PRE}) applied to the working electrode, such that any tag would be pushed out of the barrel of the nanopore. When switch 1001 is opened, the voltage at the measurement node increases because the voltage level of voltage signal 1010 (V_{LIQ}) is higher than V_{PRE}. After a voltage data point is captured (e.g., after a specified time period), switch 1001 can be closed and the voltage at the measurement node will decrease back to V_{PRE} again. The process can repeat to measure multiple voltage data points. Thus, multiple data points can be captured during the dark period, including a first point delta 1032 and subsequent data points 1034. As described above, during the dark period, any nucleotide tag is pushed out of the nanopore, and thus minimal information about any nucleotide tag is obtained, besides for use in normalization.

FIG. 10 also shows that during bright period 1040, even though voltage signal 1010 (V_{LIQ}) applied to the counter electrode is lower than the voltage (V_{PRE}) applied to the working electrode, no threading event occurs (open-channel). Thus, the resistance of the nanopore is low, and the rate of discharging of the integrating capacitor is high. As a result, the captured data points, including a first data point 1042 and subsequent data points 1044, show low voltage levels.

The voltage measured during a bright or dark period might be expected to be about the same for each measurement of a constant resistance of the nanopore (e.g., made during a bright mode of a given AC cycle while one tag is in the nanopore), but this may not be the case when charge builds up at double layer capacitor 924 (C_{Double Layer}). This charge build-up can cause the time constant of the nanopore cell to become longer. As a result, the voltage level may be shifted, thereby causing the measured value to decrease for each data point in a cycle. Thus, within a cycle, the data points may change somewhat from data point to another data point, as shown in FIG. 10.

Further details regarding measurements can be found in, for example, U.S. Patent Publication No. 2016/0178577 entitled "Nanopore-Based Sequencing With Varying Voltage Stimulus," U.S. Patent Publication No. 2016/0178554 entitled "Nanopore-Based Sequencing With Varying Voltage Stimulus," U.S. Patent Application No. 15/085,700 entitled "Non-Destructive Bilayer Monitoring Using Measurement Of Bilayer Response To Electrical Stimulus," and U.S. Patent Application No. 15/085,713 entitled "Electrical Enhancement Of Bilayer Formation,"

### 4. Normalization and Base calling

For each usable nanopore cell of the nanopore sensor chip, a production mode can be run to sequence nucleic acids. The ADC output data captured during the sequencing can be normalized to provide greater accuracy. Normalization can account for offset effects, such as cycle shape, gain drift, charge injection offset, and baseline shift. In some implementations, the signal values of a bright period cycle corresponding to a threading event can be flattened so that a single signal value is obtained for the cycle (e.g., an average) or adjustments can be made to the measured signal to reduce the intra-cycle decay (a type of cycle shape effect). Gain drift generally scales entire signal and changes on the order to 100s to 1,000s of seconds. As examples, gain drift can be triggered by changes in solution (pore resistance) or changes in bilayer capacitance. The baseline shift occurs with a timescale of ~100 ms, and relates to a voltage offset at the working electrode. The baseline shift can be driven by changes in an effective rectification ratio from threading as a result of a need to maintain charge balance in the sequencing cell from the bright period to the dark period.

After normalization, embodiments can determine clusters of voltages for the threaded channels, where each cluster corresponds to a different tag species, and thus a different nucleotide. The clusters can be used to determine probabilities of a given voltage corresponding to a given nucleotide. As another example, the clusters can be used to determine cutoff voltages for discriminating between different nucleotides (bases).

### VI. Examples

The present invention is described in further detail in the following examples, which are intended to illustrate, but not to limit the claimed invention.

### VIA. Example 1: Generation of α-HL with negatively charged amino acid substitutions

### VIA1. Variant monomer generation

DNA encoding a wild-type α-HL having the amino acid sequence of SEQ ID NO: 1 was purchased from a commercial source. Sequence modifications were performed by site-directed mutagenesis using a QuikChange Multi Site-Directed Mutagenesis kit (Stratagene, La Jolla, CA). All modified polynucleotides encode an α-HL variant having at least one non-native negatively charged amino acid and a cleavable epitope tag (such as a C-terminal linker/TEV/His tag). Other modifications include modifications that control oligomerization (e.g. H35G/L and, in some cases, H144A substitutions), widen the constriction site (e.g. E111A, K147A, and/or M113D), improve pore CV (D227N), Table 6 lists exemplary α-HL monomers encoded by the modified polynucleotides:

**Table 6**

| **ID** | **Modifications relative to SEQ ID NO: 1** | **Non-Native Negative AA** | **Other Substitutions / Purpose** | **SEQ ID NO** |
|---|---|---|---|---|
| G617 | A1D / H35G / H144A | Ent.: A1D | CO: H35G, H144A | 31 |
| G619 | K8D / H35G / H144A | Ent.: K8D | CO: H35G, H144A | 32 |
| G623 | H35L / K46D | Ent. K46D | CO: H35L | 33 |
| G697 | H35G / N47D / H144A / V149D | Ent.: N47D, V149D | CO: H35G, H144A | 34 |
| G724 | A1D / K8D / H35G / N47D / H144A / V149D | Ent. A1D, K8D, N47D, V149D | CO: H35G, H144A | 35 |
| G1143 | K8D/D13N/H35G/ H144A | Ent. K8D | CO: H35G, H144A | 36 |
| | | | EX: D13N | |
| G1147 | K8D/D13N/H35G/ H144A / V149D | Ent. K8D, V149D | EX: D13N | 37 |
| | | | CO: H35G, H144A | |
| G1313 | 0D / H35G / H144A | Ent. 0D | CO: H35G, H144A | 38 |
| G1314 | 0D / A1D / H35G / H144A | Ent. 0D, A1D | CO: H35G, H144A | 39 |
| G1315 | 0D / H35G / N47D / H144A | Ent. 0D, N47D | CO: H35G, H144A | 40 |
| G1316 | 0D / K8D / H35G / H144A | Ent. 0D, K8D | CO: H35G, H144A | 41 |
| G1317 | 0D / A1D / K8D / H35G / H144A | Ent. 0D, A1D, K8D | CO: H35G, H144A | 42 |
| G1429 | A1D / H35G / M113D / T115D / T117D / N121D / H144A | Ent. A1D | CO: H35G, H144A | 43 |
| | | Con. M113D | | |
| | | BB: T115D, T117D, N121D | | |
| G1440 | A1D / S3D / G10D / H35G / K46D / S106D / H144A | Ent. A1D, S3D, G10D, K46D, S106D | CO: H35G, H144A | 44 |
| G1441 | G10D / H35G / M113D / T115D / T117D / N121D / H144A | Ent. G10D | CO: H35G, H144A | 45 |
| | | Con.: M113D | | |
| | | BB: 115D, T117D, N121D | | |
| G1143 | K8D / D13N / H35G / H144A | Ent.: K8D | CO: H35L, H144A | 46 |
| | | | EX: D13N | |
| G1147 | K8D / D13N / H35G/ V149D | Ent.: K8D, V149D | CO: H35L | 47 |
| | | | EX: D13N | |
| G1313 | 0D / H35G / H144A | Ent.: 0D | CO: H35G, H144A | 48 |
| G1314 | 0D / A1D / H35G / H144A | Ent.: 0D, A1D | CO: H35G, H144A | 49 |
| G1315 | 0D / H35G / N47D / H144A | Ent.: 0D, N47D | CO: H35G, H144A | 50 |
| G1316 | 0D / K8D / H35G / H144A | Ent.: 0D, K8D | CO: H35G, H144A | 51 |
| G1317 | 0D / A1D / K8D / H35G / H144A | Ent.: 0D, A1D, K8D | CO: H35G, H144A | 52 |
| G1515 | H35G / E111A / M113D / T115D / T117D / N121D / H144A / K147A | Con.: M113D | WC: E111A, K147A, M113D | 53 |
| | | BB: T115D, T117D, N121D | | |
| | | | CO: H144A, H35G | |
| G1608 | A1D / T9D / H35G / N47K / T129D / G130D / H144A | Ent.: A1D, T9D | CO: H144A, H35G | 54 |
| | | BB: T129D, G130D | EX: N47K | |
| G1609 | A1D / H35G / N47K / T129D / G130D / H144A | Ent.: A1D | CO: H144A, H35G | 55 |
| | | BB: T129D, G130D | EX: N47K | |
| G1633 | H35G / M113D / T115D / T117D / N121D | Con.: M113D | CO: H35G | 56 |
| | | BB: T115D, T117D, N121D | | |
| G1637 | A1D / H35G / N47K / T129D / G130D / K131D / H144A / K147D | Ent.: A1D | CO: H144A, H35G | 57 |
| | | Const.: K147D | EX: N47K | |
| | | BB: T129D, G130D | | |
| | | Exit: K131D | | |
| G1646 | A1D / N17D / H35G / K131D / H144A | Ent.: A1D, N17D | CO: H144A, H35G | 58 |
| | | Exit: K131D | | |
| G1687 | A1D / K131D / K147D | Ent.: A1D | None | 59 |
| | | Exit: K131D | | |
| | | K147D | | |
| G1698 | N17D / H35G / N47K / G77K / E111A / D113A / T115D / T117D / N121D / D128K / K147A /N173K | Ent.: N17D | WC: E111A, K147A | 60 |
| | | BB: T115D, T117D, N121D | CO: H35G | |
| | | | EX: N47K | |
| | | | pI: G77K, D128K, N173K | |
| G1701 | N17D / N47K / G77K / E111A / D113A / T115D / | Ent.: N17D | WC: E111A, D113A, K147A | 61 |
| | | BB: T115D, T117D, N121D | | |
| | | | CO: H35G | |
| | T117D / N121D / D128K / K147A / N173K | | EX: N47K | |
| | | | pI: G77K, D128K, N173K | |
| G1713 | A1D / H35L / K131D / K147D | Ent.: A1D | CO: H35L | 62 |
| | | Con.: K147D | | |
| | | Exit: K131D | | |
| G1748 | A1D / K8D / H35L / K131D / H144A / K147D | Ent.: A1D, K8D | CO: H35L, H144A | 63 |
| | | Con.: K147D | | |
| | | Exit: K131D | | |
| G1749 | A1D / N17D / H35L / K131D / H144A / K147D | Ent.: A1D, N17D | CO: H35L, H144A | 64 |
| | | Con.: K147D | | |
| | | Exit: K131D | | |
| G1751 | A1D / K8D / H35L / T115D / T117D / K131D / H144A / K147D | Ent.: A1D, K8D | CO: H35L, H144A | 65 |
| | | Con.: K147D | | |
| | | BB: T115D, T117D | | |
| | | Exit: K131D | | |
| G1752 | N17D / H35L / N47D / E111A / M113D / T115D / T117D / N121D / K147A / D227N | Ent.: N17D, N47D | WC: E111A, K147A | 66 |
| | | Con.: M113D | CO: H35L | |
| | | BB: T115D, T117D, N121D | CV: D227N | |
| G1753 | K8D / H35L / N47D / E111A / M113D / T115D / T117D / N121D / K147A / D227N | Ent.: K8D, N47D | WC: E111A, K147A | 67 |
| | | Con.: M113D | CO: H35L | |
| | | BB: T115D, T117D, N121D | CV: D227N | |
| G1754 | A1D / T9D / H35L / K131D / H144A / K147D | Ent.: A1D, T9D | CO: H35L, H144A | 68 |
| | | Con.: K147D | | |
| | | Exit: K131D | | |
| G1755 | A1D / K8D / H35L / N121D / K131D / H144A / K147D | Ent.: A1D, K8D | CO: H35L, H144A | 69 |
| | | Con.: K147D | | |
| | | BB: N121D | | |
| | | Exit: K131D | | |
| G1756 | A1D / K8D / H35L / N123D / K131D / H144A / K147D | Ent.: A1D, K8D | CO: H35L, H144A | 70 |
| | | Con.: K147D | | |
| | | BB: N123D | | |
| | | Exit: K131D | | |
| G1757 | A1D / N17D / H35L / N121D / K131D / H144A / K147D | Ent.: A1D, N17D | CO: H35L, H144A | 71 |
| | | Con.: K147D | | |
| | | BB: N121D | | |
| | | Exit: K131D | | |
| G1758 | A1D / N17D / H35L / N123D / K131D / H144A / K147D | Ent.: A1D, N17D | CO: H35L, H144A | 72 |
| | | Con.: K147D | | |
| | | BB: N123D | | |
| | | Exit: K131D | | |
| G1814 | A1D / N17D / H35L / N47D / E111A / M113D / T115D / T117D / N121D / K147A / D227N | Ent.: A1D, N17D, N47D | CO: H35L | 73 |
| | | Con: M113D | WC: E111A, K147A, M113D | |
| | | BB: T115D, T117D, N121D | CV: D227N | |
| G1815 | N17D / H35L / N47D / M113D / T115D / T117D / N121D / K147A / D227N | Ent.: N17D, N47D | CO: H35L | 74 |
| | | Con: M113D | WC: M113D, K147A | |
| | | BB: T115D, T117D, N121D | CV: D227N | |
| G1821 | K8D / T9D / D13N / H35G | Ent.: K8D, T9D | CO: H35G | 75 |
| | | | EX: D13N | |
| G1823 | K8D / D13N / H35G / S106D | Ent.: K8D, S106D | CO: H35G | 76 |
| | | | EX: D13N | |
| G1824 | S3D / K8D / D13N / H35G | Ent.: S3D, K8D | CO: H35G | 77 |
| | | | EX: D13N | |
| G1825 | K8D / D13N / N17D / H35G | Ent.: K8D, N17D | CO: H35G | 78 |
| | | | EX: D13N | |
| G1827 | K8D / D13N / H35G / S239D | Ent.: K8D | CO: H35G | 79 |
| | | S239D | EX: D13N | |
| G1828 | K8D / D13N / H35G / N47D | Ent.: K8D, N47D | CO: H35G | 80 |
| | | | EX: D13N | |
| G1829 | K8D / D13N / H35G / K46D | Ent.: K8D, K46D | CO: H35G | 81 |
| | | | EX: D13N | |
| G1831 | K8D / D13N / H35G / M113D | Ent.: K8D | CO: H35G | 82 |
| | | Con.: M113D | WC: M113D | |
| | | | EX: D13N | |
| G1832 | K8D / D13N / H35G / T115D | Ent.: K8D | CO: H35G | 83 |
| | | BB: T115D | EX: D13N | |
| G1833 | K8D / D13N / H35G / T117D | Ent.: K8D | CO: H35G | 84 |
| | | BB: T117D | EX: D13N | |
| G1834 | K8D / D13N / H35G / N121D | Ent.: K8D | CO: H35G | 85 |
| | | BB: N121D | EX: D13N | |
| G1835 | K8D / D13N / H35G / T129D | Ent.: K8D | CO: H35G | 86 |
| | | BB: T129D | EX: D13N | |
| G1836 | K8D / D13N / H35G / G130D | Ent.: K8D | CO: H35G | 87 |
| | | BB: G130D | EX: D13N | |
| G1837 | K8D / D13N / H35G / K131D | Ent.: K8D | CO: H35G | 88 |
| | | Exit: K131D | EX: D13N | |
| G1838 | K8D / D13N / H35G / K288D | Ent.: K8D, K288D | CO: H35G | 89 |
| | | | EX: D13N | |
| G1843 | K8D / D13N / H35G / E111A | Ent.: K8D | CO: H35G, E111A | 90 |
| | | | EX: D13N | |
| G1868 | K8D / D13N / H35G / K46D / M113D / E111A / N121D | Ent.: K8D, K46D | CO: H35G, E111A | 91 |
| | | Con.: M113D | | |
| | | BB: N121D | | |
| G1910 | A1D / N17D / H35L / K131D / G133D / K147D | Ent.: A1D, N17D | CO: H35L, H144A | 92 |
| | | Con.: K147D | | |
| | | BB: G133D | | |
| | | Exit: K131D | | |
| G1911 | A1D / N17D / H35L / K131D / L135D / K147D | Ent.: A1D, N17D | CO: H35L, H144A | 93 |
| | | Con.: K147D | | |
| | | BB: L135D | | |
| | | Exit: K131D | | |
| G1914 | K8D / N17D / H35L / N47D / M113D / T115D / T117D / N121D / K147A / D227N | Ent.: K8D, N17D, N47D | CO: H35G, E111A | 94 |
| | | Con.: M113D | WC: M113D, K147A | |
| | | BB: T115D, T117D, N121D | CV: D227N | |
| G1918 | A1D / H35L / D127G / D128K / K131D / K147D | Ent.: A1D | CO: H35L, H144A | 95 |
| | | Con.: K147D | LI: D127G, D128K | |
| | | Exit: K131D | | |
| G1933 | A1D / K8D / N17D / H35L / N121D / K131D / K147D | Ent.: A1D, K8D, N17D | CO: H35L | 96 |
| | | Con.: K147D | | |
| | | BB: N121D | | |
| | | Exit: K131D | | |
| G1953 | K8D / T9D / D13N / H35G / K46D | Ent.: K8D, T9D K46D | CO: H35G | 97 |
| | | | EX: D13N | |
| G1954 | K8D / T9D / D13N / H35G / N47D | Ent.: K8D, T9D N47D | CO: H35G | 98 |
| | | | EX: D13N | |
| G1956 | K8D/T9D/D13N/H35G / K46D / N121D | Ent.: K8D, T9D K46D | CO: H35G | 99 |
| | | BB: N121D | EX: D13N | |
| G2027 | T18D / H35L / K131D / K147D | Ent.:T18D | CO: H35L | 100 |
| | | Con.: K147D | | |
| | | Exit: K131D | | |
| G2080 | K8N / H35L / E111A / M113D / T115D / T117D / K147A | Ent.:K8D | CO: H35L, E111A | 101 |
| | | Con.: M113D | WC: M113D, K147A | |
| | | BB: T115D, T117D | | |
| **Key:** Ent. = entrance of the heptameric pore; Con. = constriction site; BB = beta barrel; Exit = exit of the beta barrel; CO = modifications that control oligomerization; WC = | | | | |
| modifications that widen the constriction site; CV = Coefficient of Variation of the arrival rate; EX = Improve expression; pI = increase isoelectric point; LI = improved lifetime | | | | |

A pPR-IBA2 plasmid (IBA Life Sciences, Germany) comprising a modified α-HL polynucleotides as set forth above was transformed into *E.coli* BL21 DE3 cell line (Thermo Fisher, Waltham, MA, USA) and the transformed cells were cultivated for protein expression according to the manufacturer's instructions. The cultivated cells were harvested by centrifugation and then lysed via sonification. Polypeptides bearing the cleavable epitope tag were purified from the lysate by affinity column chromatography (PhyTip columns, PhyNexus, Inc., San Jose, CA). The epitope tags were cleaved from the polypeptides, and the α-HL monomers separated from the cleaved tags and uncleaved polypeptides via affinity column chromatography (PhyTip columns, PhyNexus, Inc., San Jose, CA).

### VIA2. Variant monomer expression

Some of the variants of Table 6 were screened for expression level by spectrophotometry. Briefly, cells were harvested from 1 mL of expression culture and the expressed variant bearing the epitope tag was eluted at a volume of 100 µL. A280 absorbance of the eluate was recorded and averaged for all colonies of each variant. A variant bearing H144A, N47K, and H35G substitutions relative to SEQ ID NO: 1 (variant G432) was used as a control. Results are shown in Table 7:

**Table 7**

| Variant ID | A280 Avg. | Variant ID | A280 Avg. | Variant ID | A280 Avg. |
|---|---|---|---|---|---|
| G367 | 0.040 | G1389 | 0.032 | G1318 | 0.024 |
| G616 | 0.166 | G1390 | 0.034 | G1319 | 0.039 |
| G617 | 0.074 | G1391† | 0.403 | G1589 | 0.030 |
| G618 | 0.099 | G1513 | 0.042 | G1590 | 0.031 |
| G619 | 0.111 | G1516 | 0.029 | G363 | 0.000 |
| G620 | 0.064 | G1514 | 0.037 | G365 | 0.012 |
| G621 | 0.067 | G1589 | 0.017 | G557 | 0.102 |
| G622 | 0.120 | G1590 | 0.012 | G403 | 0.195 |
| G623* | 0.577 | G1591 | 0.014 | G559 | 0.136 |
| G653 | 0.094 | G432 (#1) | 2.273 | G596 | 0.016 |
| G679 | 0.108 | G432 (#2) | 1.752 | G597 | 0.015 |
| G680 | 0.123 | G1598 | 0.056 | G681 | 0.147 |
| G725 | 0.024 | G1609* | 1.490 | G727 | 0.020 |
| G1599 | 0.073 | G1610* | 0.908 | G1143* | 1.120 |
| G1600 | 0.066 | G1637* | 0.883 | G1147* | 1.084 |
| G1608* | 0.907 | G1642 | 0.064 | G1145* | 0.219 |
| G1309 | 0.079 | G1643 | 0.075 | G1148 | 0.060 |
| G1310 | 0.053 | G1644* | 0.557 | G1149 | 0.107 |
| G1314 | 0.026 | G1645 | 0.080 | G1150 | 0.072 |
| G1313 | 0.049 | G1647 | 0.022 | G1308 | 0.089 |
| G1315 | 0.038 | G1651 | 0.176 | | |
| G1316 | 0.033 | G728 | 0.014 | | |
| * Variants in which at least one colony has an A280 of ≥0.3 (high expressers) | | | | | |

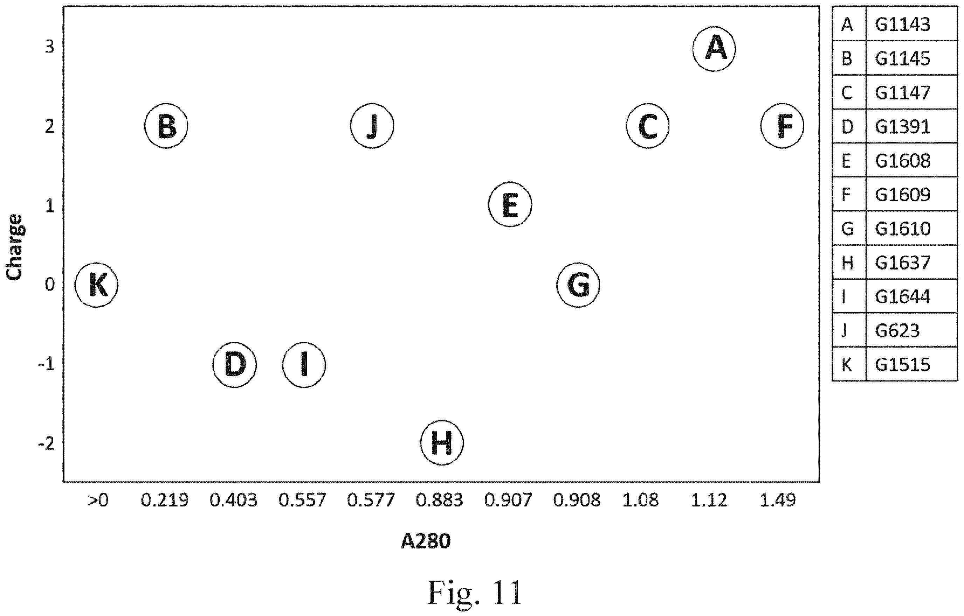

Additionally, the net charge of a homoheptamer formed from a subset of these monomers was calculated. The net charge was calculated by summing the charge at pH 7.5 of all amino acid side chains located at a solvent-facing position of a solvent-filled channel. The calculated net charges were plotted against the A280 of the monomer. Results are shown at Fig. 11.

### VIA3. Homoheptamer formation

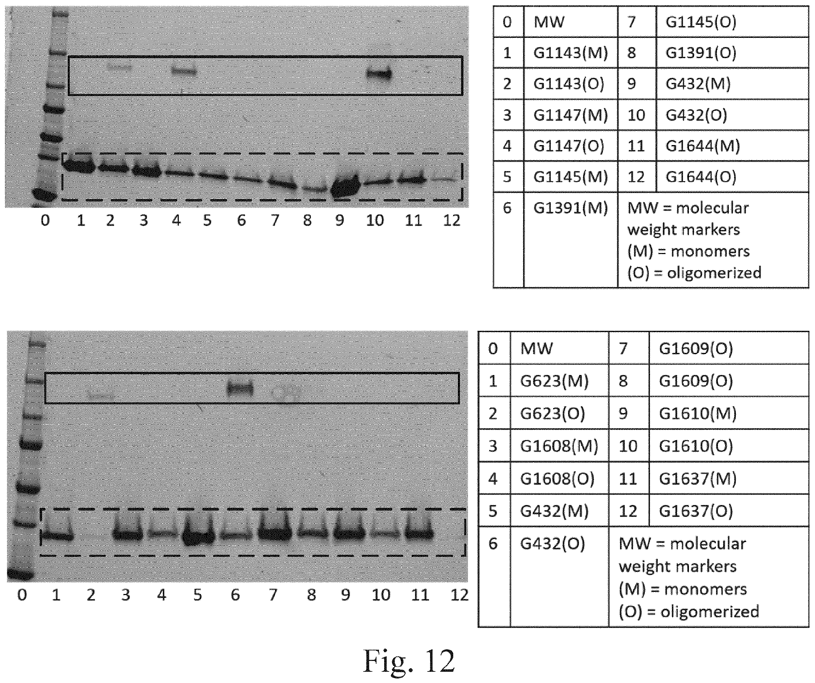

The variants deemed "high expressers" in section VA2 were tested for their ability to form heptameric oligomers. Diphytanoylphosphatidylcholine (DPhPC) lipid was solubilized in either 50mM Tris, 200mM NaCl, pH 8 to a final concentration of 50mg/ml and added to the α-HL monomers to a final lipid concentration of 5mg/ml. The mixture was incubated overnight at 37 °C. Thereafter, n-Octyl-β-D-Glucopyranoside (βOG) was added to a final concentration of 5% (weight/volume) to solubilize the resulting lipid-protein mixture. The sample was centrifuged to clear protein aggregates and left over lipid complexes and the supernatant was collected. Monomer preparations were made by diluting the monomer to a final protein concentration of 5 mg/mL in 50mM Tris, 50mM NaCl, pH 8. Samples were fractionated by sodium dodecyl sulfate-polyacrylamide gel electrophoresis (SDS-PAGE) along with samples of the monomer preparation. Exemplary images of gels are illustrated at Fig. 12. Black boxes show the expected migration rate of heptamers. As illustrated in Fig. 12, monomers G623 (bottom gel, columns 1 and 2), G1143 (top gel, columns 1 and 2), and G1147 (top gel, columns 3 and 4) were capable of forming heptamers.

Oligomerization of monomer G1637 was attempted with alternate conditions.

First, oligomerization was repeated using either a 2-(N-morpholino)ethanesulfonic acid (MES) buffer composition [50 mM MES, pH 6.0, 50 mM NaCl] or a buffer composition containing 3M trimethylamine N-oxide (TMAO) [3M TMAO, 50 mM Tris, 50 mM NaCl, pH 8]. DPhPC was solubilized in either buffer to a final concentration of 50 mg/ml and added to the α-HL monomer to a final lipid concentration of 5mg/ml. The mixture was incubated overnight at 37 °C. Thereafter, n-Octyl-β-D-Glucopyranoside (βOG) was added to a final concentration of 5% (weight/volume) to solubilize the resulting lipid-protein mixture. The sample was centrifuged to clear protein aggregates and left over lipid complexes and the supernatant was collected. Monomer preparations were made by diluting the monomer to a final protein concentration of 5 mg/mL in either buffer. Exemplary images of gels are illustrated at Fig. 13A. Solid black boxes show the expected migration rate of heptamers, while dashed black boxes show the expected migration rate of monomer. As illustrated in Fig. 13A, oligomerization occurred in the presence of TMAO, but not the MES buffer.

Second, the following buffers were used for oligomerization of G1637: (1) 50 mM sodium acetate, 50 mM NaCl, and 3M TMAO, pH 4.8; (2) 50 mM MES, 50 mM NaCl, and 3M TMAO, pH 6.0; (3) 50 mM potassium phosphate, 50 mM NaCl, and 3M TMAO, pH 7.4; and (4) 50 mM Tris, 50 mMNaCl, and 3M TMAO, pH. 8.0. DPhPC was solubilized in the buffers to a final concentration of 50 mg/ml and added to the α-HL monomer to a final lipid concentration of 5mg/ml. The mixture was incubated overnight at 37 °C. Thereafter, n-Octyl-β-D-Glucopyranoside (βOG) was added to a final concentration of 5% (weight/volume) to solubilize the resulting lipid-protein mixture. The sample was centrifuged to clear protein aggregates and left over lipid complexes and the supernatant was collected. Monomer preparations were made by diluting the monomer to a final protein concentration of 5 mg/mL in either buffer. Exemplary images of gels are illustrated at Fig. 13B. Solid black boxes show the expected migration rate of heptamers, while dashed black boxes show the expected migration rate of monomer. As illustrated in Fig. 13B, oligomerization occurred in in all 4 buffer compositions, with the highest yield of heptamers occurring in the potassium phosphate and Tris buffers.

### VIA4. 6:1 heteroheptamer formation and purification

The ability to form 6:1 pores is useful for sequencing applications because allows the number of polymerases per active well to closely controlled. Variants therefore were tested for their ability to form 6:1 heteroheptamers. Variants were mixed with a partner in following combinations:

**Table 8**

| **Variant** | **Partner** | **Variant: Partner (w/w)** |
|---|---|---|
| G1147 | WT | 4:1, 5:1, 6:1, 7:1, 8:1, 9:1 |
| G1515 | WT | 1:6, 1:7, 1:5, 1:3, 1:1, 3:1, 4:1, 5:1, 6:1, 7:1, 8:1, 9:1 |
| G1713 | WT | 9:1 |
| G1752 | WT | 9:1 |
| WT = SEQ ID NO: 1 with SpyTag modification at C-terminus | | |

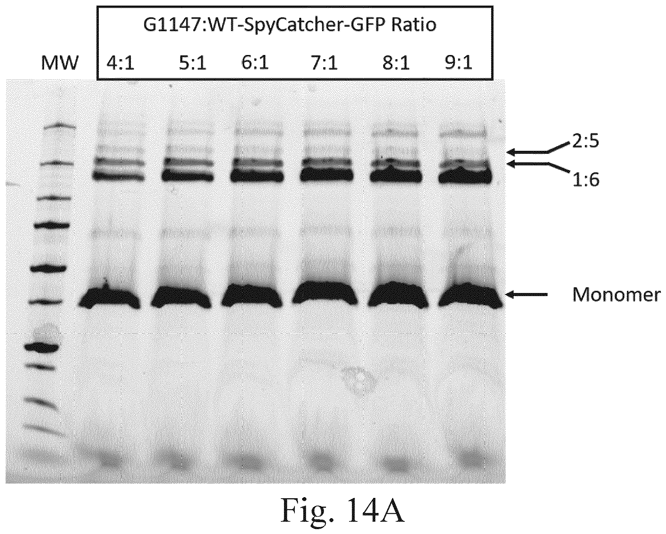

DPhPC lipid was solubilized in 50mM Tris, 200mM NaCl, pH 8 to a final concentration of 50mg/ml and added to the mixture of α-HL monomers to a final lipid concentration of 5 mg/ml. The mixture was incubated overnight at 37 °C. Thereafter, n-Octyl-β-D-Glucopyranoside (βOG) was added to a final concentration of 5% (weight/volume) to solubilize the resulting lipid-protein mixture. The sample was centrifuged to clear protein aggregates and left over lipid complexes and the supernatant was collected. Samples were purified by cation exchange chromatography to enrich the oligomeric fraction and fractionated by SDS-PAGE. A SpyCatcher-Green Fluorescent Protein (SC-GFP) conjugate was mixed with some aliquots of the resulting heptamers to attach the SC-GFP to the C-terminal SpyTag of the wild type monomer. The added mass of the SC-GFP altered the expected SDS-PAGE migration, such that 1:6 heteroheptamers (WT:G1147) migrated at a different rate than either 2:5 heteroheptamers or homoheptamers. Exemplary images of gels (and in some cases the accompanying chromatography graphs) are illustrated at FIG. 14A-14G.

FIG. 14A illustrates oligomerization of monomer G1147 with a wild-type (WT) α-hemolysin monomer. The G1147 and WT-SC-GFP monomers were mixed with G1147 in excess at ratios (w/w) of 4:1, 5:1, 6:1, 7:1, 8:1, and 9:1. The SC-GFP conjugate added additional mass to the wild type monomer, such that 1:6 heteroheptamers (WT:G1147) migrated at a different rate than either 2:5 heteroheptamers or homoheptamers. As indicated by the arrows, 1:6 heteroheptamers (1 WT monomer to 6 G1147 monomers) were formed, as well as 2:5 heteroheptamers.

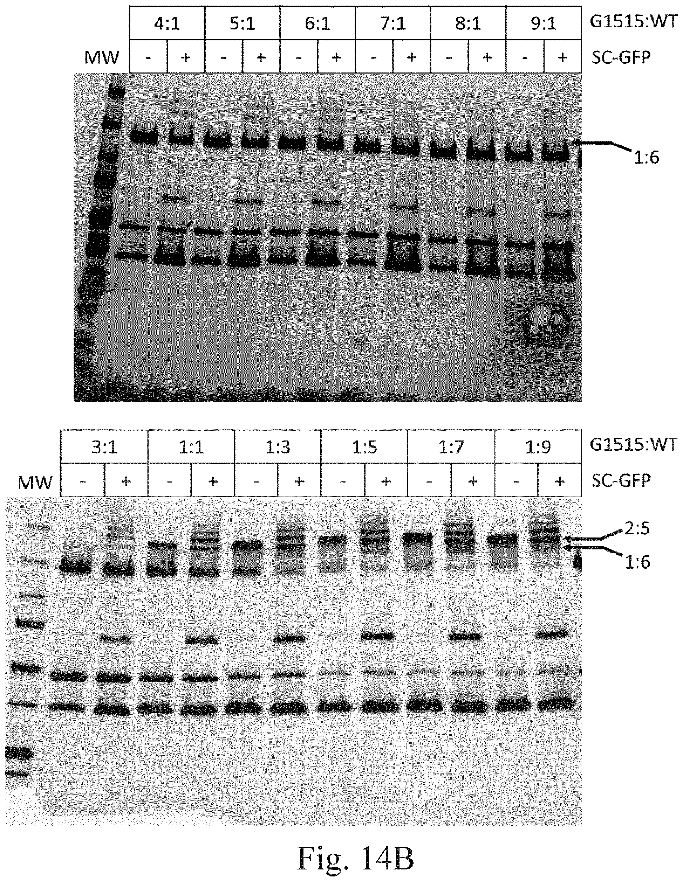

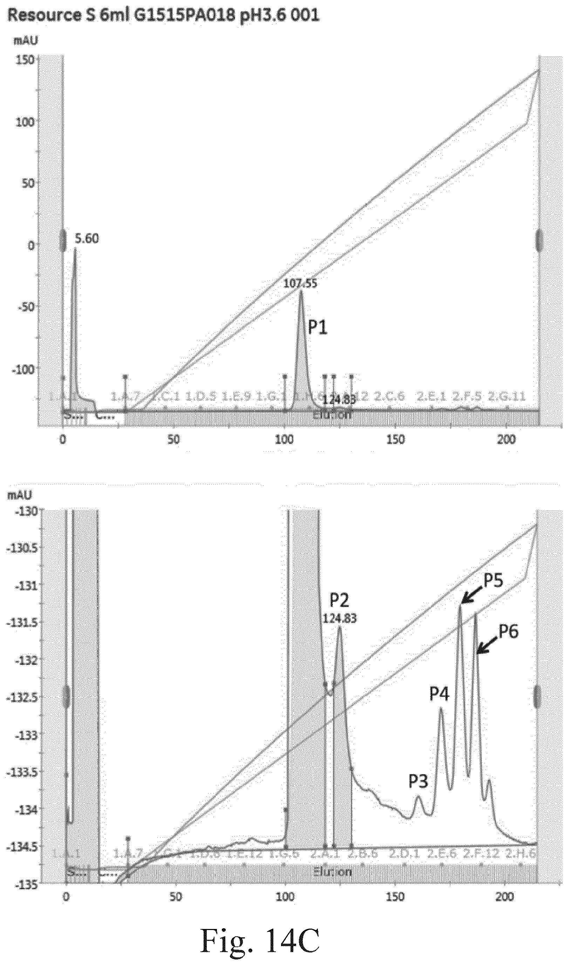

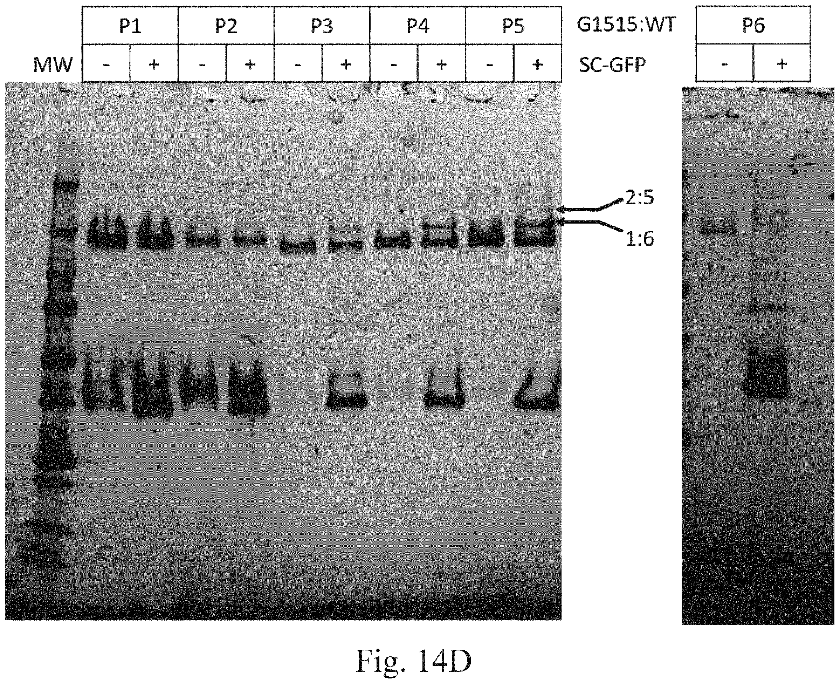

FIG. 14B - FIG. 14D illustrate oligomerization of monomer G1515 with WT monomer. FIG. 14B illustrates oligomerization with various ratios of G1515 to WT, including examples in which G1515 is in excess (ratios 3:1, 4:1, 5:1, 6:1, 7:1, 8:1, and 9:1), in which G1515 and WT are present in equivalent amounts (ratio 1:1), and in which WT monomer is in excess (ratios of 1:3, 1:5, 1:7, and 1:9). As indicated by the arrows, 1:6 heteroheptamers (1 WT monomer to 6 G1515 monomers) were formed, as well as 2:5 heteroheptamers (2 WT monomer to 5 G1515 monomers). FIG. 14C and FIG. 14D show a cation exchange chromatography run (FIG. 14C) and an SDS-PAGE gel of the resulting fractions (FIG. 14D) of a heptamer generated from a 9:1 mix of G1515 to WT. 6 fractions (labeled P1-P6) were collected (FIG. 14C). Fractions were analyzed by SDS-PAGE, results of which are shown at FIG. 14D.

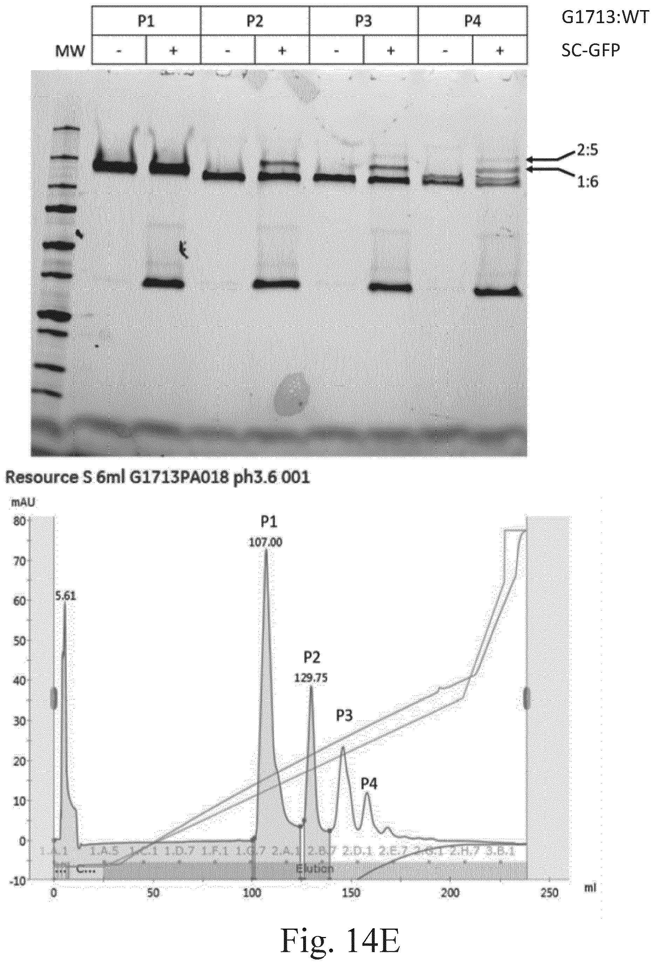

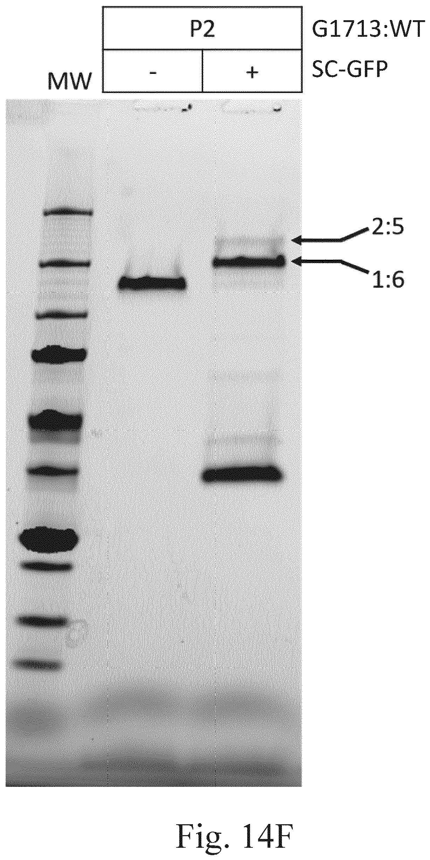

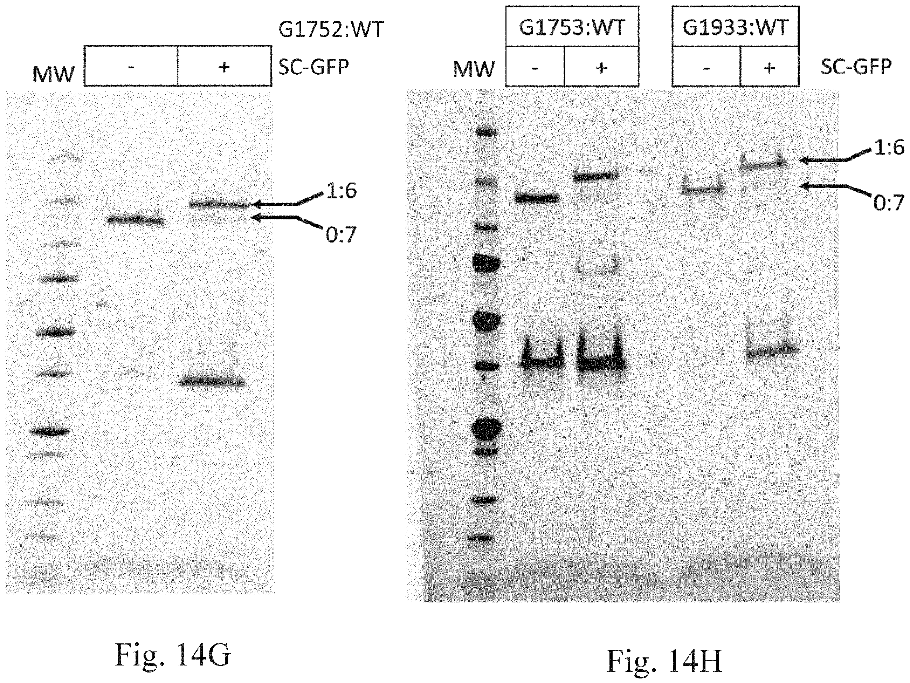

FIG. 14E illustrates purification by cation exchange chromatography run of a heptamer generated from a 9:1 mix of G1713 to WT. 4 fractions (labeled P1-P4) were collected and fractions were analyzed by SDS-PAGE, results of which are shown at FIG. 14E. Fraction P2 appeared to have the highest yield of 1:6 oligomer compared to 2:5 oligomer. Fraction P2 was subsequently further purified using immobilized metal affinity chromatography (IMAC) and analyzed by SDS-PAGE. Results are shown at FIG. 14F. As can be seen, fractionation by cation exchange chromatography followed by IMAC results in a very high purity of 1:6 heptamer. This process was repeated with heptamers formed from G1752 + WT (FIG. 14G), G1753 + WT (FIG. 14H, left), and G1933 + WT (FIG. 14H, right), each of which similarly shows a very high purity of 1:6 heptamer.

Additionally, the following pores have been generated:

**Table 9**

| **P-Number** | **Component 1** | **Comp. 1 Copies** | **Component 2** | **Comp. 2 Copies** |
|---|---|---|---|---|
| P-0022 | G617 | 7 | N/A | N/A |
| P-0023 | G617 | 6 | G1030 | 1 |
| P-0051 | G724 | 6 | pA018 | 1 |
| P-0186 | G1441 | 6 | pA018 | 1 |
| P-0192 | G1429 | 7 | N/A | N/A |
| P-0194 | G1440 | 6 | pA018 | 1 |
| P-0234 | G1515 | 7 | N/A | N/A |
| P-0236 | G697 | 7 | N/A | N/A |
| P-0237 | G1316 | 7 | N/A | N/A |
| P-0238 | G1317 | 7 | N/A | N/A |
| P-0241 | G1313 | 7 | N/A | N/A |
| P-0242 | G1314 | 7 | N/A | N/A |
| P-0243 | G1315 | 7 | N/A | N/A |
| P-0262 | G1646 | 7 | N/A | N/A |
| P-0263 | G1608 | 7 | N/A | N/A |
| P-0264 | G1609 | 7 | N/A | N/A |
| P-0271 | G1687 | 7 | N/A | N/A |
| P-0272 | G1701 | 7 | N/A | N/A |
| P-0273 | G1698 | 7 | N/A | N/A |
| P-0284 | G1637 | 7 | N/A | N/A |
| P-0285 | G1147 | 7 | N/A | N/A |
| P-0290 | G1713 | 6 | pA018 | 1 |
| P-303 | G1752 | 6 | pA018 | 1 |
| P-0326 | G1748 | 6 | pA018 | 1 |
| P-0327 | G1749 | 6 | pA018 | 1 |
| P-0331 | G1754 | 6 | pA018 | 1 |
| P-0332 | G1756 | 6 | pA018 | 1 |
| P-0338 | G1755 | 6 | pA018 | 1 |
| P-0340 | G1757 | 6 | pA018 | 1 |
| P-0341 | G1758 | 6 | pA018 | 1 |
| P-0360 | G1918 | 6 | pA018 | 1 |
| P-0361 | G1910 | 6 | pA018 | 1 |
| P-0362 | G1911 | 6 | pA018 | 1 |
| P-0365 | G1751 | 6 | pA018 | 1 |
| P-0372 | G1933 | 6 | pA018 | 1 |
| P-0339 | G1814 | 6 | pA018 | 1 |
| P-0359 | G1815 | 6 | pA018 | 1 |
| P-0366 | G1914 | 6 | pA018 | 1 |
| P-0371 | G1753 | 6 | pA018 | 1 |
| P-0408 | G1814 | 6 | pA018b | 1 |
| P-0430 | G2084 | 6 | G2132 | 1 |

As used in Table 9, "pA018" refers to a wild type α-HL having a C-terminus modified with a SpyTag construct. "pA018b" refers to pA018 construct except that the C-terminus has been modified to contain a non-native asparagine residue at which the SpyTag construct is attached. "G2132" refers to SEQ ID NO: 1 with the following modifications: H35L, E111A, M113D, T115D, T117D, K147A substitutions and the C-terminal modification of pA018b.

### VIB. Example 2: Positively-tagged nucleoside oligophosphates

### VIB1. Biotinylated tags

In order to identify candidate tags structures for use in nucleoside polyphosphate development, biotinylated polymer tags were synthesized for use in free capture experiments with modified α-HL pores. Polymers were synthesized by Fmoc solid phase peptide synthesis using a prelude X synthesizer (Gyros Protein Technologies AG) on ChemMatrix^{®} Rink amide resin (Gyros Protein Technologies AG) using N-[(7-Aza-1j-benzotriazol-1-yl) (dimethylamino)-methylene]-Nmethylmethanaminium hexafluorophosphate N-oxide (HATU) as a coupling reagent with N-methylmorpholine (NMM) as a base, 20% NMM in N,N-dimethylformamide (DMF) as deprotection solution.

The following polymer tags were generated:

**Table 10**

| **ID** | **Tag Sequence** | **SEQ ID** | **ID** | **Tag Sequence** | **SEQ ID** |
|---|---|---|---|---|---|
| 14 | K-(K₂P₂)₁₀-K₄-NH₂ | 102 | 77 | hex-4NPA-(Dap′2-P2)13-K4- NH2 | 181 |
| 15 | K₂-(KP)₂₀-K₄-NH₂ | 103 | 78 | hex-4NPA-(K′-4Ap-P)₁₅-K₄- NH₂ | 182 |
| 16 | K'K-(K₂P₂)₁₀-K₄-NH₂ | 104 | 79 | hex-4NPA-(K′2-Ahx)5-K′4- NH2 | 183 |
| 17 | K'K₂-(KP)₂₀-K₄-NH₂ | 105 | 80 | hex-4NPA-(K'₂-G₂)₅-K'₄-NH₂ | 184 |
| 18 | K'R-(R₂P₂)₁₀-R₄-NH₂ | 106 | 81 | Hex-4NPA-(K'₂-G)₁₀-K'₄-NH₂ | 185 |
| 19 | K'R-(R₂P₃)₁₀-R₄-NH₂ | 107 | 82 | Hex-4NPA-(K'₂-A)₁₀-K'₄-NH₂ | 186 |
| 20 | K'(Aeg₂-P₂)₁₀-K₄-NH₂ | 108 | 83 | Hex-4NPA-(K'₂-S)₁₀-K'₄-NH₂ | 187 |
| 21 | K'(Aeg-P₂)₁₀-K₄-NH₂ | 109 | 84 | Hex-4NPA-(K'₂-F)₁₀-K'₄-NH₂ | 188 |
| 22 | K'K-(K₂P)₁₀-K₄-NH₂ | 110 | 85 | Hex-4NPA-(K′2-Bnop)10-K′4- NH2 | 189 |
| 23 | K'K-(K₂P₃)₁₀-K₄-NH₂ | 111 | 86 | Hex-4NPA-(K'₂-Q)₁₀-K'₄-NH₂ | 190 |
| 24 | K'-(K'₂P)₁₀-K₄-NH₂ | 112 | 87 | hex-4NPA-(K′2P)8-(pnaT2-P)3- (K′2-P)1-K′4-NH2 | 191 |
| 25 | (K'₂P₂)₁₀-K₄-NH₂ | 113 | 88 | hex-4NPA-(K′2P)6-(pnaT2-P)3- (K′2-P)3-K′4-NH2 | 192 |
| 26 | K'-(K'P₂)₁₀-K₄-NH₂ | 114 | 89 | hex-4NPA-(K′2P)4-(pnaT2-P)3- (K′2P)5-K′4-NH2 | 193 |
| 27 | (K′2-BnoP-P)10-K4- NH2 | 115 | 90 | Hex-4NPA-(K′-OAhx)10-K′4- NH2 | 194 |
| 28 | K'-(KGP₂)₁₀-K₄-NH₂ | 116 | 91 | Hex-4NPA-(K′2-OAhx)10-K′4- NH2 | 195 |
| 29 | K'-(K'P₂)₅-K₄-NH₂ | 117 | 92 | prop-PEG₂₄-Hex-K'₂-K₄NH₂ | 196 |
| 30 | (K'₂P₂)₄-K₄-NH₂ | 118 | 93 | prop-PEG24-NHCO-PEG12- Hex-K′2-K4-NH2 | 197 |
| 31 | (K'₂P₂)₇-K₄-NH₂ | 119 | 94 | prop-PEG24-NHCO-PEG24- Hex-K′2-K4NH2 | 198 |
| 32 | K'-(K₂P₂)₄-K₄-NH₂ | 120 | 95 | Hex-4NPA-(Aeg-OAhx)10- K′4-NH2 | 199 |
| 33 | K'-(K₂P₂)₇-K₄-NH₂ | 121 | 96 | Hex-4NPA-(K′-OAhx₂)₁₀-K′₄- NH₂ | 200 |
| 34 | K'-(KAP₂)₇-K₄-NH₂ | 122 | 97 | 4NPA-PEG24-(pnaT2-P)3-K4- NH2 | 201 |
| 35 | K'-(KFP₂)₇-K₄-NH₂ | 123 | 98 | 4NPA-PEG24-(pnaT2-G)3-K4- NH2 | 202 |
| 36 | K'-(KHP₂)₇-K₄-NH₂ | 124 | 99 | 4NPA-PEG24-(pnaT2-K)3-K4- NH2 | 203 |
| 37 | K'-(KLP₂)₇-K₄-NH₂ | 125 | 100 | 4NPA-PEG₂₄-K'₂-K₄-NH | 204 |
| 38 | K'-(KQP₂)₇-K₄-NH₂ | 126 | 101 | 4NPA-(Aeg2-P)8-(tPNA2-P)3- Aeg2-P-K4-NH2 | 205 |
| 39 | K'-(KSP₂)₇-K₄-NH₂ | 127 | 102 | 4NPA-(K′2-P)8-((Kpeg4)4-P)3- K′2-P-K4-NH2 | 206 |
| 40 | Pra-(K₂P₂)₇-K₄-NH₂ | 128 | 103 | 4NPA-(K′2P)8-(G-Kpeg-G- Kpeg-P)3-K′2-P-K4-NH2 | 207 |
| 41 | Pra-(K'₂P₂)₇-K₄-NH₂ | 129 | 104 | 4NPA-(K′2-P)7-(tPNA2-P)4- K′2-P-K4-NH2 | 208 |
| 42 | PyrAla-(K₂P₂)₇-K₄-NH₂ | 130 | 105 | 4NPA-(K'2-P)8-(tPNA2-K)3- K'2-P-K4-NH2 | 209 |
| 43 | K'-(K₂P₂)₁₀-K₄-NH₂ | 131 | 106 | 4NPA-(K′2-P)8-(tPNA2-P)3- (K′2-P)3-K4-NH2 | 210 |
| 44 | K'-(K₂P₂)₁₃-K₄-NH₂ | 132 | 107 | Hex-4NPA-(Aeg2-G)6- (tPNA2-P)3-(Aeg2-G)3-K4-NH2 | 211 |
| 45 | K'-(K₂P₂)₁₆-K₄-NH₂ | 133 | 108 | Hex-4NPA-(Aeg2-G)7- (tPNA2-P)3-(Aeg2-G)2-K4-NH2 | 212 |
| 46 | K'-(K₂P₂)₁₀-K₈-NH₂ | 134 | 109 | Hex-4NPA-(Aeg2P)8-((G- KPeg4)2-P)3-(Aeg2P)1-K4-NH2 | 213 |
| 47 | K′-(K2P2)10-(Dap)4- NH2 | 135 | 110 | hex-4NPA-(Aeg2P)8-(Kpeg- G)8-(Aeg2-P)1-K4-NH2 | 214 |
| 48 | K'-(K₂P₂)₁₀-R₄-NH₂ | 136 | 111 | hex-4NPA-(Aeg2-P)7-(pnaT2- P)4-(Aeg2-P)1- K4-NH2 | 215 |
| 49 | K'-(K₂P₂)₁₀-R₈-NH₂ | 137 | 112 | hex-4NPA-(Aeg2P)8-(Aeg- pnaT3)2-Aeg-K4- NH2 | 216 |
| 50 | K'-(K₂P₂)₁₀-(Dapa)₄-NH₂ | 138 | 113 | Hex-4NPA-PEG24-K'2-K4- NH2 | 217 |
| 51 | K'-(bAlaK)₁₅-K₄-NH₂ | 139 | 114 | Hex-4NPA-(Aeg2-P)8-(G- Kpeg3-G-Kpeg3-P)3-Aeg2-P- K4-NH2 | 218 |
| 52 | K'-(bAla₂K)₁₀-K₄-NH₂ | 140 | 115 | Hex-4NPA-(Aeg2-P)7-(G- Kpeg3-G-Kpeg3-P)4-(Aeg2- P)1- K4-NH2 | 219 |
| 53 | K'-(Apa)₁₅-K₄-NH₂ | 141 | 116 | Hex-4NPA-(Aeg2-P)8- (Aegpeg42-P)3-(Aeg2-P)1-K4- NH2 | 220 |
| 54 | K'-(Ds)₁₀-K₄-NH₂ | 142 | 117 | Hex-4NPA-(Aeg2P)8-(pnaC2- P)3-(Aeg2-P)1-K4-NH2 | 221 |
| 55 | K'-(Ds-Dap)₈-K₄-NH₂ | 143 | 118 | Hex-K-(K₂-P₂)₁₀-K₄-NH2 | 222 |
| 56 | K'-(Ts)₈-K₄-NH₂ | 144 | 119 | Hex-K-(K₂-P₃)₁₀-K₄-NH₂ | 223 |
| 57 | K'-(KA₃)₁₀-K₄-NH₂ | 145 | 120 | Hex-K'-(K'₂-P)₁₀-K₄-NH₂ | 224 |
| 58 | K'-(KG)₂₀-K₄-NH₂ | 146 | 121 | Hex-4NPA-(K'₂-P)₁₀-K₄-NH₂ | 225 |
| 59 | K'-(K₂G₂)₁₀-K₄-NH₂ | 147 | 122 | Hex-4NPA-(bAla-K)20-K4- NH2 | 226 |
| 60 | K'-(K₂GP)₁₀-K₄-NH₂ | 148 | 123 | hex-K'-(AEG₂-P₂)₁₀-K₄-NH₂ | 227 |
| 61 | K′-(4Ap2–P2)10-K4- NH2 | 149 | 124 | Hex-4NPA-PEG24-(Aeg2P)- K4-NH2 | 228 |
| 62 | K'-(4Ap-P)₂₀-K₄-NH₂ | 150 | 125 | Hex-4NPA-(Aeg2P)6-PEG12- (Aeg2P)-K4-NH2 | 229 |
| 63 | K'-(4Ap)₄₀-K₄-NH₂ | 151 | 126 | Hex-4NPA-(Aeg-OAhx-P)7- (pnaT2-P)4-(Aeg2-P)1-K4- NH2 | 230 |
| 64 | Hex-4NPA-K′20-K4- NH2 | 168 | 127 | Hex-4NPA-(Aeg-OAhx-P)4- (Aeg2-P)3-(pnaT2-P)4-(Aeg2- P)1-K4-NH2 | 231 |
| 65 | Hex-4NPA-(Aeg- P2)10-K4-NH2 | 169 | 128 | Hex-4NPA-(Aeg2-P)3-(Aeg- OAhx-P)4-(pnaT2-P)4-(Aeg2- P)1-K4-NH2 | 232 |
| 66 | Hex-4NPA-(K′2-P)10- K4-NH2 | 170 | 129 | Hex-4NPA-(Aeg2-P)5-(Aeg- OAhx-P)2-(pnaT2-P)4-(Aeg2- P)1-K4-NH2 | 233 |
| 67 | Hex-4NPA-(Aeg2- P2)10-K4-NH2 | 171 | 130 | hex-4NPA-(Aeg-G2)11-K4- NH2 | 234 |
| 68 | 4NPA-(Aeg2-P)10-K4- NH2 | 172 | 131 | hex-4NPA-(Aeg-K-P)11-K4- NH2 | 235 |
| 69 | pra-(K'₂-P₂)₇-K₄-NH₂ | 173 | 132 | hex-4NPA-(Aeg-K-BnOP)11- K4-NH2 | 236 |
| 70 | 4NPA-(G-4Ap-P)₁₅-K₄-NH₂ | 174 | 133 | hex-4NPA-(Aeg2P)7-(Aeg-K- P)5-K4-NH2 | 237 |
| 71 | 4NPA-(K'₂-P)₁₀-K'₄-NH₂ | 175 | 134 | Hex-4NPA-(Aeg2P)7- (Aeg(Peg4)-Aeg-P)4- (Aeg2P)1-K4-NH2 | 238 |
| 72 | Hex-4NPA-(K-G)20- K4-NH2 | 176 | 135 | Hex-4NPA-(Aeg2P)7-(Aeg- pnaT-P)4-(Aeg2P)-K4-NH2 | 239 |
| 73 | Hex-4NPA-(K′-K- P)10-K4-NH2 | 177 | 136 | Hex-4NPA-(K'2P2)13-R4 | 240 |
| 74 | Hex-4NPA-(K′2P2)10- K4-NH2 | 178 | 137 | Hex-4NPA-(AEG2-P)12-K4- NH2 | 241 |
| 75 | 4NPA-(K′2-pnaT)10- K4-NH2 | 179 | 138 | Hex-4NPA-(Acp2-P)12-K4- NH2 | 242 |
| 76 | hex-4NPA-(K2-G2)13- K4-NH2 | 180 | Empty | | |

The foregoing tags are set forth with the amino-terminal amino acid at the left and the carboxy terminal amino acid at the right. The -NH₂ moiety at the carboxy terminus in each of the foregoing tags indicates that the carboxy-terminal amino acid is an amidated amino acid. Peptide tags were N-biotinylated on resin using N-hydroxysuccinimide ester of biotin (biotin-NHS) / N,N-Diisopropylethylamine (DIEA) in DMF. Peptides were fully deprotected and cleaved from the resin using trifluoroacetic acid (TFA) / triisopropylsilane (TIS) / water cleavage solution. The peptides were precipitated with ether and further purified using reversed-phase high-performance liquid chromatography (RP-HPLC) with 0.1% TFA/water/Acetonitrile solvent system. The quantity of these peptides were determined using a 4'-hydroxyazobenzene-2-carboxylic acid (HABA) test.

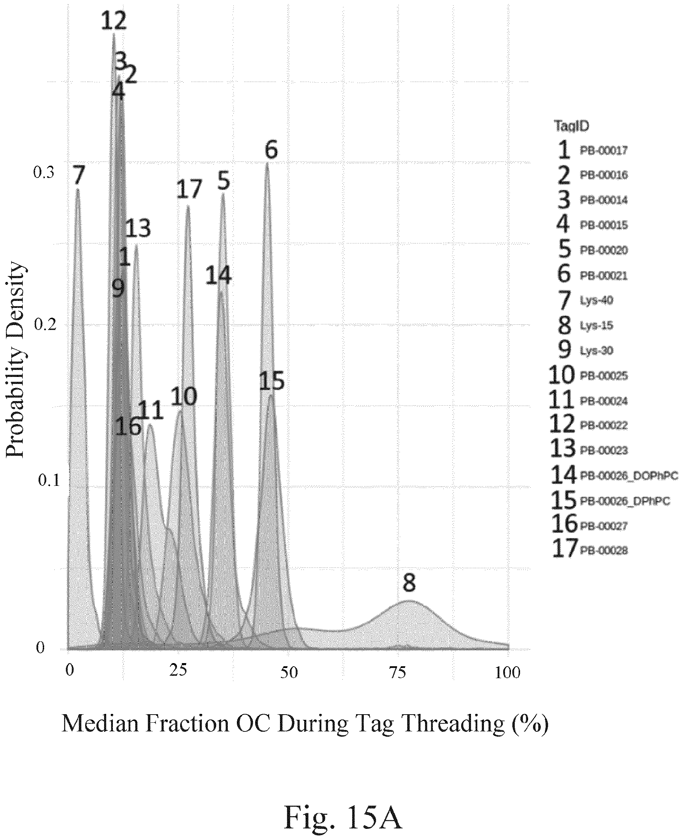

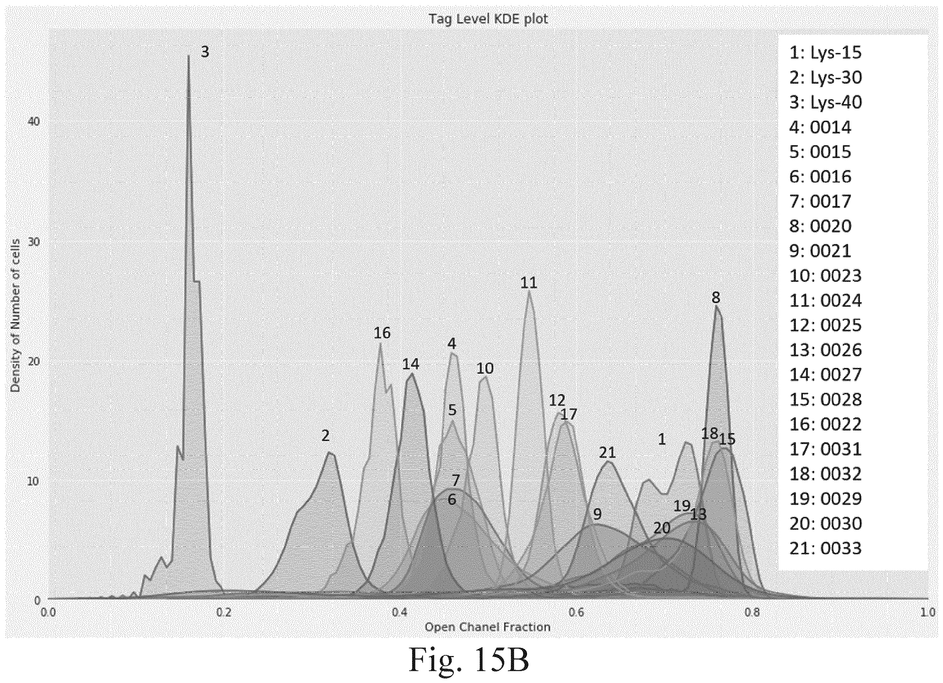

Various of the biotinylated tag structures were tested on an α-hemolysin-based chip to determine (a) the nanopore signals generated by the tag and (b) the arrival rate. Pore ID No. P-0234 (see Table 9) was used as the pore in two different membrane compositions (DOPhPC and DPhPC). Median fraction of open channel (Fraction OC) and mean arrival rate (AR) are reported at Table 11 (DOPhPC) and Table 12 (DPhPC). Tag level distributions are illustrated at Fig. 15A (DOPhPC) and Fig. 15B (DPhPC). As can be seen, different tag levels can be achieved.

**Table 11**

| **Tag ID** | **Fraction OC** | **AR** | **Tag ID** | **Fraction OC** | **AR** |
|---|---|---|---|---|---|
| Biotin-15x(Lysine)-Amidated | N/A | N/A | 22 | 0.106 | 0.62 |
| Biotin-30x(Lysine)-Amidated | 0.130 | 0.13 | 23 | 0.183 | 0.2 |
| Biotin-40x(Lysine)-Amidated | 0.028 | 0.87 | 24 | 0.208 | 5.79 |
| 14 | 0.116 | 1.42 | 25 | 0.261 | 1.71 |
| 15 | 0.123 | 1.23 | 26 + DOPhPC | 0.351 | 0.64 |
| 16 | 0.136 | 1.04 | 26 + DPhPC | 0.460 | 0.40 |
| 17 | 0.125 | 1.33 | 27 | 0.124 | 11.06 |
| 20 | 0.354 | 1.43 | 28 | 0.274 | 0.99 |
| 21 | 0.453 | 0.95 | | | |

**Table 12**

| **Tag ID** | **Fraction OC** | **AR** | **Tag ID** | **Fraction OC** | **AR** |
|---|---|---|---|---|---|
| Biotin-15x(Lysine)-Amidated | 0.70 | 1.83 | 24 | 0.55 | 0.3 |
| Biotin-30x(Lysine)-Amidated | 0.32 | 0.14 | 25 | 0.58 | 0.45 |
| Biotin-40x(Lysine)-Amidated | 0.16 | 0.15 | 26 | 0.71 | 0.3 |
| 14 | 0.46 | 0.61 | 27 | 0.41 | 0.47 |
| 15 | 0.46 | 0.55 | 28 | 0.76 | 0.3 |
| 16 | 0.46 | 0.42 | 29 | 0.71 | 1.96 |
| 17 | 0.47 | 0.48 | 30 | 0.69 | 1.70 |
| 20 | 0.76 | 0.275 | 31 | 0.58 | 0.41 |
| 21 | 0.62 | 2 | 32 | 0.72 | 2.66 |
| 22 | 0.38 | 0.24 | 33 | 0.64 | 0.35 |
| 23 | 0.49 | 0.18 | | | |

### VIB2. Tagged nucleotides

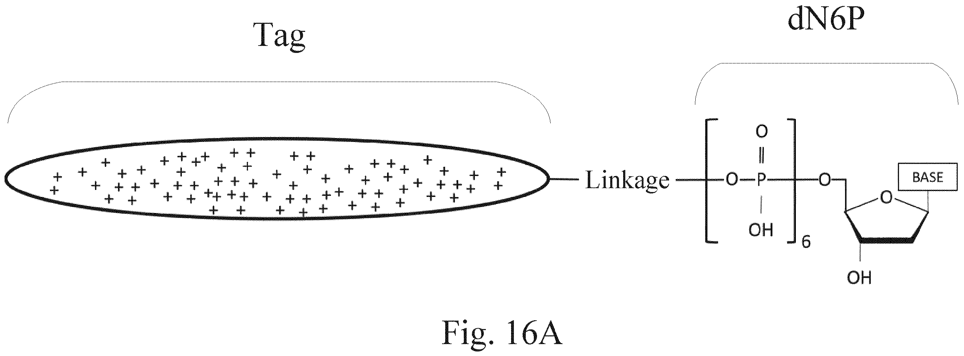

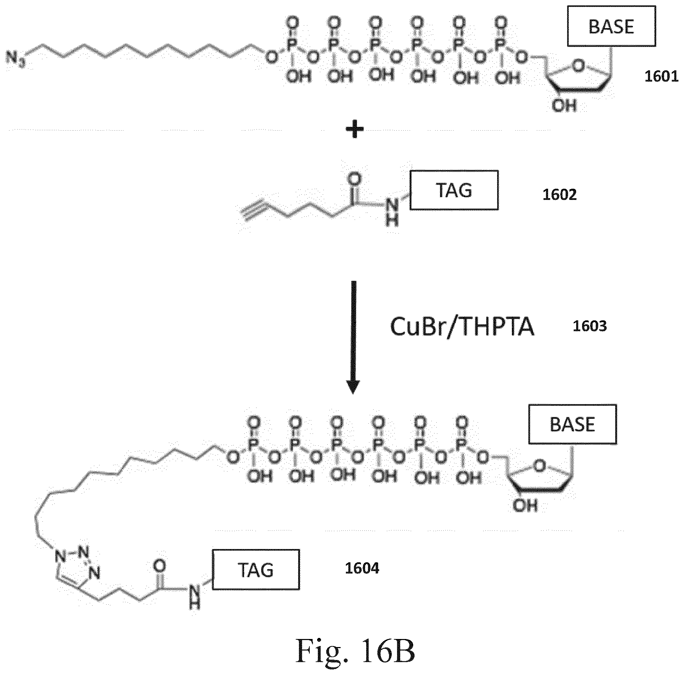

Tagged nucleotides were generated having the general structure of Fig. 16A, comprising a deoxyribonucleoside hexaphosphate (labeled "dN6P") attached to a positively charged tag moiety (labeled "Tag") via a chemical linkage between the tag and the terminal phosphate group of the dN6P. The general reaction scheme is set forth in Fig. 16B. In sum, an untagged dN6P molecule **1601** was provided, the untagged dN6P having the structure of Formula 5:

dN6P - (CH₂)₁₁ - N₃ **Formula 5**

wherein "dN6P" is selected from the group consisting of deoxyadenosine hexaphosphate (dA6P), deoxycytidine hexaphosphate (dC6P), deoxythymidine hexaphosphate (dT6P), anddeoxyguanosine hexaphosphate (dG6P), and wherein (CH₂)₁₁ is an unbranched 11 carbon chain in which one of the terminal carbons is bound to the terminal phosphate of dN6P and the other terminal carbon is attached to the azide group. Also provided was a peptide tag molecule bearing a 5-hexynamide moiety attached the N-terminal amino acid **1602,** the peptide tag molecule having a structure according to Formula 6: 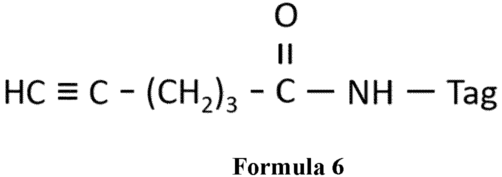 wherein: "Tag" is a tag structure of Formula 2 in which the carboxylic acid group of the C-terminal amino acid is converted to an amide group, and wherein the 5-hexynamide moiety (Hex) is located at the α-amine of the N-terminal amino acid unless the N-terminal amino acid is ε-linked lysine (K'), in which case Hex is located at the ε-amine of K'. The untagged dN6P molecule **1601** was reacted with the peptide tag molecule **1602** in the presence of a CuBr / tris-hydroxypropyltriazolylmethylamine (THPTA) solution **1603** to obtain a tagged dN6P having a 1,2,3-triazole linkage **1604** according to the structure of Formula 7: 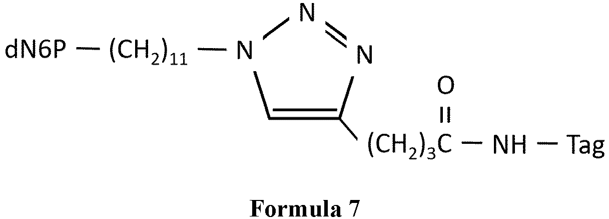 wherein "dN6P" is selected from the group consisting of deoxyadenosine hexaphosphate (dA6P), deoxycytidine hexaphosphate (dC6P), deoxythymidine hexaphosphate (dT6P), anddeoxyguanosine hexaphosphate (dG6P); wherein (CH₂)₁₁ is an unsaturated and unbranched 11 carbon hydrocarbon chain in which one of the terminal carbons is bound to the terminal phosphate of dN6P and the other terminal carbon is bound to the triazole ring, and Tag is the tag structure of Formula 2, the "NH" forming the illustrated peptide bond is contributed by the α-amine of the N-terminal amino acid unless the N-terminal amino acid is ε-linked lysine (K'), in which case the "NH" forming the illustrated peptide bond is contributed by the ε-amine of K'. Exemplary tagged nucleoside hexaphosphates synthesized according to this method are listed at Table 13.

**Table 13**

| **ID** | **dN6P** | **Tag Sequence (SEQ ID NO)** | **ID** | **dN6P** | **Tag Sequence (SEQ ID NO)** |
|---|---|---|---|---|---|
| P-A-00002 | dA6P | K₄₀-NH₂ (SEQ ID NO: 152) | P-T-00079 | dT6P | 4Npa-(K'₂-P)₁₀-K₄-NH₂ (SEQ ID NO: 155) |
| P-T-00069 | dT6P | K-(K₂P₂)₁₀-K₄-NH₂ (SEQ ID NO: 102) | P-G-00080 | dG6P | 4Npa-K'₂₀-K₄-NH₂ (SEQ ID NO: 156) |
| P-T-00070 | dT6P | K'-(Aeg-P₂)₁₀-K₄-NH₂ (SEQ ID NO: 109) | P-T-00081 | dT6P | 4Npa-K'₂₀-K₄-NH₂ (SEQ ID NO: 156) |
| P-T-00071 | dT6P | (K'₂P₂)₁₀-K₄-NH₂ (SEQ ID NO: 113) | P-A-00082 | dA6P | 4Npa-K'₂₀-K₄-NH₂ (SEQ ID NO: 156) |
| P-T-00072 | dT6P | K'-(K₂P)₁₀-K₄-NH₂ (SEQ ID NO: 153) | P-C-00083 | dC6P | 4Npa-K'₂₀-K₄-NH₂ (SEQ ID NO: 156) |
| P-T-00073 | dT6P | K'-(K₂P₃)₁₀-K₄-NH₂ (SEQ ID NO: 154) | P-T-00084 | dT6P | 4Npa-(Aeg-P₂)₁₀-K₄-NH₂ (SEQ ID NO: 157) |
| P-C-00074 | dC6P | K'-(Aeg₂-P₂)₁₀-K₄-NH₂ (SEQ ID NO: 108) | P-C-00085 | dC6P | 4Npa-(Aeg-P₂)₁₀-K₄-NH₂ (SEQ ID NO: 157) |
| P-T-00075 | dT6P | K'-(K'₂P)₁₀-K₄-NH₂ (SEQ ID NO: 112) | P-G-00086 | dG6P | 4Npa-(K'₂-P)₁₀-K₄-NH₂ (SEQ ID NO: 155) |
| P-G-00076 | dG6P | K'-(Aeg-P₂)₁₀-K₄-NH₂ (SEQ ID NO: 109) | P-C-00087 | dC6P | 4Npa-(K'₂-P)₁₀-K₄-NH₂ (SEQ ID NO: 155) |
| P-G-00077 | dG6P | K₄₀-NH₂ (SEQ ID NO: 152) | P-G- 00088 | dG6P (SEQ | 4Npa-(K'₂-P)₁₀-K₄-NH₂ ID NO: 155) |
| P-A-00078 | dA6P | K'-(Aeg₂-P₂)₁₀-K₄-NH₂ (SEQ ID NO: 108) | | | |

The foregoing tags are set forth with the N-terminal amino acid at the left and the C-terminal amino acid at the right. The -NH₂ moiety at the carboxy terminus of the tags indicates that the carboxy-terminal amino acid is an amidated amino acid.

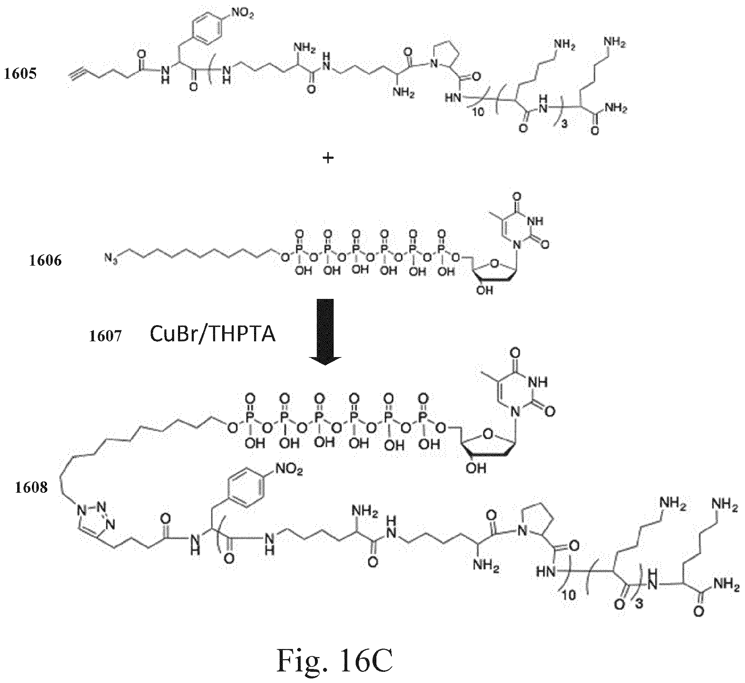

The synthesis protocol for tag P-T-00079 is set forth below and in Fig. 16C to illustrate this method. The solid peptide tag Hex-4Npa-(K'₂-P)₁₀-K₄-NH₂ (SEQ ID NO: 155 with an N-terminal 5-hexynamide) **1605** was dissolved in deionized water to make 10 mM concentration and the pH of the solution was adjusted to 7. A solution of NaCl was added, followed by dT6P-(CH₂)₁₁-N₂ **1606.** A solution of CuBr / tris-hydroxypropyltriazolylmethylamine (THPTA) was freshly prepared and mixed with the peptide/nucleotide solution **1607.** For some peptide sequences, cloudy solution or precipitation was observed. The reaction solution was sonicated briefly for about 30 seconds and vortexed vigorously for about a minute. The mixture solution was placed on a thermomixer at 25 °C mixing overnight. The precipitation was dissolved by gradual addition (5 µL at a time) of EDTA solution (1M) and vortexed vigorously. The solution was purified by preparative high performance liquid chromatography (prep-HPLC) using a polymer column (Hamilton PS-DVB) with the following gradient: 5-50% acetonitrile 0.085% trifluoroacetic acid (TFA) with deionized water 0.1% TFA in 30 minutes. The collected fractions were pooled and made neutral to basic with 0.1M triethylamine acetate (TEAA) using a 2M solution. The pH was adjusted to 7 by triethylamine. The solution was concentrated to remove acetonitrile and volatile salts and the remaining aqueous solution was lyophilized overnight to give oily residue. The oil was taken up in 500 µL of TEAA (2M) and was passed through a desalting column (Glen-Pak Cartridge), eluting with 0.1M TEAA (pH 7.5). Each fraction volume (500 µL) was collected and analyzed by a mass spectrometer. The fractions were lyophilized to give 20-40% yield of white solid product illustrated at **1608.**

### VIB3. Template extension using positive tags

A series of experiments were performed to demonstrate the ability of dN6P bearing positively charged tags to be polymerized. Two positively-tagged dN6P structures according to Formula 7 were used: (a) Formula 7 wherein the Tag is 4Npa-K'₂₀-K₄-NH₂ (SEQ ID NO: 156); and (b) Formula 7 wherein the Tag is K'-(Aeg-P₂)₁₀-K₄-NH₂ (SEQ ID NO: 109). Additionally, a negatively-tagged dN6P was used as a control. The negatively-tagged dN6P had a general structure of Formula 7, except that instead of using a positively charged polymer tag, the tag had the structure (sp2)₈-T₆-(sp2)₁₆-C3 ("sp2 tag", SEQ ID NO: 243), wherein T is deoxythymidine, C3 is a 3' propanol group resulting from oligonucleotide synthesis using a 3-(4,4'-dimethoxytrityl)-1,3-propandiol functionalized solid support or an initial spacer phosphoramidite C3 (3-(4,4'-dimethoxytrityloxy)propyl-1-[(2-cyanoethyl)-(N,N-diisopropyl)]-phosphoramidite) when using an universal synthesis support, and "sp2" is a monomer unit of Formula 8:

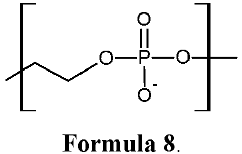

In some experiments, only tagged dG6P was used, and the remaining nucleotides were untagged dA6P, dC6P, and dT6P. In other experiments, each of dA6P, dT6P, dG6P, and dC6P were tagged with the indicated tag.

A fluorescent displacement assay was performed using 4 different variant Pol6 DNA polymerases in combination with tagged dG6P with untagged dA6P, dC6P, and dT6P. In brief, a hairpin template with a fluorophore was annealed to a primer with a quencher molecule. Upon extension of the hairpin template by the polymerase, the quencher primer was displaced and the fluorescent signal was measured. The change in fluorescence over time was measured in real time and used to determine extension rates. The graphs of Fig. 17A show fluorescence intensity (y-axis) versus time (x-axis) for each tag at 15, 10, 5, and 1 µM concentration with Pol6 variants 313, 431, 867, 1369, and 1743. The maximum fluorescence intensity of the positive tags is approximately 10% of the maximum fluorescence intensity of the negative tag. The fluorescence displacement assay was repeated in which each of dA6P, dT6P, dC6P, and dG6P were tagged, and the Kext of each polymerase with each concentration of tag was calculated. Fig. 17B illustrates the Kₑₓₜ using the negative tag, Fig. 17C illustrates the Kₑₓₜ using the 4Npa-K'₂₀-K₄-NH₂ tag, and Fig. 17D illustrates Kₑₓₜ of Pol6 1743 at various concentrations of tagged dN6P. Although a reduction in the catalysis rate was seen when using positively-charged tags, the data nonetheless demonstrate that positively-tagged dN6P can be polymerized by a DNA polymerase.

Polymerase extension rate (Kₑₓₜ) of a Pol6 variant 1743 was measured while attached to each of the following α-HL heptamers:

**Table 14**

| **Pore ID** | **Pore Constituents** | |
|---|---|---|
| | **Copy** | **Substitutions relative to SEQ ID NO: 1** |
| P269 | 6 | H35G, E111N, M113A, D127G, D128K, T129G, K131G, H144A, K147N, V149K |
| | 1 | H35G, E111N, M113A, D127G, D128G, T129G, K131G, K147N |
| P290 | 6 | A1D, H35L, K131D, H144A, K147D |
| | 1 | WT |
| P303 | 6 | N17D, G35L, K47D, E111A, M113D, T115D, T117D, N121D, H144A, K147A, D227N |
| | 1 | WT |

In each case, a variant Pol6 is attached to the "1" substituent of the heptamer via SpyCatcher/SpyTag system. In each case, untagged dA6P, dG6P, and dC6P were used. In one case, untagged dT6P (No Tag) was used. In another case, dT6P tagged with the sp2 tag was used (sp2 Tag). In the third case, dT6P tagged with K'-(Aeg-P₂)₁₀-K₄-NH₂ (Positive Tag) was used. The observed Kₑₓₜ is reported in Table 15 (unit = bases / sec):

**Table 15**

| | **P269** | **P290** | **P303** |
|---|---|---|---|
| No Tag | 1.99 | 2.15 | 2.00 |
| Positive Tag | 1.19 | 1.37 | 1.24 |
| sp2 Tag | 1.91 | 2.01 | 1.88 |

As can be seen, polymerization proceeded with the positively-tagged dN6P when the DNA polymerase was attached to an α-hemolysin pore.

### VIB4. Nanopore detection of positively-tagged dN6P polymerization

A series of experiments were performed to demonstrate that positively-charged tags can be detected on an α-hemolysin sequencing platform. In each experiment, α-HL heptamer P290 as described in Table 9 was used as the nanopore.

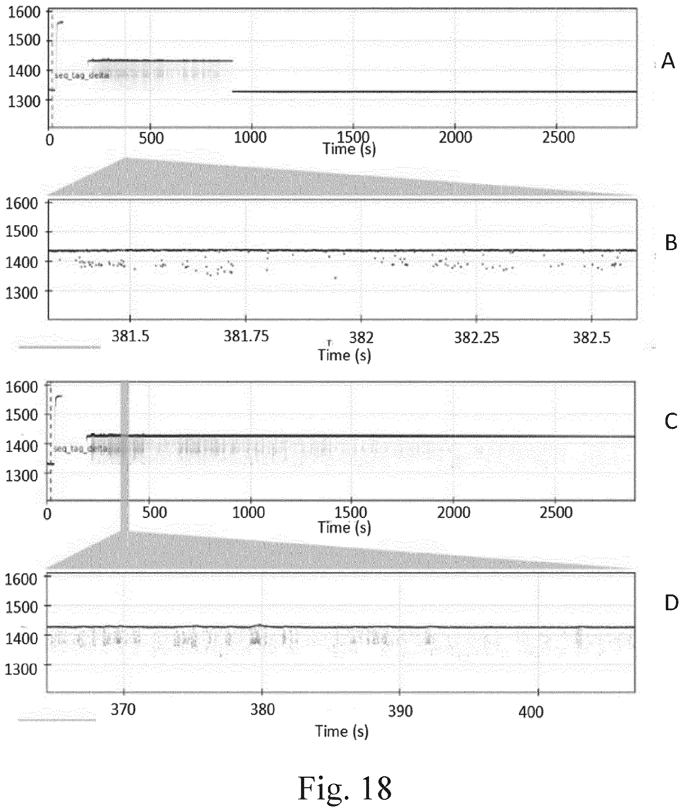

In one experiment, the following dN6P described in Table 13 were used: P-C-83, P-A-82, P-G-80, and P-T-81. Each of these tagged dN6P have the same positive tag: 4Npa-K'₂₀-K₄-NH₂. As illustrated in Fig. 18, a single tag level was observed as expected.

In another experiment, the following dN6P described in Table 13 were used: P-C-74, P-A-78, P-G-76, and P-T-70. In this case, dC6P and dA6P share the same tag [K'-(Aeg₂-P₂)₁₀-K₄-NH_{2]} (SEQ ID NO: 108), while dG6P and dT6P share a different tag [K'-(Aeg-P₂)₁₀-K₄-NH₂] (SEQ ID NO: 109). As illustrated in Fig. 19, two tag levels were observed as expected. Additionally, the observed capture rate (i.e. number of cells displaying a capture event) with these tags was lower than the tags containing 4Npa as the N-terminal amino acid.

This data demonstrates that multiple tag levels can be generated and detected with positively-tagged dN6P in a sequencing-by-synthesis reaction using an α-hemolysin pore modified to contain multiple non-native negatively charged amino acids.

### VIB5. Additional Tagged Nucleotides

Additional tagged nucleotides were generated as described above. Tags with clean signals are reported below in Table 16, with the observed tag level and net charge.

**Table 16**

| | **dN6P** | **Tag** | **Tag Level** | **Charge** |
|---|---|---|---|---|
| P-A-109 | dA6P | SEQ ID NO: 176 | ∼0.2 | 24 |
| P-A-112 | dA6P | SEQ ID NO: 226 | ∼0.2 | 24 |
| P-A-114 | dA6P | SEQ ID NO: 180 | ∼0.2 | 30 |
| P-A-122 | dA6P | SEQ ID NO: 179 | ∼0.5 | 24 |
| P-A-212 | dA6P | SEQ ID NO: 241 | ∼0.6 | 28 |
| P-A-213 | dA6P | SEQ ID NO: 242 | ∼0.45 | 28 |
| P-A-78 | dA6P | SEQ ID NO: 227 | ∼0.65 | 25 |
| P-C-102 | dC6P | SEQ ID NO: 129 | ∼0.6 | 18 |
| P-C-120 | dC6P | SEQ ID NO: 174 | ∼0.55 | 19 |
| P-C-131 | dC6P | SEQ ID NO: 175 | ∼0.5 | 24 |
| P-C-134 | dC6P | SEQ ID NO: 177 | ∼0.45 | 24 |
| P-C-135 | dC6P | SEQ ID NO: 178 | ∼0.5 | 24 |
| P-C-136 | dC6P | SEQ ID NO: 179 | ∼0.55 | 24 |
| P-C-138 | dC6P | SEQ ID NO: 181 | ∼0.4 | 30 |
| P-C-140 | dC6P | SEQ ID NO: 182 | ∼0.5 | 34 |
| P-C-144 | dC6P | SEQ ID NO: 183 | ∼0.62 | 14 |
| P-C-145 | dC6P | SEQ ID NO: 184 | ∼0.65 | 14 |
| P-C-146 | dC6P | SEQ ID NO: 185 | ∼0.7 | 24 |
| P-C-147 | dC6P | SEQ ID NO: 186 | ∼0.62 | 24 |
| P-C-148 | dC6P | SEQ ID NO: 187 | ∼0.55 | 24 |
| P-C-151 | dC6P | SEQ ID NO: 189 | ∼0.43 | 24 |
| P-C-152 | dC6P | SEQ ID NO: 188 | ∼0.45 | 24 |
| P-C-157 | dC6P | SEQ ID NO: 191 | ∼0.05 | 22 |
| P-C-158 | dC6P | SEQ ID NO: 192 | ∼0.05 | 22 |
| P-C-159 | dC6P | SEQ ID NO: 193 | ∼0.42 | 22 |
| P-C-160 | dC6P | SEQ ID NO: 194 | ∼0.55 | 14 |
| P-C-161 | dC6P | SEQ ID NO: 195 | ∼0.62 | 24 |
| P-C-162 | dC6P | SEQ ID NO: 196 | ∼0.76 | 6 |
| P-C-163 | dC6P | SEQ ID NO: 197 | ∼0.72 | 6 |
| P-C-164 | dC6P | SEQ ID NO: 198 | ∼0.72 | 6 |
| P-C-165 | dC6P | SEQ ID NO: 199 | ∼0.8 | 14 |
| P-C-166 | dC6P | SEQ ID NO: 200 | ∼0.58 | 14 |
| P-C-169 | dC6P | SEQ ID NO: 201 | ∼0.75 | 4 |
| P-C-170 | dC6P | SEQ ID NO: 204 | ∼0.75 | 6 |
| P-C-172 | dC6P | SEQ ID NO: 202 | ∼0.72 | 4 |
| P-C-193 | dC6P | SEQ ID NO: 228 | ∼0.75 | 6 |
| P-C-194 | dC6P | SEQ ID NO: 229 | ∼0.55 | 18 |
| P-C-196 | dC6P | SEQ ID NO: 230 | ∼0.6 | 13 |
| P-C-197 | dC6P | SEQ ID NO: 231 | ∼0.6 | 16 |
| P-C-198 | dC6P | SEQ ID NO: 232 | ∼0.6 | 16 |
| P-C-199 | dC6P | SEQ ID NO: 233 | ∼0.55 | 18 |
| P-C-220 | dC6P | SEQ ID NO: 234 | ∼0.7 | 26 |
| P-C-83 | dC6P | SEQ ID NO: 168 | ∼0.55 | 24 |
| P-C-85 | dC6P | SEQ ID NO: 169 | ∼0.6 | 14 |
| P-C-87 | dC6P | SEQ ID NO: 170 | ∼0.5 | 24 |
| P-C-94 | dC6P | SEQ ID NO: 172 | ∼0.55 | 24 |
| P-C-94 | dC6P | SEQ ID NO: 171 | ∼0.55 | 24 |
| P-G-115 | dG6P | SEQ ID NO: 177 | ∼0.5 | 24 |
| P-G-116 | dG6P | SEQ ID NO: 178 | ∼0.4 | 24 |
| P-G-185 | dG6P | SEQ ID NO: 235 | ∼0.5 | 26 |
| P-G-186 | dG6P | SEQ ID NO: 236 | ∼0.4 | 26 |
| P-G-195 | dG6P | SEQ ID NO: 237 | ∼0.55 | 28 |
| P-G-207 | dG6P | SEQ ID NO: 238 | ∼0.6 | 24 |
| P-G-208 | dG6P | SEQ ID NO: 239 | ∼0.5 | 24 |
| P-T-103 | dT6P | SEQ ID NO: 173 | ∼0.45 | 19 |
| P-T-123 | dT6P | SEQ ID NO: 181 | ∼0.5 | 30 |
| P-T-124 | dT6P | SEQ ID NO: 182 | ∼0.4 | 34 |
| P-T-130 | dT6P | SEQ ID NO: 240 | ∼0.15 | 30 |
| P-T-175 | dT6P | SEQ ID NO: 205 | ∼0.15 | 22 |
| P-T-178 | dT6P | SEQ ID NO: 206 | ∼0.2 | 22 |
| P-T-179 | dT6P | SEQ ID NO: 207 | ∼0.15 | 22 |
| P-T-180 | dT6P | SEQ ID NO: 208 | ∼0.05 | 20 |
| P-T-181 | dT6P | SEQ ID NO: 210 | ∼0.08 | 26 |
| P-T-183 | dT6P | SEQ ID NO: 211 | ∼0.35 | 22 |
| P-T-184 | dT6P | SEQ ID NO: 212 | ∼0.3 | 22 |
| P-T-189 | dT6P | SEQ ID NO: 213 | ∼0.15 | 22 |
| P-T-190 | dT6P | SEQ ID NO: 214 | ∼0.55 | 22 |
| P-T-192 | dT6P | SEQ ID NO: 216 | ∼0.25 | 23 |
| P-T-200 | dT6P | SEQ ID NO: 217 | ∼0.7 | 6 |
| P-T-209 | dT6P | SEQ ID NO: 220 | ∼0.35 | 22 |
| P-T-210 | dT6P | SEQ ID NO: 221 | ∼0.15 | 22 |
| P-T-69 | dT6P | SEQ ID NO: 222 | ∼0.35 | 25 |
| P-T-73 | dT6P | SEQ ID NO: 223 | ∼0.45 | 25 |
| P-T-75 | dT6P | SEQ ID NO: 224 | ∼0.45 | 25 |
| P-T-79 | dT6P | SEQ ID NO: 225 | ∼0.45 | 24 |
| P-T-84 | dT6P | SEQ ID NO: 169 | ∼0.6 | 14 |
| P-T-91 | dT6P | SEQ ID NO: 171 | ∼0.6 | 24 |

### X. References

1. Ayub et al., Nucleobase Recognition by Truncated α-Hemolysin Pores, ACS Nano, 2015, Vol. 9, Issue 8, 7895-7903.
2. Bhattacharya et al., Rectification of the Current in α-Hemolysin Pore Depends on the Cation Type: The Alkali Series Probed by Molecular Dynamics Simulations and Experiments, The Journal of Physical Chemistry, 2011, Vol. 115, Issue 10, pp. 4255-4264.
3. Chen & Liu, Fabrication and Applications of Solid-State Nanopores, 2019, Sensors, Vol. 19, Issue 8, E1886 ("Chen I").
4. Chen et al., Outer membrane protein G: Engineering a quiet pore for biosensing, Proceedings of the National Academy of Sciences of the United States of America, 2008, Vol. 105, Issue 17, pp. 6272-77 ("Chen II").
5. Chen et al., Fusion Protein Linkers: Property, Design and Functionality, Advanced Drug Delivery Reviews, 15 October 2013, Vol. 65, Issue 10, pp. 1357-1369 ("Chen III")
6. Chen, DNA polymerases drive DNA sequencing-by-synthesis technologies: both past and present, Frontiers in Microbiology, 2014, Vol. 5, Article 305 ("Chen IV").
7. Feng et al., Nanopore-based Fourth-generation DNA Sequencing Technology, 2015, Genomics, Proteomics & Bioinformatics, Vol. 13, Issue 1, pp. 4-16.
8. Gari et al., Quiet Outer Membrane Protein G (OmpG) Nanopore for Biosensing, ACS Sensors, April 14, 2019, Vol. 4, pp. 1230-35.
9. Goodman et al., Foldamers as versatile frameworks for the design and evolution of function, 2007, Vol. 3, pp. 252-62.
10. Hammerstein et al., Subunit dimers of α-hemolysin expand the engineering toolbox for protein Nanopores, Journal of Biological Chemistry, Vol. 286, Issue 16, pp. 14324-34.
11. Kumar et al., PEG-labeled nucleotides and nanopore detection for single molecule DNA sequencing by synthesis, 2012, Scientific Reports, Vol. 2, Art. 684.
12. Lee et al., Recent progress in solid-state nanopores, 2018, Advanced Materials, Vol. 30, Issue 42.
13. Licini et al., Oligopeptide Foldamers: From Structure to Function, European Journal of Organic Chemistry, 2005, Vol. 2005, Issue 6, pp. 969-77.
14. Manrao et al., Reading DNA at single-nucleotide resolution with a mutant MspA nanopore andphi29 DNA polymerase, 2012, Nature Biotechnology, Vol. 30, pp. 349-53.
15. Martinek & Fueloep, Peptidic foldamers: ramping up diversity, 2012, Chemical Society Reviews, Vol. 41, Issue 2, pp. 687-702.
16. Noskov et al., Ion Permeation through the α-Hemolysin Channel: Theoretical Studies Based on Brownian Dynamics and Poisson-Nernst-Plank Electrodiffusion Theory, Biophysical Journal, 2004, Vol. 87, Issue 4, pp. 2299-2309.
17. Pavlenok & Niederweis, Hetero-oligomeric MspA pores in Mycobacterium smegmatis, 2016, FEMS Microbiology Letters, Vol. 363, Issue 7, fnw046.
18. Song et al., Structure of Staphylococcal α-Hemolysin, a Heptameric Transmembrane Pore, Science, 1996, Vol. 274, No. 5294, pp. 1859-65.
19. Stoddart et al., Single-nucleotide discrimination in immobilized DNA oligonucleotides with a biological nanopore, Proceedings of the National Academy of Sciences of the United States of America, 2009, Vol. 106, Issue 19, pp. 7702-7707.
20. Szuromi, Synthesizing graphene nanopores, 2018, Science, Vol. 360, Issue 6385, pp. 166-68.
21. Wang et al., The evolution of nanopore sequencing, 2015, Frontiers in Genetics, Vol. 5, Art. 449 ("Wang I").
22. Wang et al., Engineering of Protein Nanopores for Sequencing, Chemical or Protein Sensing and Disease Diagnosis, 2018, Current Opinions in Biotechnology, Vol. 51, pp. 80-89 ("Wang II").
23. Wasfi et al., Graphene-based nanopore approaches for DNA sequencing: A literature review, 2018, Biosensors and Bioelectronics, Vol. 119, pp. 191-203.

## Claims

1. A nucleoside-5 ' -oligophosphate covalently linked to a polymer tag, wherein said polymer tag comprises a positively-charged segment (PCS), wherein said PCS is a homopolymer or a heteropolymer comprising from 5 monomer units to 100 monomer units, having a net-positive charge of at least +5 at pH 7.0, and having a charge density of at least 0.1, with the proviso that said PCS is not a homopolymer of arginine or α-linked lysine, wherein PCS comprises a structure according to Formula 1:
(REPEAT)ₐ Formula 1,
wherein:
"REPEAT" is a heteropolymeric sequence of at least 2 monomer units, wherein REPEAT has a charge density of at least 0.1; and
"a" is an integer ≥ 2, with the proviso that said integer is sufficient to result in a PCS with a net-charge of at least +5 at pH 7.0 and an overall length of from 5 to 100 monomer units.

2. The nucleoside-5'-oligophosphate of claim 1, wherein the amino acids or amino acid analogs are selected from the group consisting of Lysine (K), Diaminopropionic acid (Dap), ε-linked Lysine (K'), Aminoethyl-substituted diaminopropionic acid (Dapa), Proline (P), Propargylglycine (Pra), Arginine (R), 4-Benzyloxyproline (BnoP), Glycine (G), Aminoethyl-piperazineacetic acid (Apa), Alanine (A), Pyrrolyl alanine (PyrAla), Phenylalanine (F), Triethylenetriamine-succinamic acid (TETA), Histidine (H), Diethylenetriamine-succinamic acid (DETA), Leucine (L), 4-aminoproline (4Ap), Glutamine (Q), Aminoethylglycine (Aeg), Serine (S), β-alanine (bAla), 4-Nitrophenylalanine (4Npa), Aminohexanoic acid (Ahx), 2-aminobenzoic acid (2Aba), 3-aminobenzoic acid (3Aba), 4-aminobenzoic acid (4Aba), Oxoaminohexanoic acid (OAhx), Peptide Nucleic Acid (Thymine) (pnaT), Gamma-diaminopropionic acid (Dap'), Peptide nucleic acid (cytosine) (pnaC), Glutamate (Glu), 4-hydroxoproline (4-OHP), Ornithine (Orn), Citrulline (Cit), Lysine-Peg4 (KPeg4), Lysine-Peg3 (KPeg3), Lysine-Peg2 (KPeg2), and Aminoethylglycine-Peg4 (Aegpeg4).

3. The nucleoside-5'-oligophosphate of claim 2, wherein the PCS further comprises one or more Hexynyl (Hex) or Ethylene glycol (PEG) moiety.

4. The nucleoside-5'-oligophosphate of any of claims 1-3, wherein the polymer tag has a structure of Formula 2:
U - PCS -V Formula 2,
wherein:
U is optional and, when present, comprises 1 to 10 monomer units;
V is optional and, when present, comprises 1 to 10 monomer units; and
wherein said structure of Formula 2 has a net-positive charge of at least +5 at pH 7.0, and a charge density of at least 0.1.

5. The nucleoside-5'-oligophosphate of any of claim 4, wherein U and V have a net-neutral or a net-positive charge.

6. The nucleoside-5'-oligophosphate of claim 5, wherein U comprises one or more amino acids or amino acid analogs selected from the group consisting of lysine, a lysine derivative, arginine, ornithine, an aliphatic amino acid, a derivative of an aliphatic amino acid, an aromatic amino acids, a derivatives of an aromatic amino acid, proline, a derivative of proline, Dap, and Dapa.

7. The nucleoside-5'-oligophosphate of claim 6, wherein U is from 1 to 3 amino acids or amino acid analogs in length, wherein said amino acids or amino acid analogs are selected from the group consisting of K, K', 4Npa, Pra, and PyrAla.

8. The nucleoside-5'-oligophosphate of any of claims 1-7, having a structure according to Formula 3: 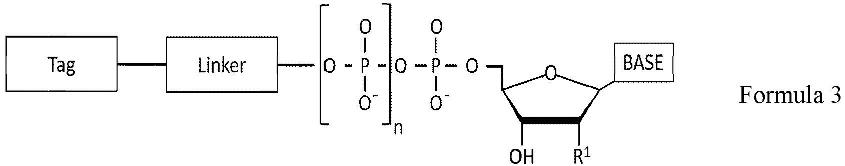
wherein Base is selected from adenine, cytosine, guanine, thymine, and uracil;
R¹ is selected from Hand OH; n is from 2 to 12; Linker is a linker comprising a covalently bonded chain of 2 to 100 atoms; and Tag is the polymer tag.

9. The nucleoside-5'-oligophosphate of claim 8, wherein the linker comprises a chemical group selected from the group consisting of: ester, ether, thioether, amine, amide, imide, carbonate, carbamate, squarate, thiazole, thiazolidine, hydrazone, oxime, triazole, dihydropyridazine, phosphodiester, polyethylene glycol (PEG), Pictet-Spengler adduct, and any combination thereof.

10. The nucleoside-5'-oligophosphate of claim 9, wherein Linker has a structure of Formula 4:
R⁴-R²-R³ Formula 4
wherein:
R² is selected from the group consisting of ester linkage, ether linkage, thioether linkage, amine linkage, amide linkage, imide linkage, carbonate linkage, carbamate linkage, squarate linkage, thiazole linkage, thiazolidine linkage, hydrazone linkage, oxime linkage, triazole linkage, dihydropyridazine linkage, phosphodiester linkage, polyethylene glycol (PEG) linkage, Pictet-Spengler adduct, and any combination thereof;
R³ comprises a saturated or unsaturated, branched or unbranched, substituted or unsubstituted carbon chain at least 2 carbons in length covalently bonded at one end to one of the phosphate moieties of the nucleotide oligophosphate and at the other end to R²; and
R⁴ comprises a saturated or unsaturated, branched or unbranched, substituted or unsubstituted carbon chain at least 2 carbons in length covalently bonded at one end to R² and at the other end to the polymer tag.

11. The nucleoside-5'-oligophosphate of claim 10, wherein R² is a triazole of Formula 4a: 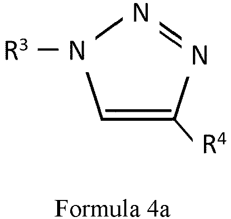 wherein the sum of the carbons in carbon chain R₃ and carbon chain R₄ is ≤ 96.

12. The nucleoside-5'-oligophosphate of claim 10, wherein R² is a triazole of Formula 4b: 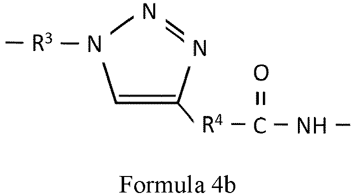 and wherein the -NH- of the peptide bond connected to R⁴ is contributed by the N-terminal amino acid or amino acid analog of the polymer tag.

13. A kit comprising:
a first nucleoside-5'-oligophosphate according to any of claims 1-12, wherein the base is adenosine,
a second nucleoside-5'-oligophosphate according to any of claims 1-12, wherein the base is cytosine,
a third nucleoside-5'-oligophosphate according to any of claims 1-12, wherein the base is guanine, and
a fourth nucleoside-5'-oligophosphate according to any of claims 1-12, wherein the base is thymine or uracil;
wherein the polymer tag of each of the first through fourth nucleoside-5'-oligophosphates is different.

14. A system for performing nucleic acid sequencing-by-synthesis (SBS), the system comprising:
(a) a chip comprising a plurality of sensing electrodes;
(b) an electrochemically resistive barrier disposed on a surface of the chip, wherein the barrier has a *cis* side and a *trans* side;
(c) a first electrolyte solution on the *cis* side of the barrier;
(d) a second electrolyte solution on the *trans* side of the barrier;
(e) a plurality of nanopores comprising a channel having sufficient negatively charged moieties to substantially repel a template and/or primer nucleic acid, wherein the nanopores are disposed in the barrier such that the channel permits ion exchange between the first electrolyte solution and the second electrolyte solution, and wherein at least a portion of the nanopores are close enough to one of the sensing electrodes that the sensing electrode can detect at least one characteristic of an electrical current flowing through the channel of the nanopore;
(f) a computer system in electronic communication with the sensing electrodes, wherein the computing system is adapted to record the characteristic of the electrical current flowing through the nanopore that is detected by the sensing electrode;
(g) a nucleic acid polymerase associated with the nanopore on the *cis* side of the barrier, wherein the nucleic acid polymerase is capable of catalyzing a template-dependent nucleic acid amplification reaction in the first electrolyte solution; and
(h) a set of nucleoside-5'-oligophosphates disposed in the first electrolyte solution, the set including at least a polymer-tagged adenosine nucleoside-5'-oligophosphate, a polymer-tagged guanine nucleoside-5'-oligophosphate, a polymer-tagged cytosine nucleoside-5'-oligophosphate, and either a polymer-tagged thymidine nucleoside-5'-oligophosphate or a polymer-tagged uracil nucleoside-5'-oligophosphate, wherein each of the polymer-tagged nucleoside-5'-oligophosphates is the nucleoside-5'-oligophosphate according to any of claims . 1- 12.

15. A sequencing-by-synthesis (SBS) method of sequencing a template nucleic acid, the method comprising:
providing a system according to claim 14 having a plurality of active nanopore sequencing complexes, each active nanopore sequencing complex comprising:
∘ at least one of the sensing electrodes;
∘ one of the nanopores inserted in the barrier in proximity to the sensing electrode, wherein a current is flowing through the nanopore and a characteristic of the current is detected by the sensing electrode;
o the nucleic acid polymerase associated with the nanopore; and
o the template nucleic acid complexed with the nucleic acid polymerase;
at the active nanopore sequencing complexes, incorporating the tagged nucleoside-5'-oligophosphates into a complementary nucleic acid of the template nucleic acid by a template-dependent nucleic acid amplification reaction catalyzed by the nucleic acid polymerase, wherein the polymer tag of the tagged nucleoside-5'-oligophosphate moves into or in proximity to the channel of the nanopore as the tagged nucleoside-5'-oligophosphate is incorporated into the complementary nucleic acid, and wherein movement of the polymer tag into or in proximity to the channel changes the characteristic of the current flowing through the nanopore;
detecting the change in the characteristic of the current flowing through the nanopore caused by the polymer tags with the sensing electrode and recording the change on the computer system; and
correlating each recorded change to one of the tagged nucleoside-5'-oligophosphates, thereby generating a sequence of the complementary nucleic acid generated at that electrode.

## Patentansprüche

1. Nukleosid-5'-oligophosphat, das kovalent mit einem Polymer-Tag verknüpft ist, wobei das Polymer-Tag ein positiv geladenes Segment (PCS) umfasst, wobei das PCS ein Homopolymer oder ein Heteropolymer ist, das 5 Monomereinheiten bis 100 Monomereinheiten umfasst, eine positive Nettoladung von mindestens +5 bei pH 7,0 aufweist und eine Ladungsdichte von mindestens 0,1 aufweist, mit der Maßgabe, dass das PCS kein Homopolymer von Arginin oder α-verknüpftem Lysin ist, wobei das PCS eine Struktur gemäß Formel 1 umfasst:
(REPEAT)ₐ Formel 1,
wobei:
"REPEAT" eine heteropolymere Sequenz von mindestens 2 Monomereinheiten ist, wobei REPEAT eine Ladungsdichte von mindestens 0,1 aufweist; und
"a" eine ganze Zahl ≥ 2 ist, mit der Maßgabe, dass die ganze Zahl ausreicht, ein PCS mit einer Nettoladung von mindestens +5 bei pH 7,0 und einer Gesamtlänge von 5 bis 100 Monomereinheiten zu ergeben.

2. Nukleosid-5'-oligophosphat nach Anspruch 1, wobei die Aminosäuren oder Aminosäureanaloga aus der Gruppe ausgewählt sind, die aus Lysin (K), Diaminopropionsäure (Dap), ε-verknüpftem Lysin (K'), Aminoethyl-substituierter Diaminopropionsäure (Dapa), Prolin (P), Propargylglycin (Pra), Arginin (R), 4-Benzyloxyprolin (BnoP), Glycin (G), Aminoethylpiperazinessigsäure (Apa), Alanin (A), Pyrrolylalanin (PyrAla), Phenylalanin (F), Triethylentriaminsuccinamidsäure (TETA), Histidin (H), Diethylentriaminsuccinamidsäure (DETA), Leucin (L), 4-Aminoprolin (4Ap), Glutamin (Q), Aminoethylglycin (Aeg), Serin (S), β-Alanin (bAla), 4-Nitrophenylalanin (4Npa), Aminohexansäure (Ahx), 2-Aminobenzoesäure (2Aba), 3-Aminobenzoesäure (3Aba), 4-Aminobenzoesäure (4Aba), Oxoaminohexansäure (OAhx), Peptidnukleinsäure (Thymin) (pnaT), Gamma-Diaminopropionsäure (Dap), Peptidnukleinsäure (Cytosin) (pnaC), Glutamat (Glu), 4-Hydroxoprolin (4-OHP), Ornithin (Orn), Citrullin (Cit), Lysin-Peg4 (KPeg4), Lysin-Peg3 (KPeg3), Lysin-Peg2 (KPeg2) und Aminoethylglycin-Peg4 (Aegpeg4) besteht.

3. Nukleosid-5'-oligophosphat nach Anspruch 2, wobei das PCS ferner eine oder mehrere Hexinyl-(Hex)- oder Ethylenglykol-(PEG)-Einheit(en) umfasst.

4. Nukleosid-5'-oligophosphat nach einem der Ansprüche 1-3, wobei das Polymer-Tag eine Struktur der Formel 2 aufweist:
U-PCS-V Formel 2,
wobei:
U optional ist und, wenn vorhanden, 1 bis 10 Monomereinheiten umfasst;
V optional ist und, wenn vorhanden, 1 bis 10 Monomereinheiten umfasst; und
wobei die Struktur der Formel 2 eine positive Nettoladung von mindestens +5 bei pH 7,0 und eine Ladungsdichte von mindestens 0,1 aufweist.

5. Nukleosid-5'-oligophosphat nach Anspruch 4, wobei U und V eine neutrale oder eine positive Nettoladung aufweisen.

6. Nukleosid-5'-oligophosphat nach Anspruch 5, wobei U eine oder mehrere Aminosäuren oder Aminosäureanaloga umfasst, die aus der Gruppe ausgewählt sind, die aus Lysin, einem Lysinderivat, Arginin, Ornithin, einer aliphatischen Aminosäure, einem Derivat einer aliphatischen Aminosäure, einer aromatischen Aminosäure, einem Derivat einer aromatischen Aminosäure, Prolin, einem Derivat von Prolin, Dap und Dapa besteht.

7. Nukleosid-5'-oligophosphat nach Anspruch 6, wobei U 1 bis 3 Aminosäuren oder Aminosäureanaloga lang ist, wobei die Aminosäuren oder Aminosäureanaloga aus der Gruppe ausgewählt sind, die aus K, K', 4Npa, Pra und PyrAla besteht.

8. Nukleosid-5'-oligophosphat nach einem der Ansprüche 1-7 mit einer Struktur gemäß Formel 3: 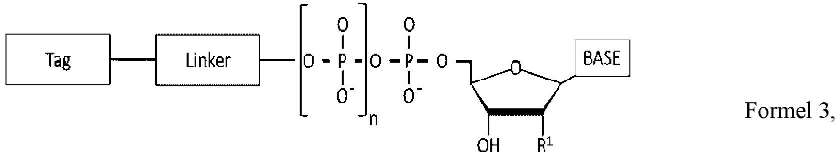 wobei Base aus Adenin, Cytosin, Guanin, Thymin und Uracil ausgewählt ist; R¹ aus H und OH ausgewählt ist; n 2 bis 12 ist; Linker ein Linker ist, der eine kovalent gebundene Kette von 2 bis 100 Atomen umfasst und Tag das Polymer-Tag ist.

9. Nukleosid-5'-oligophosphat nach Anspruch 8, wobei der Linker eine chemische Gruppe umfasst, die aus der Gruppe ausgewählt ist, die aus Folgendem besteht: Ester, Ether, Thioether, Amin, Amid, Imid, Carbonat, Carbamat, Squarat, Thiazol, Thiazolidin, Hydrazon, Oxim, Triazol, Dihydropyridazin, Phosphodiester, Polyethylenglykol (PEG), Pictet-Spengler-Addukt und einer Kombination davon.

10. Nukleosid-5'-oligophosphat nach Anspruch 9, wobei Linker eine Struktur der Formel 4 aufweist:
R⁴-R²-R³ Formel 4,
wobei:
R² aus der Gruppe ausgewählt ist, die aus Esterverknüpfung, Etherverknüpfung, Thioetherverknüpfung, Aminverknüpfung, Amidverknüpfung, Imidverknüpfung, Carbonatverknüpfung, Carbamatverknüpfung, Squaratverknüpfung, Thiazolverknüpfung, Thiazolidinverknüpfung, Hydrazonverknüpfung, Oximverknüpfung, Triazolverknüpfung, Dihydropyridazinverknüpfung, Phosphodiesterverknüpfung, Polyethylenglykol-(PEG)-Verknüpfung, Pictet-Spengler-Addukt und einer Kombination davon besteht;
R³ eine gesättigte oder ungesättigte, verzweigte oder unverzweigte, substituierte oder unsubstituierte Kohlenstoffkette umfasst, die mindestens 2 Kohlenstoffe lang ist und an einem Ende kovalent an eine der Phosphateinheiten des Nukleotidoligophosphats und an dem anderen Ende an R² gebunden ist, und
R⁴ eine gesättigte oder ungesättigte, verzweigte oder unverzweigte, substituierte oder unsubstituierte Kohlenstoffkette umfasst, die mindestens 2 Kohlenstoffe lang ist und an einem Ende kovalent an R² und an dem anderen Ende an das Polymer-Tag gebunden ist.

11. Nukleosid-5'-oligophosphat nach Anspruch 10, wobei R² ein Triazol der Formel 4a ist: 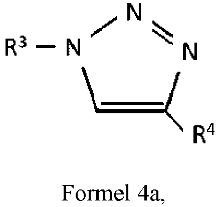 wobei die Summe der Kohlenstoffe in der Kohlenstoffkette R₃ und der Kohlenstoffkette R₄ ≤ 96 ist.

12. Nukleosid-5'-oligophosphat nach Anspruch 10, wobei R² ein Triazol der Formel 4b ist: 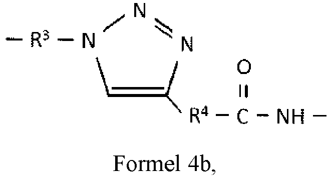 und wobei das -NH- der Peptidbindung, das mit R⁴ verbunden ist, von der/dem N-terminalen Aminosäure oder Aminosäureanalogon des Polymer-Tags beigesteuert wird.

13. Kit, umfassend:
ein erstes Nukleosid-5'-oligophosphat nach einem der Ansprüche 1-12, wobei die Base Adenosin ist,
ein zweites Nukleosid-5'-oligophosphat nach einem der Ansprüche 1-12, wobei die Base Cytosin,
ein drittes Nukleosid-5'-oligophosphat nach einem der Ansprüche 1-12, wobei die Base Guanin ist, und
ein viertes Nukleosid-5'-oligophosphat nach einem der Ansprüche 1-12, wobei die Base Thymin oder Uracil ist;
wobei das Polymer-Tag von jedem von dem ersten bis vierten Nukleosid-5'-oligophosphat verschieden ist.

14. System zum Durchführen von Nukleinsäuresequenzierung-durch-Synthese (SBS), wobei das System Folgendes umfasst:
(a) einen Chip, der eine Vielzahl von Messelektroden umfasst;
(b) eine Barriere mit elektrochemischem Widerstand, die auf einer Oberfläche des Chips angeordnet ist, wobei die Barriere eine *cis*-Seite und eine *trans*-Seite aufweist;
(c) eine erste Elektrolytlösung auf der *cis*-Seite der Barriere;
(d) eine zweite Elektrolytlösung auf der *trans*-Seite der Barriere;
(e) eine Vielzahl von Nanoporen, die einen Kanal mit ausreichend negativ geladenen Einheiten umfassen, um eine Matrizen- und/oder Primernukleinsäure im Wesentlichen abzustoßen, wobei die Nanoporen in der Barriere derart angeordnet sind, dass der Kanal einen Ionenaustausch zwischen der ersten Elektrolytlösung und der zweiten Elektrolytlösung ermöglicht, und wobei mindestens ein Teil der Nanoporen nahe genug an einer der Messelektroden liegt, damit die Messelektrode mindestens ein Merkmal eines durch den Kanal der Nanopore fließenden elektrischen Stroms erfassen kann;
(f) ein Computersystem in elektronischer Kommunikation mit den Messelektroden, wobei das Computersystem zum Aufzeichnen des Merkmals des durch die Nanopore fließenden elektrischen Stroms, das von der Messelektrode erfasst wird, ausgelegt ist;
(g) eine Nukleinsäurepolymerase, die mit der Nanopore auf der *cis*-Seite der Barriere assoziiert ist, wobei die Nukleinsäurepolymerase eine matrizenabhängige Nukleinsäureamplifikationsreaktion in der ersten Elektrolytlösung katalysieren kann; und
(h) einen Satz von Nukleosid-5'-oligophosphaten, der in der ersten Elektrolytlösung angeordnet ist, wobei der Satz mindestens ein polymermarkiertes Adenosinnukleosid-5'-oligophosphat, ein polymermarkiertes Guaninnukleosid-5'-oligophosphat, ein polymermarkiertes Cytosinnukleosid-5'-oligophosphat und entweder ein polymermarkiertes Thymidinnukleosid-5'-oligophosphat oder ein polymermarkiertes Uracilnukleosid-5'-oligophosphat einschließt, wobei jedes der polymermarkierten Nukleosid-5'-oligophosphate das Nukleosid-5'-oligophosphat nach einem der Ansprüche 1-12 ist.

15. Sequenzierung-durch-Synthese-(SBS)-Verfahren zum Sequenzieren einer Matrizennukleinsäure, wobei das Verfahren Folgendes umfasst:
Bereitstellen eines Systems nach Anspruch 14 mit einer Vielzahl von aktiven Nanoporensequenzierungskomplexen, wobei jeder aktive Nanoporensequenzierungskomplex Folgendes umfasst:
o mindestens eine der Messelektroden;
∘ eine der Nanoporen, die in die Barriere in der Nähe der Messelektrode eingeführt sind, wobei ein Strom durch die Nanopore fließt und ein Merkmal des Stroms von der Messelektrode erfasst wird;
∘ die Nukleinsäurepolymerase, die mit der Nanopore assoziiert ist, und
∘ die Matrizennukleinsäure, die mit der Nukleinsäurepolymerase komplexiert ist;
Einbauen der markierten Nukleosid-5'-oligophosphate in eine komplementäre Nukleinsäure der Matrizennukleinsäure an den aktiven Nanoporensequenzierungskomplexen durch eine matrizenabhängige Nukleinsäureamplifikationsreaktion, die von der Nukleinsäurepolymerase katalysiert wird, wobei sich das Polymer-Tag des markierten Nukleosid-5'-oligophosphats in den Kanal der Nanopore oder in dessen Nähe bewegt, wenn das markierte Nukleosid-5'-oligophosphat in die komplementäre Nukleinsäure eingebaut wird, und wobei die Bewegung des Polymer-Tags in den Kanal oder in dessen Nähe das Merkmal des durch die Nanopore fließenden Stroms verändert;
Erfassen der Veränderung des Merkmals des durch die Nanopore fließenden Stroms, die durch die Polymer-Tags verursacht wird, mit der Messelektrode und Aufzeichnen der Veränderung auf dem Computersystem; und
Korrelieren jeder aufgezeichneten Veränderung mit einem der markierten Nukleosid-5'-oligophosphate, wodurch eine Sequenz von der komplementären Nukleinsäure erzeugt wird, die an dieser Elektrode erzeugt wurde.

## Revendications

1. Nucléoside-5 '-oligophosphate lié de manière covalente à un marqueur polymère, dans lequel ledit marqueur polymère comprend un segment à charge positive (SCP), dans lequel ledit SCP est un homopolymère ou un hétéropolymère comprenant de 5 motifs monomères à 100 motifs monomères, ayant une charge nette positive d'au moins +5 à pH 7,0 et ayant une densité de charge d'au moins 0,1, à condition que ledit SCP ne soit pas un homopolymère de l'arginine ou de la lysine α-liée, dans lequel le SCP comprend une structure selon la formule 1 :
(RÉPÉTITION)ₐ Formule 1,
dans lequel :
« RÉPÉTITION » représente une séquence hétéropolymère d'au moins 2 motifs monomères, dans lequel RÉPÉTITION a une densité de charge d'au moins 0,1 ; et
« a » représente un entier ≥ 2, à condition que ledit entier soit suffisant pour donner un SCP avec une charge nette d'au moins +5 à pH 7,0 et une longueur globale de 5 à 100 motifs monomères.

2. Nucléoside-5 '-oligophosphate selon la revendication 1, dans lequel les acides aminés ou analogues d'acides aminés sont choisis dans le groupe constitué par la lysine (K), l'acide diaminopropionique (Dap), la lysine ε-liée (K '), l'acide diaminopropionique substitué par un aminoéthyle (Dapa), la proline (P), la propargylglycine (Pra), l'arginine (R), la 4-benzyloxyproline (BnoP), la glycine (G), l'acide aminoéthyl-pipérazineacétique (Apa), l'alanine (A), la pyrrolylalanine (PyrAla), la phénylalanine (F), l'acide triéthylènetriamine-succinamique (TETA), l'histidine (H), l'acide diéthylènetriamine-succinamique (DETA), la leucine (L), la 4-aminoproline (4Ap), la glutamine (Q), l'aminoéthylglycine (Aeg), la sérine (S), la β-alanine (bAla), la 4-nitrophénylalanine (4Npa), l'acide aminohexanoïque (Ahx), l'acide 2-aminobenzoïque (2Aba), l'acide 3-aminobenzoïque (3Aba), l'acide 4-aminobenzoïque (4Aba), l'acide oxoaminohexanoïque (OAhx), l'acide nucléique peptidique (thymine) (pnaT), l'acide gamma-diaminopropionique (Dap'), l'acide nucléique peptidique (cytosine) (pnaC), le glutamate (Glu), la 4-hydroxoproline (4-OHP), l'ornithine (Orn), la citrulline (Cit), la lysine-Peg4 (KPeg4), la lysine-Peg3 (KPeg3), la lysine-Peg2 (KPeg2) et l'aminoéthylglycine-Peg4 (Aegpeg4).

3. Nucléoside-5 '-oligophosphate selon la revendication 2, dans lequel le SCP comprend en outre une ou plusieurs fractions hexynyle (Hex) ou éthylène glycol (PEG).

4. Nucléoside-5 '-oligophosphate selon l'une quelconque des revendications 1 à 3, dans lequel le marqueur polymère a une structure de formule 2 :
U - SCP - V Formule 2,
dans lequel :
U est facultatif et, lorsqu'il est présent, comprend 1 à 10 motifs monomères ;
V est facultatif et, lorsqu'il est présent, comprend 1 à 10 motifs monomères ; et
dans lequel ladite structure de formule 2 a une charge nette positive d'au moins +5 à pH 7,0 et une densité de charge d'au moins 0,1.

5. Nucléoside-5 '-oligophosphate selon l'une quelconque de la revendication 4, dans lequel U et Vont une charge nette neutre ou une charge nette positive.

6. Nucléoside-5 '-oligophosphate selon la revendication 5, dans lequel U comprend un ou plusieurs acides aminés ou analogues d'acides aminés choisis dans le groupe constitué par la lysine, un dérivé de la lysine, l'arginine, l'ornithine, un acide aminé aliphatique, un dérivé d'un acide aminé aliphatique, un acide aminé aromatique, un dérivé d'un acide aminé aromatique, la proline, un dérivé de la proline, le Dap et le Dapa.

7. Nucléoside-5 '-oligophosphate selon la revendication 6, dans lequel U a une longueur de 1 à 3 acides aminés ou analogues d'acides aminés, dans lequel lesdits acides aminés ou analogues d'acides aminés sont choisis dans le groupe constitué par K, K', 4Npa, Pra et PyrAla.

8. Nucléoside-5 '-oligophosphate selon l'une quelconque des revendications 1 à 7, ayant une structure selon la formule 3 : 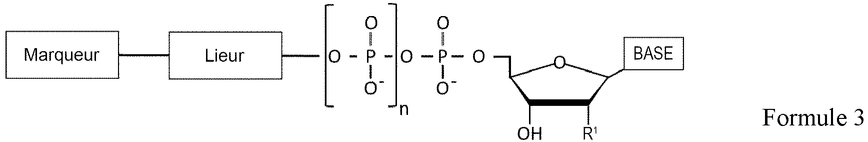 dans lequel Base est choisie parmi l'adénine, la cytosine, la guanine, la thymine et l'uracile ; R¹ est choisi parmi H et OH ; n représente de 2 à 12 ; Lieur représente un lieur comprenant une chaîne liée de manière covalente de 2 à 100 atomes ; et Marqueur représente le marqueur polymère.

9. Nucléoside-5 '-oligophosphate selon la revendication 8, dans lequel le lieur comprend un groupe chimique choisi dans le groupe constitué par : un ester, un éther, un thioéther, une amine, un amide, un imide, un carbonate, un carbamate, un squarate, un thiazole, une thiazolidine, une hydrazone, une oxime, un triazole, une dihydropyridazine, un phosphodiester, un polyéthylène glycol (PEG), un produit d'addition de Pictet-Spengler et une quelconque combinaison de ceux-ci.

10. Nucléoside-5 '-oligophosphate selon la revendication 9, dans lequel Lieur a une structure de formule 4 :
R⁴-R²-R³ Formule 4
dans lequel :
R² est choisi dans le groupe constitué par une liaison ester, une liaison éther, une liaison thioéther, une liaison amine, une liaison amide, une liaison imide, une liaison carbonate, une liaison carbamate, une liaison squarate, une liaison thiazole, une liaison thiazolidine, une liaison hydrazone, une liaison oxime, une liaison triazole, une liaison dihydropyridazine, une liaison phosphodiester, une liaison polyéthylène glycol (PEG), un produit d'addition de Pictet-Spengler et une quelconque combinaison de ceux-ci ;
R³ comprend une chaîne carbonée substituée ou non substituée, ramifiée ou non ramifiée, saturée ou insaturée d'au moins 2 carbones de longueur liée de manière covalente au niveau d'une extrémité à l'une des fractions phosphate de l'oligophosphate nucléotidique et au niveau de l'autre extrémité à R² ; et
R⁴ comprend une chaîne carbonée substituée ou non substituée, ramifiée ou non ramifiée, saturée ou insaturée d'au moins 2 carbones de longueur liée de manière covalente au niveau d'une extrémité à R² et au niveau de l'autre extrémité au marqueur polymère.

11. Nucléoside-5 '-oligophosphate selon la revendication 10, dans lequel R² représente un triazole de formule 4a : 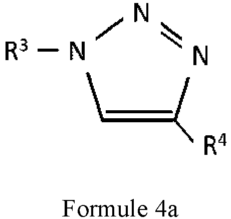 dans lequel la somme des carbones dans la chaîne carbonée R₃ et dans la chaîne carbonée R₄ est ≤ 96.

12. Nucléoside-5 '-oligophosphate selon la revendication 10, dans lequel R² représente un triazole de formule 4b : 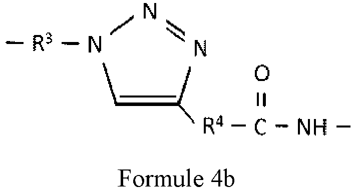 et dans lequel le -NH- de la liaison peptidique liée à R⁴ est apporté par l'acide aminé ou l'analogue d'acide aminé d'extrémité N-terminale du marqueur polymère.

13. Kit comprenant :
un premier nucléoside-5 '-oligophosphate selon l'une quelconque des revendications 1 à 12, dans lequel la base est l'adénosine,
un deuxième nucléoside-5 '-oligophosphate selon l'une quelconque des revendications 1 à 12, dans lequel la base est la cytosine,
un troisième nucléoside-5 '-oligophosphate selon l'une quelconque des revendications 1 à 12, dans lequel la base est la guanine, et
un quatrième nucléoside-5 '-oligophosphate selon l'une quelconque des revendications 1 à 12, dans lequel la base est la thymine ou l'uracile ;
dans lequel le marqueur polymère de chacun des premier au quatrième nucléoside-5 '-oligophosphates est différent.

14. Système pour la réalisation d'un séquençage par synthèse (SBS) d'acides nucléiques, le système comprenant :
(a) une puce comprenant une pluralité d'électrodes de détection ;
(b) une barrière électrochimiquement résistive disposée sur une surface de la puce, la barrière ayant un côté *cis* et un côté *trans* ;
(c) une première solution d'électrolyte sur le côté *cis* de la barrière ;
(d) une seconde solution d'électrolyte sur le côté *trans* de la barrière ;
(e) une pluralité de nanopores comprenant un canal ayant suffisamment de fractions à charge négative pour repousser sensiblement un acide nucléique matrice et/ou amorce, les nanopores étant disposés dans la barrière de telle sorte que le canal permet un échange d'ions entre la première solution d'électrolyte et la seconde solution d'électrolyte, et au moins une partie des nanopores étant suffisamment proches de l'une des électrodes de détection pour que l'électrode de détection puisse détecter au moins une caractéristique d'un courant électrique passant à travers le canal du nanopore ;
(f) un système informatique en communication électronique avec les électrodes de détection, le système informatique étant adapté pour enregistrer la caractéristique du courant électrique passant à travers le nanopore qui est détectée par l'électrode de détection ;
(g) une acide nucléique polymérase associée au nanopore sur le côté *cis* de la barrière, l'acide nucléique polymérase étant capable de catalyser une réaction d'amplification d'acide nucléique dépendant d'une matrice dans la première solution d'électrolyte ; et
(h) un ensemble de nucléoside-5 '-oligophosphates disposés dans la première solution d'électrolyte, l'ensemble comprenant au moins un nucléoside-5 '-oligophosphate d'adénosine marqué par un polymère, un nucléoside-5 '-oligophosphate de guanine marqué par un polymère, un nucléoside-5 '-oligophosphate de cytosine marqué par un polymère, et soit un nucléoside-5 '-oligophosphate de thymidine marqué par un polymère soit un nucléoside-5 '-oligophosphate d'uracile marqué par un polymère, chacun des nucléoside-5 '-oligophosphates marqués par un polymère étant le nucléoside-5'-oligophosphate selon l'une quelconque des revendications 1 à 12.

15. Procédé de séquençage par synthèse (SBS) permettant de séquencer un acide nucléique matrice, le procédé comprenant :
la fourniture d'un système selon la revendication 14 ayant une pluralité de complexes de séquençage par nanopore actif, chaque complexe de séquençage par nanopore actif comprenant :
∘ au moins l'une des électrodes de détection ;
∘ l'un des nanopores insérés dans la barrière à proximité de l'électrode de détection, un courant passant à travers le nanopore et une caractéristique du courant étant détectée par l'électrode de détection ;
∘ l'acide nucléique polymérase associée au nanopore ; et
∘ l'acide nucléique matrice complexé avec l'acide nucléique polymérase ;
au niveau des complexes de séquençage par nanopore actif, l'incorporation des nucléoside-5 '-oligophosphates marqués dans un acide nucléique complémentaire de l'acide nucléique matrice par une réaction d'amplification d'acide nucléique dépendant d'une matrice catalysée par l'acide nucléique polymérase, le marqueur polymère du nucléoside-5 '-oligophosphate marqué se déplaçant dans ou à proximité du canal du nanopore lorsque le nucléoside-5 '-oligophosphate marqué est incorporé dans l'acide nucléique complémentaire, et le mouvement du marqueur polymère dans ou à proximité du canal modifiant la caractéristique du courant passant à travers le nanopore ;
la détection de la modification de la caractéristique du courant passant à travers le nanopore provoquée par les marqueurs polymères avec l'électrode de détection et l'enregistrement de la modification sur le système informatique ; et
la corrélation de chaque modification enregistrée à l'un des nucléoside-5 '-oligophosphates marqués, générant ainsi une séquence de l'acide nucléique complémentaire générée au niveau de cette électrode.
